# EUROPEAN PATENT APPLICATION

(11) **EP 4 726 059 A1**
(43) Date of publication of application: **15.04.2026**
(21) Application number: 24818725.4
(22) Date of filing: 06.06.2024
(51) Int. Cl.: C12Q 1/6886, A61K 31/675, A61K 31/7068, A61K 31/4709, A61K 31/337, A61K 31/496, A61K 31/513, A61K 31/704, A61K 31/4745, A61K 31/7048, A61K 31/14, A61K 31/4045, A61K 31/519, A61K 31/517, A61K 31/4747, A61K 31/55, A61K 31/397, A61K 31/438, A61P 35/00

(54) **CORRELATION OF AKR1C3 ENZYME EXPRESSION LEVEL WITH KRAS MUTATION, AND MEDICAL USE**

(30) Priority: 06.06.2023 CN 202310662375
(71) Applicant: Ascentawits Pharmaceuticals, Ltd., Shenzhen, Guangdong 518122 (CN)
(72) Inventor: LI, Anrong, Shenzhen, Guangdong 518000 (CN); DUAN, Jianxin, Shenzhen, Guangdong 518000 (CN); QI, Tianyang, Shenzhen, Guangdong 518000 (CN); MENG, Fanying, San Francisco, California 94121 (US)
(74) Representative: Haseltine Lake Kempner LLP
(86) International application number: PCT/CN2024/097734
(87) International publication number: WO 2024/251193

(57) **Abstract**

Correlation of the AKR1C3 enzyme expression level with KRAS mutation, and a medical use. For an AKR1C3 enzyme activated anti-cancer prodrug, KRAS gene mutation can directly serve as a test target before drug administration, that is, a patient with KRAS mutation is the patient having a high AKR1C3 enzyme expression level and AKR1C3 enzyme expression level or AKR1C3 RNA testing is not required. That is to say, a positive KRAS mutation testing result can be used as a screening index to screen the patient having a high AKR1C3 enzyme expression level.

## Description

### TECHNICAL FIELD

The present invention relates to a treatment method for cancer, and in particular to a treatment method for cancer having specific genes.

### BACKGROUND

AST-3424 (OBI-3424/TH3424) is an AKR1C3 enzyme-activated DNA alkylating agent prodrug that has entered phase II clinical trials (clinical registrations in China are CTR20201915, CTR20201908, CTR20191399, and CTR20191371; clinical registrations in the United States of America are NCT04315324, and NCT03592264) in both China and the United States of America, and it is specifically activated by AKR1C3 enzyme highly expressed in a variety of tumors and releases DNA alkylating agent to exert anti-tumor effects.

Preclinical studies have shown that the efficacy of AST-3424 is highly correlated with the expression of AKR1C3 enzyme, and minor differences in the enzyme expression will greatly affect the final therapeutic efficacy of the drug (AACR 2016, Abstract# 1369: *In vitro* and *in vivo* antitumor activity of TH3424: Preclinical rationale for a highly selective AKR1C3 prodrug for treating hepatocellular carcinomas; AACR-NCI-EORTC Annual Meeting, 2017, Abstract: LB-B16: The AKR1C3-Activated Prodrug OBI-3424 Exerts Profound *In Vivo* Efficacy Against Preclinical Models of T-Cell Acute Lymphoblastic Leukemia (T-ALL); a Pediatric Preclinical Testing Consortium Study; Clin Cancer Res 2019; 25:4493-503:OBI-3424, a Novel AKR1C3-Activated Prodrug, Exhibits Potent Efficacy against Preclinical Models of T-ALL; Am J Cancer Res 2021; 11(7):3645-3659, A novel selective AKR1C3-activated prodrug AST-3424/OBI-3424 exhibits broad anti-tumor activity).

The above-mentioned Phase II clinical trials in China and the United States of America will investigate the relationship between AKR1C3 enzyme expression level and therapeutic efficacy in cancer patients, and determine quantitative value of AKR1C3 expression level for screening patients who would benefit from the experimental drug.

That is to say, there is a need to detect the expression level of AKR1C3 enzyme in patients before the administration of the AKR1C3 enzyme-activated anti-cancer prodrug described above, and the drug may have better therapeutic effect only for patients whose expression level of AKR1C3 enzyme has achieved the predetermined expression level.

Based on the Phase I clinical results of the drug, researchers have determined that the patient may enroll in Phase II clinical patients whose AKR1C3 enzyme expression level is H-score≥135 (IHC) (Safety, Pharmacokinetics, and Clinical Activity of OBI-3424, an AKR1C3-Activated Prodrug, in Patients with Advanced or Metastatic Solid Tumors: A Phase 1 Dose-Escalation Study, J Clin Oncol 40, 2022, suppl 16; abstr 3030, DOI:10.1200/JCO.2022.40.16_suppl.3030; Tsimberidou, A.M., Verschraegen, C.F., Wesolowski, R. et al. Phase 1 dose-escalation study evaluating the safety, pharmacokinetics, and clinical activity of OBI-3424 in patients with advanced or metastatic solid tumors. Br J Cancer (2023). https://doi.org/10.1038/s41416-023-02280-4).

In addition to AST-3424, similar AKR1C3 enzyme-activated DNA alkylating agent anti-cancer prodrug TFX05-01 has entered phase I clinical trials (clinical registration in China is CTR20220957; clinical registration in the United States of America is NCT05434299; Charles Z Ding, Zhe Cai, Wei Sha. Preclinical evaluation of TFX05-01, a selective AKR1C3-targeted prodrug for solid tumor [abstract]. In: Proceedings of the American Association for Cancer Research Annual Meeting 2022; 2022 Apr 8-13. Philadelphia (PA): AACR; Cancer Res 2022;82(12_Suppl): Abstract nr 5691.), which is also need to investigate the relationship between AKR1C3 enzyme expression level and therapeutic effect of the drug in clinical trials.

To quantitatively detect the expression level of AKR1C3 enzyme, the prior art WO2022048492 discloses an IHC detection method using FFPE tumor tissue sample sections, and WO2022183483 discloses a PCR detection method using blood samples. The former requires the use of FFPE sections of patient's tumor tissue as detection sample, which is accordingly suitable for detecting patients with solid tumors. The latter requires the use of patient's blood sample, which is accordingly suitable for hematological tumors, especially leukemia.

However, in fact, for some patients with malignant solid tumors, the viable tissue cannot be taken from their tumor tissues to make FFPE sections as IHC detection samples, such as most pancreatic cancers, some liver cancers, almost all intrahepatic cholangiocarcinomas, primary brain cancers, and metastatic brain cancers, etc.

Therefore, it is necessary to find alternative detection technologies, detection targets and screen out patients who may benefit from AKR1C3 enzyme-activated anti-tumor (cancer) prodrug therapy based on detection results and complete the drug administration treatment.

### SUMMARY OF THE INVENTION

In the further preclinical efficacy studies, the inventors found that AKR1C3 enzyme-activated prodrug AST-3424 and AST have significant inhibitory effects on PDX tumor model and cells with Kras-G12D/G12C mutation (Examples 1-9 of PCT/CN2022/120817, with Publication No. WO2023/046060). The inventors speculated whether the Kras-G12D/G12C mutation is somehow associated with high expression of AKR1C3 enzyme: whether a patient with Kras-G12D/G12C mutation is also a patient with high expression of AKR1C3 enzyme.

Based on the above speculation, the inventors conducted an AKR1C3 RNA detection and an AKR1C3 protein expression level detection on the corresponding tissues of the PDX model as described above (Example 10 of PCT/CN2022/120817, with Publication No. WO2023/046060). The results demonstrated that RNA and protein expression levels of AKR1C3 in the above models with Kras-G12D/G12C mutation were high, which confirmed the above speculation: the Kras-G12D/G12C mutation is associated with the high expression of AKR1C3 enzyme; Kras-G12D/G12C mutation is accompanied with the high expression of AKR1C3 enzyme.

For verifying the conclusion that Kras-G12D mutation is accompanied with the high expression of AKR1C3, the inventors collected the AKR1C3 expression data of 179 PDX models with Kras-G12D mutation and found that:
The distribution trend of AKR1C3 expression level in the KRAS G12D PDX models was mainly concentrated in medium and high expressions, which account for 90.4%.

For verifying the conclusion that Kras-G12C mutation is accompanied with the high expression of AKR1C3 enzyme, the inventors collected the AKR1C3 expression data of 51 PDX models with Kras-G12C mutation and found that:
The AKR1C3 expression level in the KRAS G12C PDX models was equally distributed in low, medium and high expressions, wherein the expression levels above medium expression levels account for 66.7%.

For verifying the conclusion that Kras-G13D mutation is accompanied with the high expression of AKR1C3 enzyme, the inventors collected the AKR1C3 expression data of 48 PDX models with Kras-G13D mutation and found that:
The distribution trend of AKR1C3 expression level in the KRAS G13D PDX models was mainly concentrated in medium and high expressions, wherein the expression levels above medium expression levels account for 83.3%.

The inventors further learned from the literature that KRAS G12D mutant tumor cells can directly up-regulate and activate NRF2 (Nature, 2011, 475: 106; Cancer Res, 2014, 74: 7430) through the RAF-MEK-ERK-Jun signal transduction pathway (Nature, 2011, 475: 106). Activated NRF2 can further up-regulate and activate a series of downstream genes, including AKR1C3 (Chem Res Toxicol, 2017, 30:162; Cancer, 2019, 11: 1715). Based on above regulatory pathways, it can be seen that a gene mutation that can up-regulate or activate NRF2, such as G12D mutation, can eventually up-regulate AKR1C3 gene, resulting in increased expression level of AKR1C3 enzyme in cancer cells.

Based on the above experiments and literature research, the inventors proposed that for an AKR1C3 enzyme-activated anti-cancer prodrug, KRAS gene mutation can be directly used as a detection target before drug administration, i.e., a patient with KRAS mutation is the patient with high expression of AKR1C3 enzyme, and AKR1C3 enzyme expression level or AKR1C3 RNA detection is not required. That is, positive KRAS mutation detection result can be used as a screening index to screen the patient with high expression of AKR1C3 enzyme.

For this purpose, the present application provides the following technical solutions.

### Solution 1

An administration method for cancer, tumor, or disorders or cell proliferative diseases caused by cancer or tumor, including administering an AKR1C3 enzyme-activated prodrug to a patient when the tumor or cancer tissue of the patient is detected to have a KRAS gene mutation or the patient is detected to have a KRAS gene mutation, without detecting the AKR1C3 protein expression level or the AKR1C3 RNA content in the tumor or cancer tissue of the patient.

### Solution 2

A method for treating cancer, tumor, or disorders or cell proliferative diseases caused by cancer or tumor, including a step of administering a drug or formulation containing an AKR1C3 enzyme-activated prodrug and a step of detecting KRAS gene mutation, and not including a step of detecting the AKR1C3 protein expression level or the AKR1C3 RNA content in the tumor or cancer tissue of the patient, and administering the drug or formulation containing AKR1C3 enzyme-activated prodrug to the patient when the tumor or cancer tissue of the patient is detected to have a KRAS gene mutation or the patient is detected to have a KRAS gene mutation.

### Solution 3

Pharmaceutical use of an AKR1C3 enzyme-activated prodrug in the manufacture of a drug for treating cancer, tumor, or disorders or cell proliferative diseases caused by cancer or tumor, characterized in that: the drug is formulated for administration to the patient whose tumor or cancer tissue is detected to have a KRAS gene mutation or the patient who is detected to have a KRAS gene mutation; and the AKR1C3 protein expression level detection or the AKR1C3 RNA content detection in the tumor or cancer tissue of the patient is not required.

### Solution 4

A formulation unit package, comprising an independent package container for holding the drug formulation, an outer package component for accommodating the independent package container, and drug instructions, characterized in that: the drug formulation comprises an AKR1C3 enzyme-activated prodrug, and the drug instructions record:
the drug is used for treating a patient with cancer, tumor, or disorders or cell proliferative diseases caused by cancer or tumor;
the patient was conducted for KRAS gene mutation detection, without conducting the detection of the AKR1C3 protein expression level or the AKR1C3 RNA content in the tumor or cancer tissue;
when the tumor or cancer tissue of the patient is detected to have a KRAS gene mutation or the patient is detected to have a KRAS gene mutation, the drug or formulation containing AKR1C3 enzyme-activated prodrug is administered to the patient.

AKR1C3 enzyme-activated prodrug refers to a compound that is a prodrug, and the prodrug molecule is reacted with AKR1C3 enzyme to release cytotoxic anti-tumor compound after reaction.

Broadly speaking, AKR1C3 enzyme-activated prodrugs meet, but are not limited to, at least one of the following conditions:
A. The inhibitory effect of a compound on cancer cell proliferation detected in the presence of AKR1C3 inhibitor (for example, compound 36, i.e. 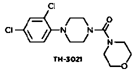 in Bioorganic and Medicinal Chemistry, 2014: 962-977) is smaller than that detected in the absence of AKR1C3 inhibitor. When the inhibitory effect on cancer cell proliferation is quantified with IC₅₀, if the IC₅₀ of a compound on a cancer cell line detected in the presence of AKR1C3 inhibitor is greater than the IC₅₀ detected in the absence of AKR1C3 inhibitor, it can be determined that the compound is an AKR1C3-activated anticancer drug (Lysis-Prodrug). Specific compounds are as the compounds disclosed in the following patent documents:
   PCT/US2016/021581 with Publication No. WO2016145092A1, corresponding to the Chinese Application No. 2016800150788 with Publication No. CN107530556A;
   PCT/US2016/062114 with Publication No. WO2017087428, corresponding to the Chinese Application No. 2016800446081 with Publication No. CN108290911A;
   PCT/US2016/025665 with Publication No. WO2016161342, corresponding to the Chinese Application No. 2016800200132 with Publication No. CN108136214A; and
   PCT/NZ2019/050030 with Publication No. WO2019190331, corresponding to the Chinese Application No. CN2019800234236 with Publication No. CN111918864A. The entire contents of above patent documents are incorporated herein by reference in their entirety.

Among them, the compounds disclosed in Patent Application PCT/US2016/021581 with Publication No. WO2016145092A1, corresponding to the Chinese Application No. 2016800150788 with Publication No. CN107530556A; Patent Application PCT/US2016/062114 with Publication No. WO2017087428, corresponding to the Chinese Application No. 2016800446081 with Publication No. CN108290911A; and Patent Application PCT/US2016/025665 with Publication No. WO2016161342, corresponding to the Chinese Application No. 2016800200132 with Publication No. CN108136214A are lysis-prodrugs that ultimately lyse and are metabolized to primary drugs that play a role being 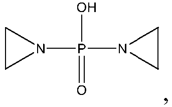 paclitaxel, camptothecin and other drugs; the compounds disclosed in Patent Application PCT/NZ2019/050030 with Publication No. WO2019190331, corresponding to the Chinese Application No. CN2019800234236 with Publication No. CN111918864A are lysis-prodrugs that ultimately lyse and are metabolized to primary drugs that play a role being nitrogen mustard structural drugs.

B. The inhibitory effect of a compound on the proliferation of cancer cells with different expression levels of AKR1C3 enzyme is significantly different, and the inhibitory effect on the proliferation of cancer cells with high expression of AKR1C3 enzyme is much greater than that of cancer cells with low expression of AKR1C3 enzyme. When the inhibitory effect on cancer cell proliferation is quantified with IC₅₀, if the IC₅₀ value of a compound on cancer cells with high expression of AKR1C3 enzyme is lower than the IC₅₀ value on cancer cells with low expression of AKR1C3 enzyme, the compound can be determined. Specific compounds are as the compounds disclosed in Patent Application PCT/CN2020/120281 with Publication No. WO2021068952A1, the entire contents of which are incorporated herein by reference in its entirety.

In prior art, AKR1C3 enzyme-activated drugs (such as AST-3424, AST-001 that have entered clinical trial phase) all require the patients with solid tumors to provide pathological wax blocks or sections (including archived pathological wax blocks and sections) for AKR1C3 expression analysis, and it is preferred that they are recent samples. Only patients whose AKR1C3 expression in tumor tissues confirmed to reach a certain level with IHC detection can be enrolled in clinical trials.

In fact, due to various reasons, it is possible that some patients do not obtain qualified tissue samples during tumor resection surgery, and some patients do not receive tumor resection surgery treatment before. Therefore, a large number of patients are unable to provide samples for IHC detection to detect the expression level of AKR1 C3 enzyme.

The above four solutions adopt relatively mature KRAS gene mutation detection positive results (detecting the presence of KRAS gene mutations) to replace the detection results of high levels of AKR1C3 protein expression or high AKR1C3 RNA content. In one aspect, for patients whose previous detection results were positive for KRAS gene mutations, it can be directly determined that they have high expression of AKR1C3 enzyme, without additional detection. In another aspect, for patients who have not been detected for KRAS gene mutations before, mature KRAS gene mutation kits can be used for detection, which is convenient and readily available. Furthermore, the samples for gene mutation detection are blood, which is easy to collect and has high patient compliance, while the tumor tissue samples used in IHC detection need to be obtained by biopsy as invasive procedure or obtained during tumor resection surgery.

The KRAS gene mutation is selected from KRAS-G12D mutation, KRAS-G12V mutation, KRAS-G12C mutation, KRAS-G12A and KRAS-G13D.

Mutations in either or both of the genes corresponding to KRAS can be detected and diagnosed with commercially available (companion) diagnostic kits, such as those approved in China:
Amoy Dx, State instrument registration 20153401126, Human KRAS gene mutation detection kit (fluorescent PCR method)
Tellgen, State instrument registration 20163401341, Human K-RAS gene 7 mutations detection kit (PCR fluorescence method).

Evidently, NGS sequencing (YS 450 gene NGS large panel) can also be used to determine the specific KRAS mutation subtype.

The corresponding specific information is as shown in the following table.

| No. | Product Name | Registration Certificate Number | Registrant Name |
|---|---|---|---|
| 1 | KRAS Gene Mutation Detection Kit (Sequencing Method) | State Instrument Registration 20153401472 | Sun Yat-sen University DaAn Gene Co., Ltd |
| 2 | KRAS Gene Mutation Detection Kit (PCR-Fluorescent Probe Method) | State Instrument Registration 20163400765 | Guangzhou Heas BioTech Co., Ltd |
| 3 | Human KRAS/NRAS/BRAF/PIK3CA Gene Mutation Combined Detection Kit (Reversible Termination Sequencing Method) | State Instrument Registration 20213400151 | Genecast BioTech Jiangsu Co., Ltd |
| 4 | Human KRAS Gene Mutation Detection Kit (Fluorescent PCR Method) | State Instrument Registration 20153401126 | Amoy Dx BioTech Co., Ltd |
| 5 | Human KRAS/NRAS/PIK3CA/BRAF Gene Mutation Combined Detection Kit (Fluorescent PCR Method) | State Instrument Registration 20153401124 | Amoy Dx BioTech Co., Ltd |
| 6 | Human EGFR/ALK/BRAF/KRAS Gene Mutation Combined Detection Kit (Reversible Termination Sequencing Method) | State Instrument Registration 20183400286 | Burning Rock Dx Co., Ltd |
| 7 | Human KRAS Gene Mutation Detection Kit (Fluorescent PCR Method) | State Instrument Registration 20163400902 | Wuxi Shenrui BIO-PHARMACEUTICALS Co., Ltd |
| 8 | Human KRAS Gene 7 Mutations Detection Kit (PCR-Fluorescent Probe Method) | State Instrument Registration 20173401254 | YZY Bio Co., Ltd |
| 9 | Human KRAS Gene Codon 12/13 Mutation Detection Kit (Pyrosequencing Method) | State Instrument Registration 20183401600 | Genetech (Shanghai) Co., Ltd |
| 10 | Human EGFR/KRAS/ALK Gene Mutation Detection Kit (Combined Probe Anchor Polymerization Sequencing Method) | State Instrument Registration 20193401032 | Suzhou GenePlus biomedicine Co., Ltd |
| 11 | Human KRAS/NRAS/BRAF Gene Mutation Combined Detection Kit (Fluorescent PCR Method) | State Instrument Registration 20153401886 | Amoy Dx BioTech Co., Ltd |
| 12 | Human KRAS Gene Mutation Detection Kit (Fluorescent PCR Method) | State Instrument Registration 20213401103 | AccBio Co., Ltd |
| 13 | Human EGFR, KRAS, BRAF, PIK3CA, ALK, ROS1 Gene Mutation Detection Kit (Semiconductor Sequencing Method) | State Instrument Registration 20183400294 | Novogene BioTech Co., Ltd |
| 14 | Human KRAS Gene Mutation Detection Kit (Taqman-ARMS Method) | State Instrument Registration 20173404514 | MicroDiag Biomedicine Co., Ltd. |
| 15 | EGFR/ALK/ROS1/BRAF/KRAS/HER2 Gene Mutation Detection Kit (Reversible Termination Sequencing Method) | State Instrument Registration 20183400408 | Geneseeq Medical Device and Diagnostic Inc |
| 16 | EGFR/KRAS/ALK Gene Mutation Combined Detection Kit (Combined Probe Anchor Polymerization Sequencing Method) | State Instrument Registration 20193400621 | BGITech (Wuhan) Co., Ltd. |
| 17 | Human EGFR/KRAS/BRAF/HER2/ALK/ROS1 Gene Mutation Detection Kit (Semiconductor Sequencing Method) | State Instrument Registration 20203400094 | SpaceGen BioTech Co., Ltd |
| 18 | Human EGFR/KRAS/BRAF/PIK3CA/ALK/ROS1 Gene Mutation Detection Kit (Reversible Termination Sequencing Method) | State Instrument Registration 20213400525 | Geneis (Suzhou) Co., Ltd |
| 19 | Human KRAS Gene 7 Mutations Detection Kit (Fluorescent PCR Method) | State Instrument Registration 20193401794 | Amoy Dx BioTech Co., Ltd |
| 20 | Human KRAS/NRAS Gene Mutation Combined Detection Kit (Fluorescent PCR Method) | State Instrument Registration 20153401885 | Amoy Dx BioTech Co., Ltd |
| 21 | Human 8 Gene Mutations Combined Detection Kit (Semiconductor Sequencing Method) | State Instrument Registration 20203400072 | Genetron Co., Ltd |
| 22 | Human 9 Gene Mutations Combined Detection Kit (Reversible Termination Sequencing Method) | State Instrument Registration 20223400343 | Burning Rock Dx Co., Ltd |
| 23 | Human 10 Gene Mutations Combined Detection Kit (Reversible Termination Sequencing Method) | State Instrument Registration 20183400507 | Amoy Dx BioTech Co., Ltd |
| 24 | KRAS Gene Mutation Detection Kit (TaqMan Melting Curve Fluorescent PCR Method) | State Instrument Registration 20193401504 | Roche Diagnostics |

Generally speaking, KRAS gene mutations are detected using completed samples from tumor or cancer tissue of a patient. However, due to various reasons, such as the patient's inability to provide qualified tumor or cancer tissue samples, or the current detection methods being inaccurate, or the tumor or cancer tissue no longer reflecting the recent conditions of a patient, it may be necessary to detect or diagnose the KRAS gene mutation status of the patient *per se.* For instance, blood can be used for gene mutation detection, and the detection result can represent the conditions of tumor or cancer tissue of the patient. Circulating tumor cell (CTC) detection can detect KRAS gene mutation status of specific cancer cells (all tumor cells free in the peripheral blood) by using peripheral blood completed detection samples of a patient. In some cases, the gene mutation status of both parents in the biological parent-child relationship can be used to diagnose or auxiliary diagnose the gene mutation status. This is the situation where a patient is detected to have a KRAS gene mutation, or a patient with a KRAS gene mutation is detected.

In present application, the gene mutation refers to pathogenic gene mutation.

The TMB (Tumor Mutation Load (burden)) level of the described gene mutation is medium.

Since the TMB (Tumor Mutation Load (burden)) is differently high or low between different tumor types: it is generally considered that: a TMB of more than 20 mutations/Mb (Mb represents bases per million) is high; a TMB of less than 10 mutations/Mb is low, and a TMB in the middle is medium. At the World Conference on Lung Cancer in 2017, Squibb Inc. had announced the results of a clinical trial named CheckMate-032. This was a phase II clinical trial that enrolled 401 patients with advanced lung cancer who had failed first-line therapy and were treated with a PD-1 inhibitor alone or in combination with Ipilimumab. The patients were divided into three categories of patients with high TMB, medium TMB, and low TMB according to their TMB levels, then among those who received the combination therapy, the efficiency of the three groups was 62%, 20%, and 23%, respectively, and the efficiency of the group with high TMB was three times higher than the remaining two groups; and the median overall survival of the three groups was: 22.0 months, 3.6 months, and 3.4 months, respectively, with 22.0 months versus 3.4 months, a 6-fold difference! This trial demonstrated that for different cancer treatment drugs, different TMB levels have a significant impact on the efficacy of the drugs.

The AKR1C3 enzyme-activated prodrug is selected from the compound of formulae (1) - (12) or pharmaceutically acceptable salt, solvate, isotope isomer thereof. 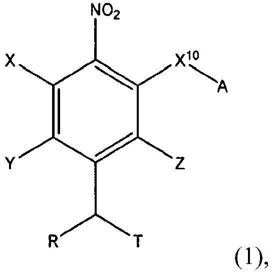 wherein the definitions of X, Y, Z, R, T, A and X¹⁰ are described in the claims of Patent Application PCT/US2016/021581 with Publication No. WO2016145092A1 (corresponding to the Chinese Application No. 2016800150788 with Publication No. CN107530556A), and the synthesis and preparation methods of specific compounds are also described in the above patent application, which is incorporated herein by reference in its entirety. The specific definitions are as follows.
X¹⁰ is O, S, SO, or SO₂;
A is C₆-C₁₀ aryl, 5-15-membered heteroaryl or -N=CR¹R²;
R¹ and R² are each independently hydrogen, C₁-C₆ alkyl, C₃-C₈ cycloalkyl, C₆-C₁₀ aryl, 4-15-membered heterocycle, ether, -CONR¹³R¹⁴ or -NR¹³COR¹⁴;
X, Y and Z are independently hydrogen, CN, halogeno, C₁-C₆ alkyl, C₂-C₆ alkenyl, C₂-C₆ alkynyl, C₃-C₈ cycloalkyl, C₆-C₁₀ aryl, 4-15-membered heterocycle, ether, -CONR¹³R¹⁴ or -NR¹³COR¹⁴;
R is hydrogen, C₁-C₆ alkyl, C₂-C₆ alkenyl, C₂-C₆ alkynyl, C₃-C₈ cycloalkyl, C₆-C₁₀ aryl, 4-15-membered heterocycle, ether, -CONR¹³R¹⁴ or -NR¹³COR¹⁴;
R¹³ and R¹⁴ are each independently hydrogen, C₁-C₆ alkyl, C₃-C₈ cycloalkyl, C₆-C₁₀ aryl, 4-15-membered heterocycle or ether;
T comprises a phosphoramidate alkylating agent comprising one or more Z⁵-X⁵-Y⁵ moieties bonded to an -O-P(Z¹) moiety, where Z⁵ is a heteroatom comprising nitrogen, sulfur or oxygen, X⁵ is substituted or unsubstituted ethylene, Y⁵ is halogeno or another leaving group, or Z⁵-X⁵⁻Y⁵ together form an aziridinyl (NCH₂CH₂) moiety, and Z¹ is O or S; and
wherein the alkyl, alkenyl, alkynyl, cycloalkyl, aryl, heterocyclyl, heteroaryl, ether groups are substituted or unsubstituted.

The AKR1C3 enzyme-activated prodrug compound of formula (1) is selected from the compounds with the following structural formulae:

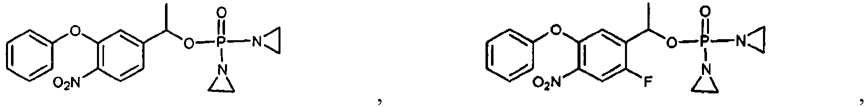

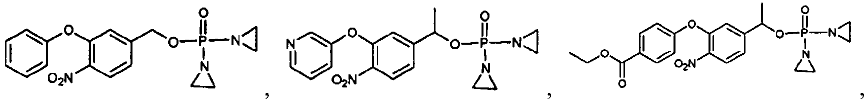

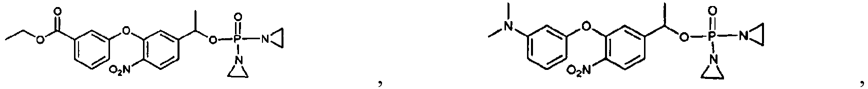

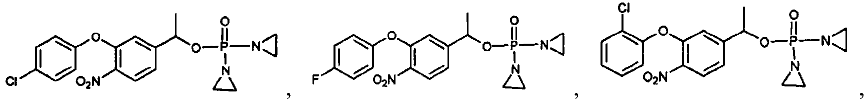

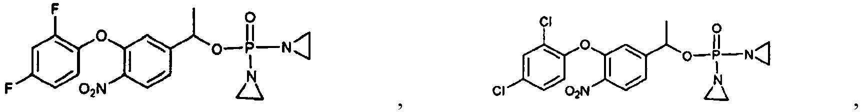

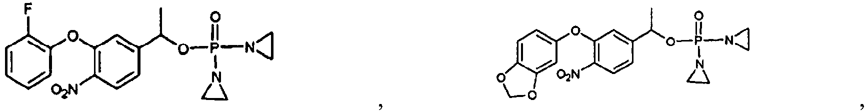

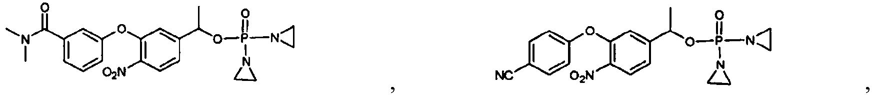

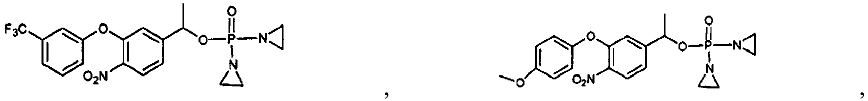

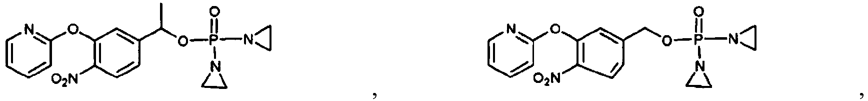

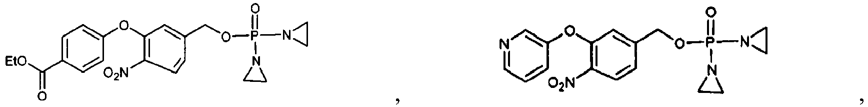

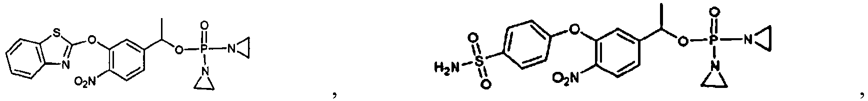

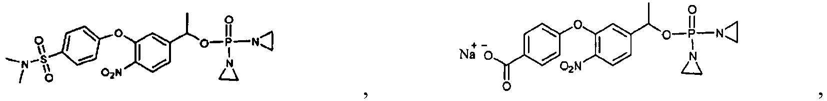

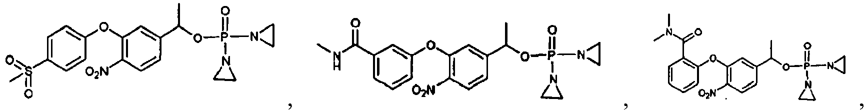

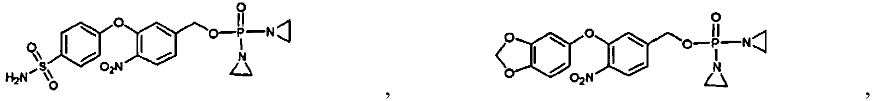

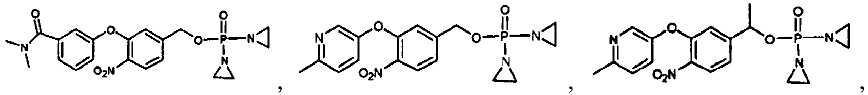

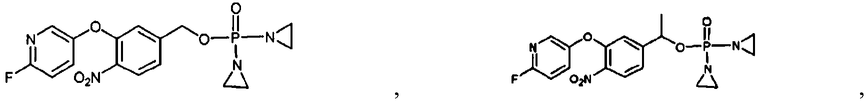

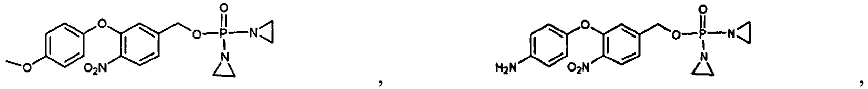

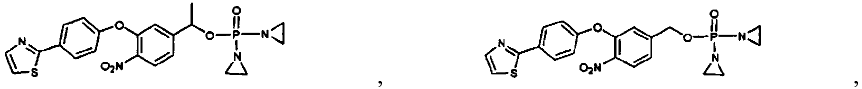

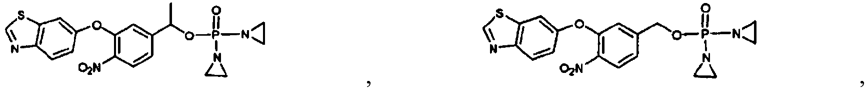

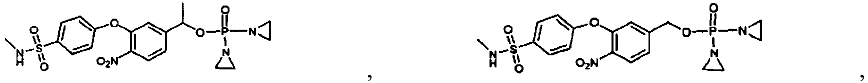

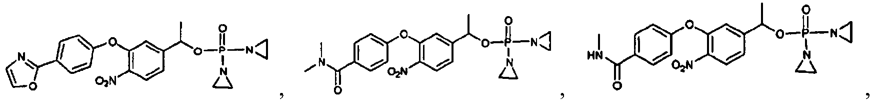

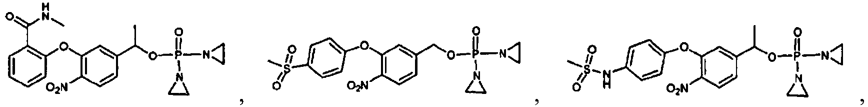

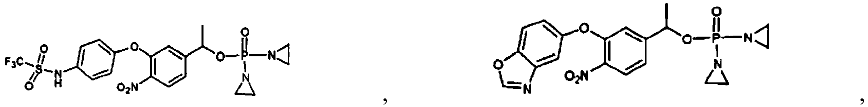

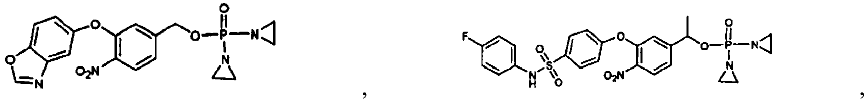

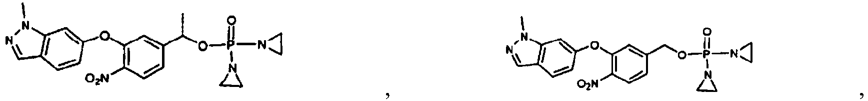

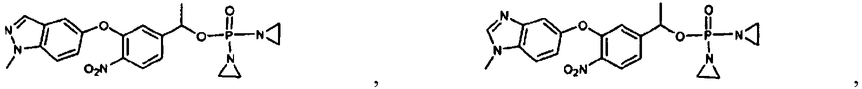

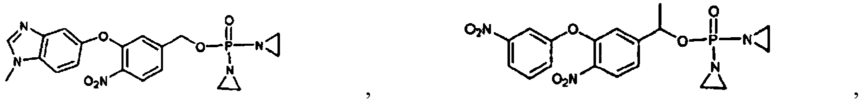

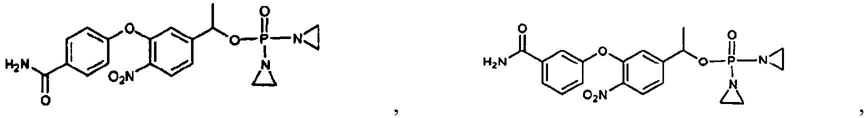

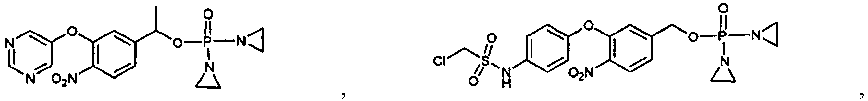

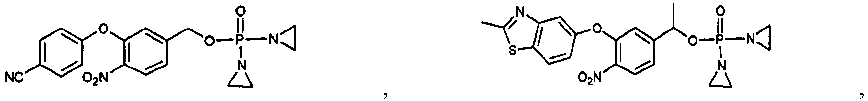

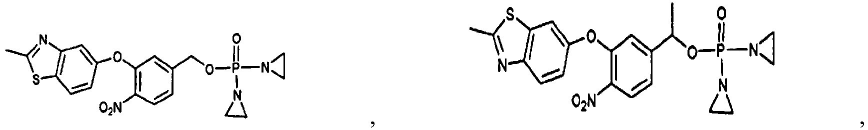

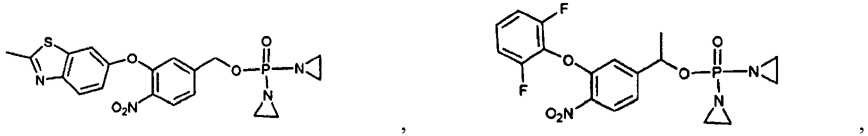

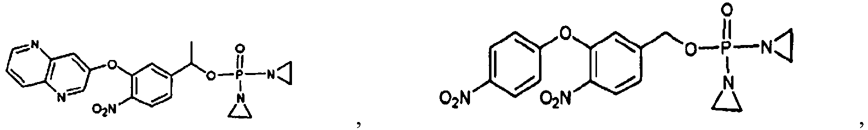

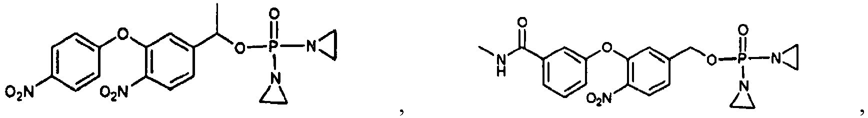

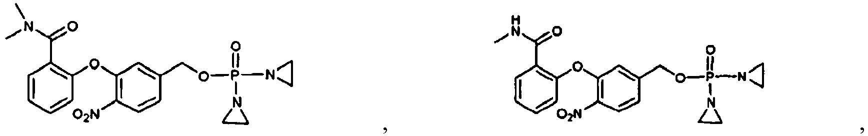

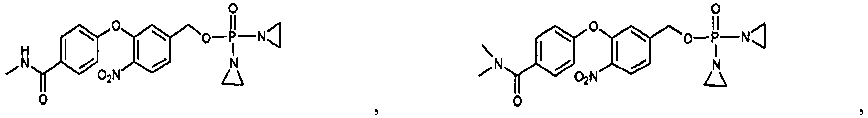

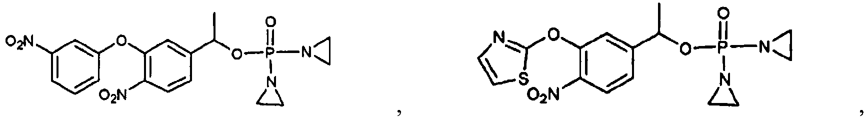

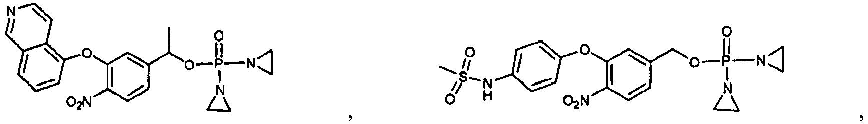

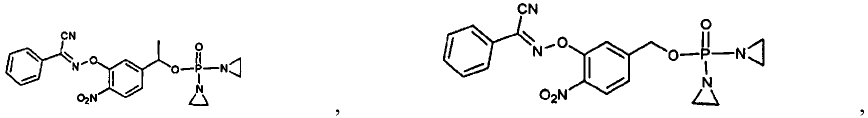

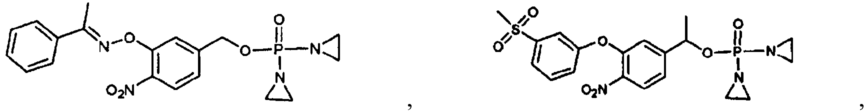

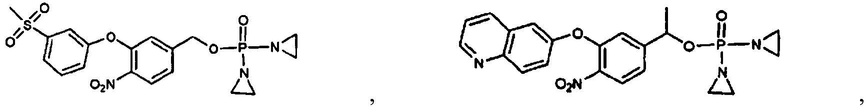

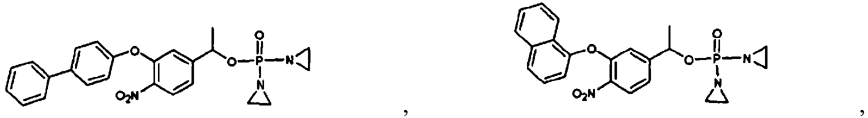

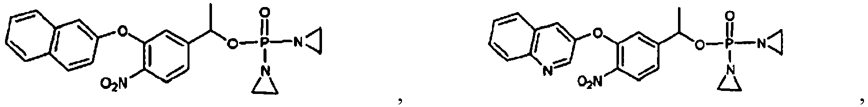

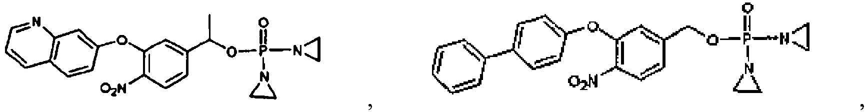

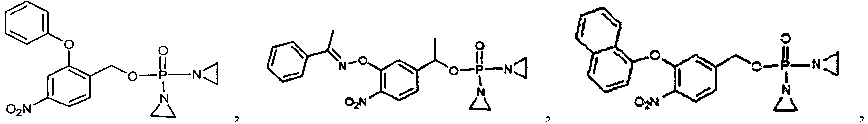

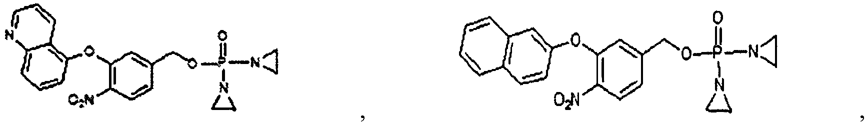

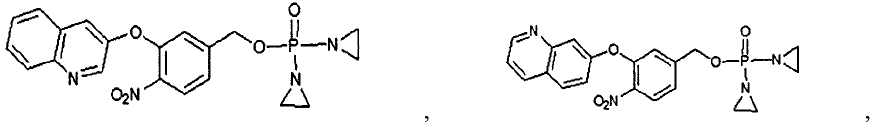

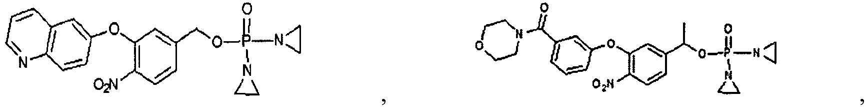

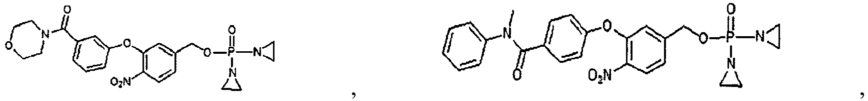

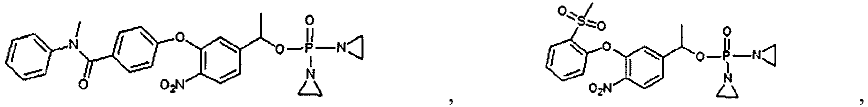

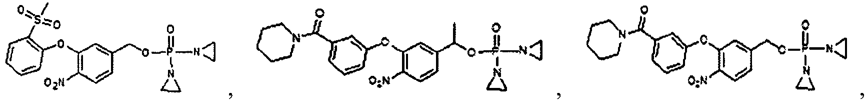

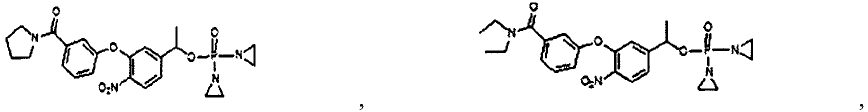

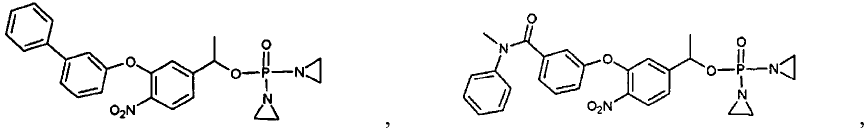

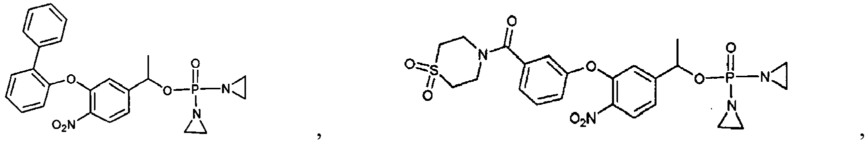

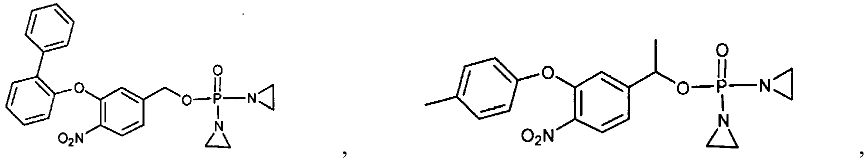

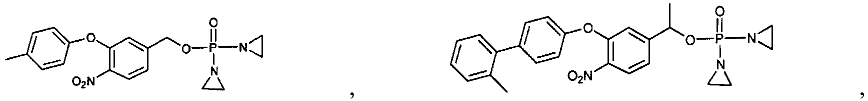

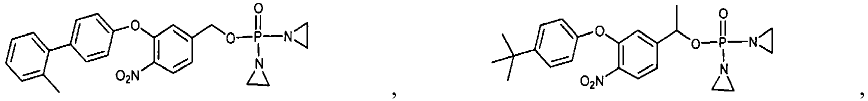

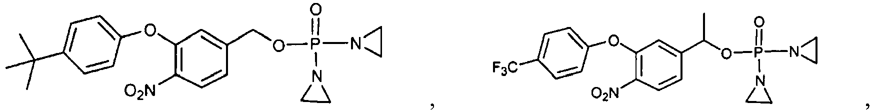

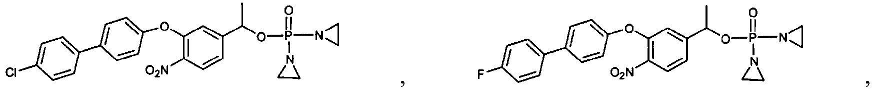

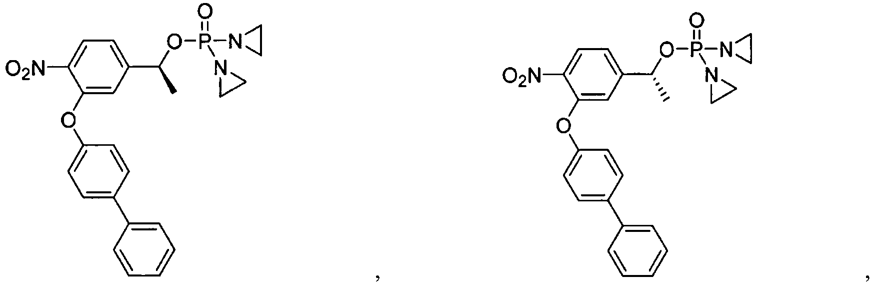

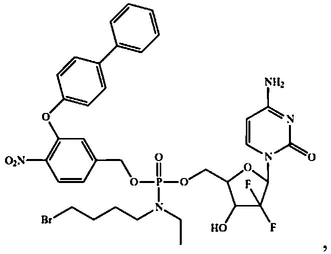

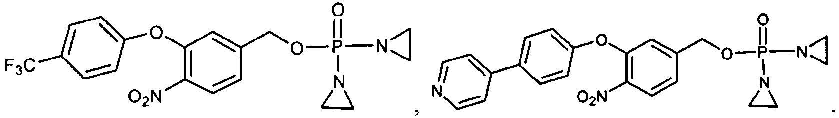

The specific synthetic method of the compound of formula (1) and the corresponding spectral data are disclosed in WO2016145092A1 (corresponding to the Chinese publication text CN107530556A), which is incorporated herein by reference in their entirety. 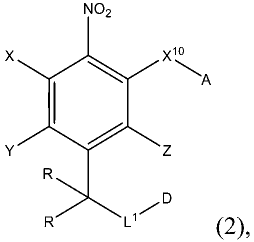 wherein the definitions of X, Y, Z, R, D, L¹, A and X¹⁰ are described in the claims of Patent Application PCT/US2016/025665 with Publication No. WO2016/161342A1 (corresponding to the Chinese Application No. 2016800200132 with Publication No. CN108136214A), and the synthesis and preparation methods of specific compounds are also described in the above patent application, which is incorporated herein by reference in its entirety. The specific definitions are as follows.
X¹⁰ is O, S, SO, or SO₂;
A is C₆-C₁₀ aryl, 5-15-membered heteroaryl, or -N=CR¹R²;
R¹ and R² are each independently hydrogen, C₁-C₆ alkyl, C₃-C₈ cycloalkyl, C₆-C₁₀ aryl, 4-15-membered heterocycle, 5-15-membered heteroaryl, ether, -CONR¹³R¹⁴ or -NR¹³COR¹⁴;
X, Y and Z are independently hydrogen, CN, halogeno, C₁-C₆ alkyl, C₂-C₆ alkenyl, C₂-C₆ alkynyl, C₃-C₈ cycloalkyl, C₆-C₁₀ aryl, 4-15-membered heterocycle, 5-15-membered heteroaryl, ether, -CONR¹³R¹⁴ or -NR¹³COR¹⁴;
Each R is independently hydrogen, C₁-C₆ alkyl, C₂-C₆ alkenyl, C₂-C₆ alkynyl, C₃-C₈ cycloalkyl, C₆-C₁₀ aryl, 4-15-membered heterocycle, 5-15-membered heteroaryl, ether, -CONR¹³R¹⁴ or -NR¹³COR¹⁴;
R¹³ and R¹⁴ are each independently hydrogen, C₁-C₆ alkyl, C₃-C₈ cycloalkyl, C₆-C₁₀ aryl, 4-15-membered heterocycle, 5-15-membered heteroaryl, or ether;
wherein L¹ and D are defined as follows:
   L¹ is selected from 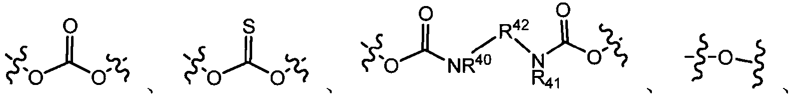 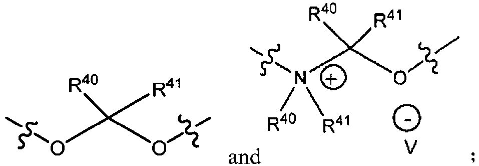
   R⁴⁰ and R⁴¹ are independently hydrogen, C₁-C₆ alkyl, C₂-C₆ alkenyl, C₂-C₆ alkynyl, C₃-C₈ cycloalkyl, C₆-C₁₀ aryl, 4-15-membered heterocycle or 5-15-membered heteroaryl;
   R⁴² is C₂-C₃ alkylene or heteroalkylene optionally substituted with 1-3 C₁-C₆ alkyl groups;
   V (-) is any anion, preferably a pharmaceutically acceptable anion;
   D is a moiety that makes D-OH an anticancer drug, wherein OH is aliphatic hydroxyl or phenolic hydroxyl, or is an OH moiety attached to a phosphorus atom as provided herein; or
   L¹ is 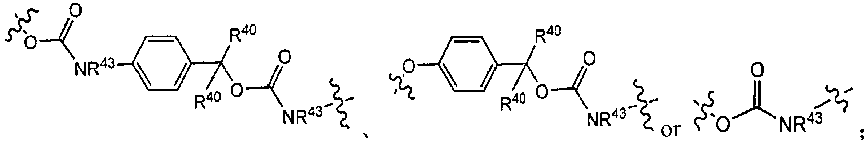
   R⁴⁰ is as defined above, R⁴³ is hydrogen or together with D forms a heterocycle, and the phenyl moiety is optionally substituted; and
   D is a moiety that makes D-NR⁴³H an anticancer drug; or
   L¹ is a bond, -O-C(R⁴⁰R⁴¹)₂-, -O-C(R⁴⁰R⁴¹)-NR⁴⁰R⁴¹(+)-C(R⁴⁰R⁴¹)- or 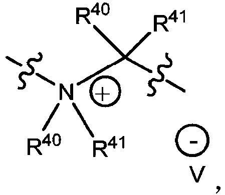
   wherein R⁴⁰, R⁴¹, and V are as defined above; and
   D is an anticancer drug containing tertiary or secondary nitrogen atom, wherein the tertiary or secondary nitrogen atom is bonded to L¹;
      and
   wherein the alkyl, alkenyl, alkynyl, cycloalkyl, aryl, heterocycle, heteroaryl, and ether are optionally substituted.

The AKR1C3 enzyme-activated prodrug compound of formula (2) is selected from the compounds with the following structural formulae:

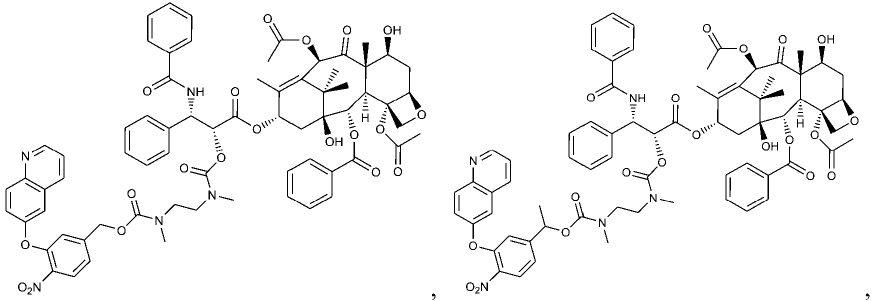

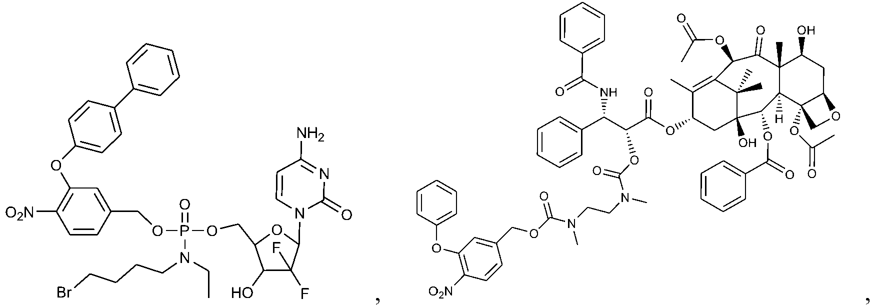

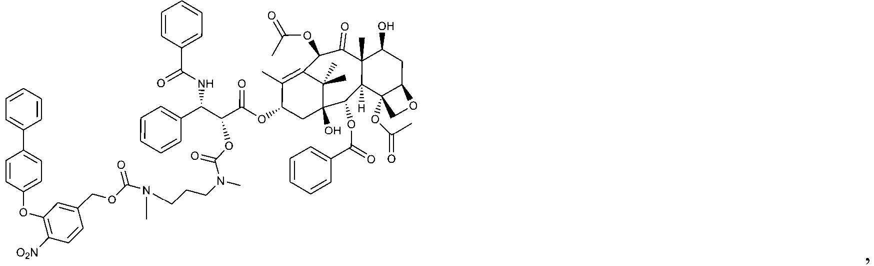

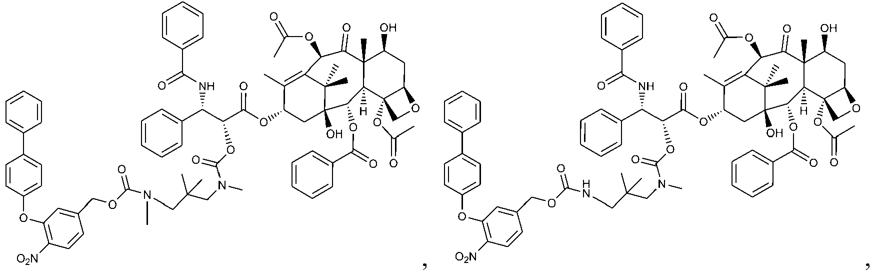

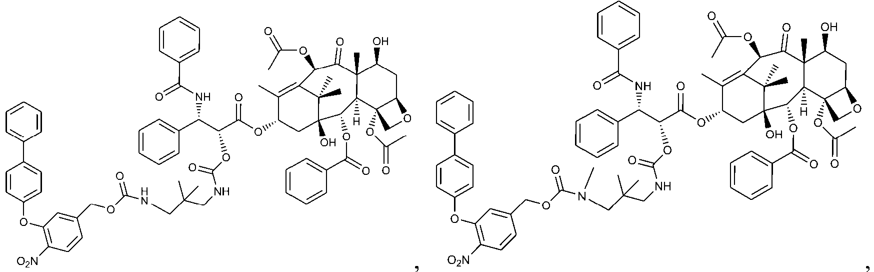

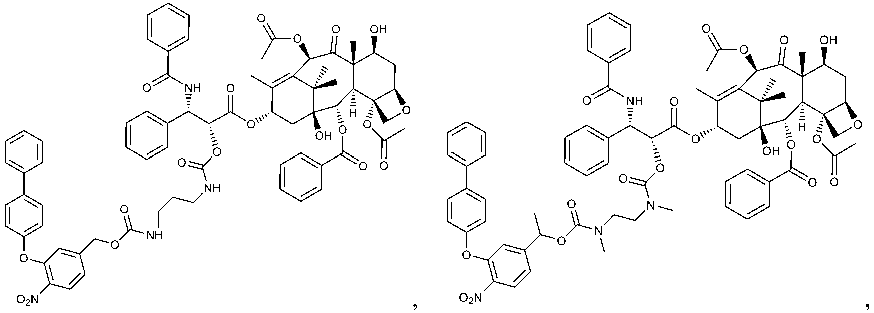

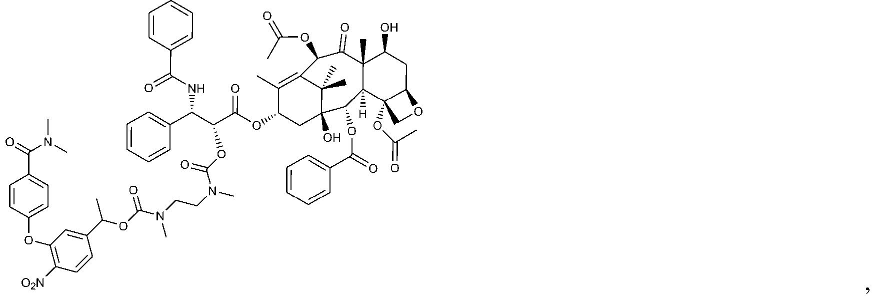

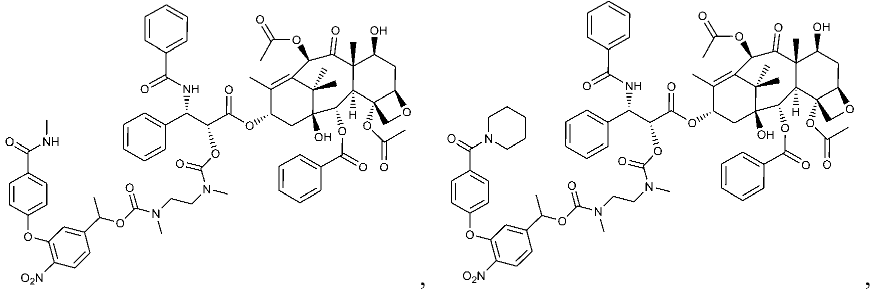

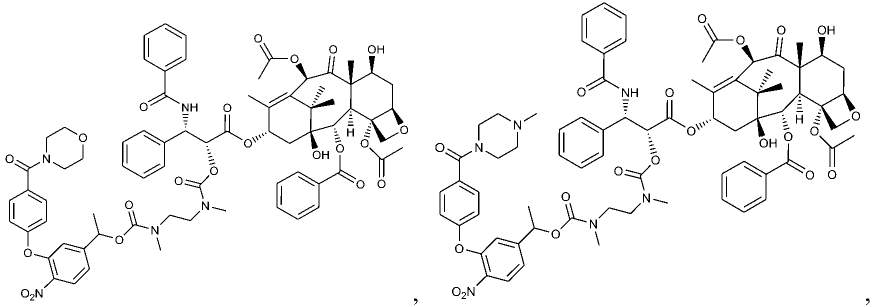

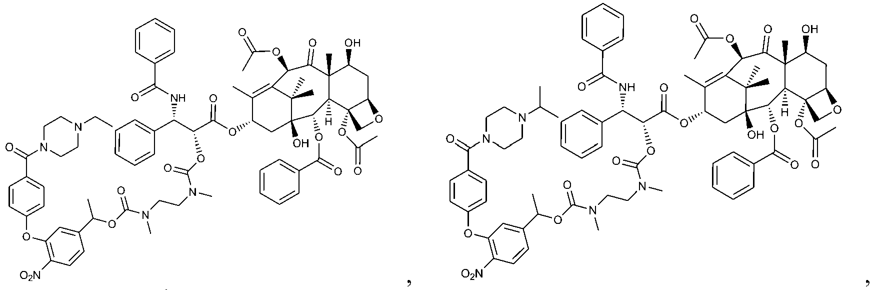

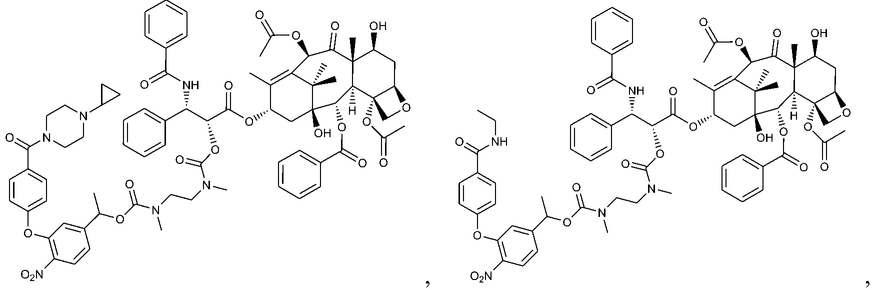

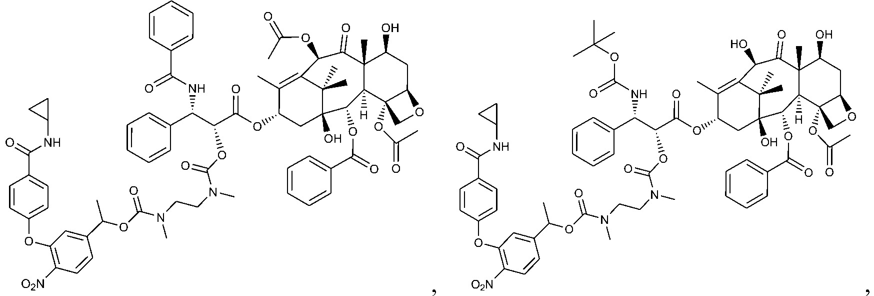

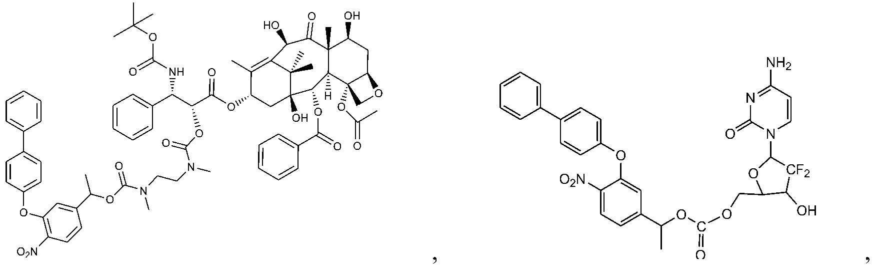

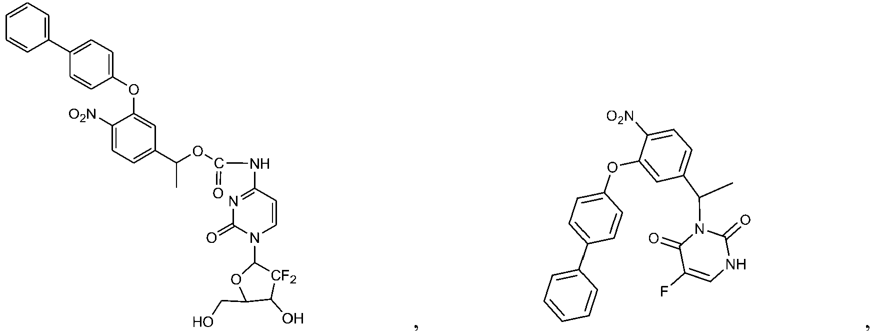

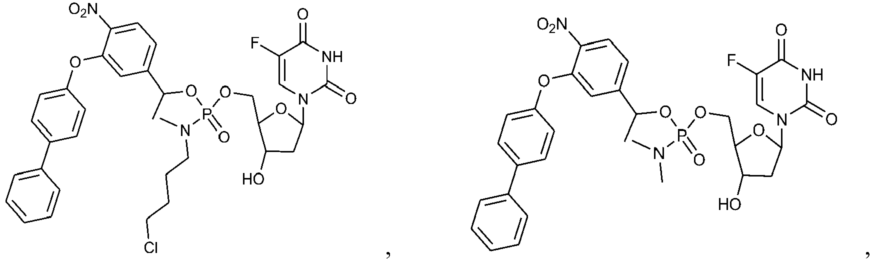

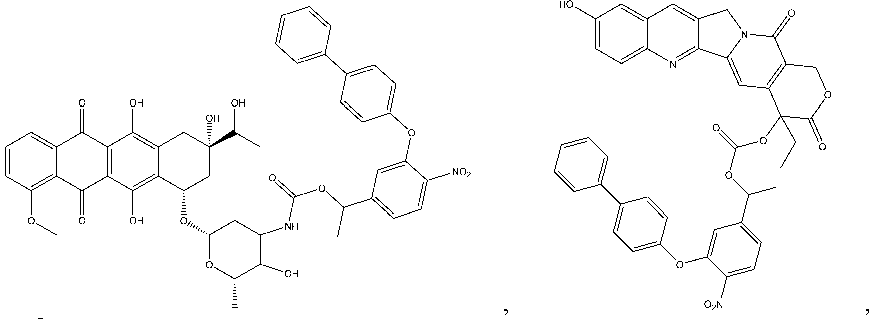

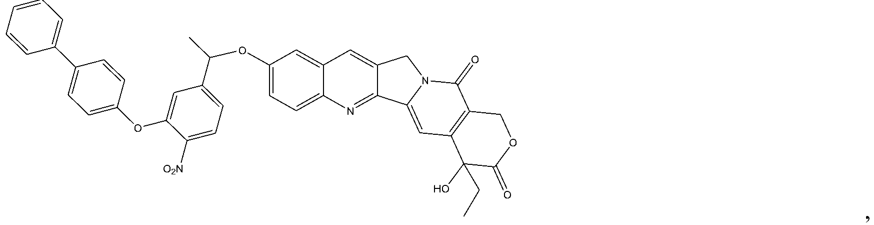

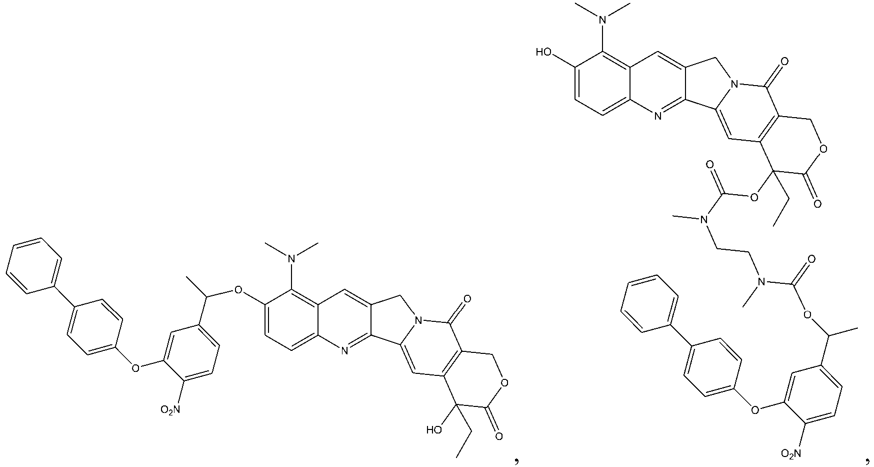

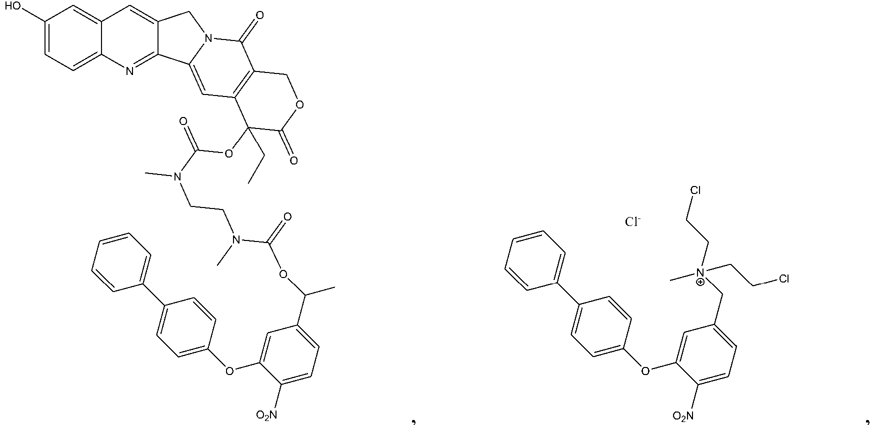

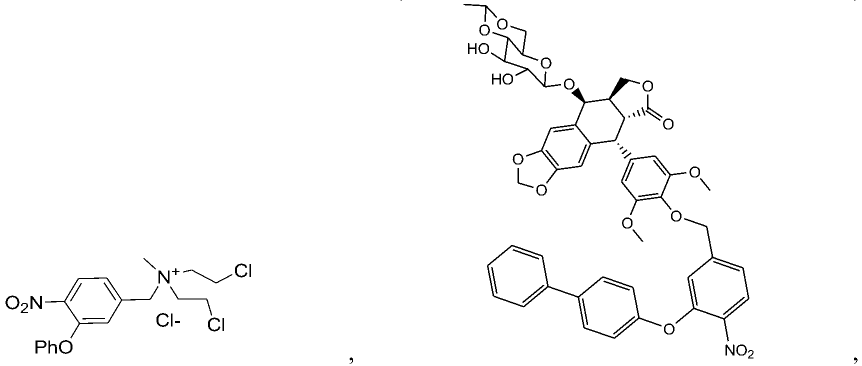

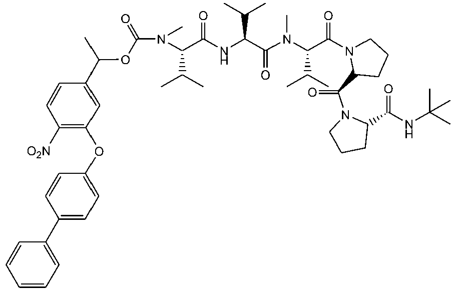

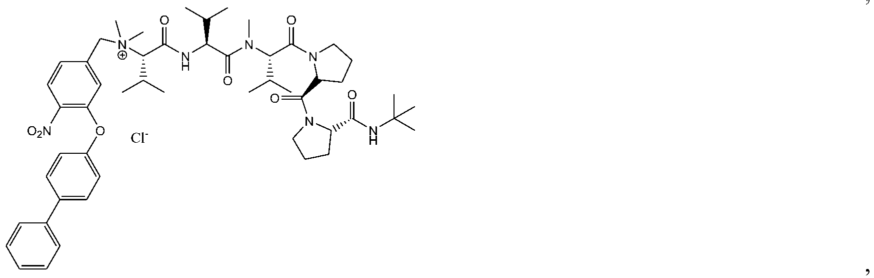

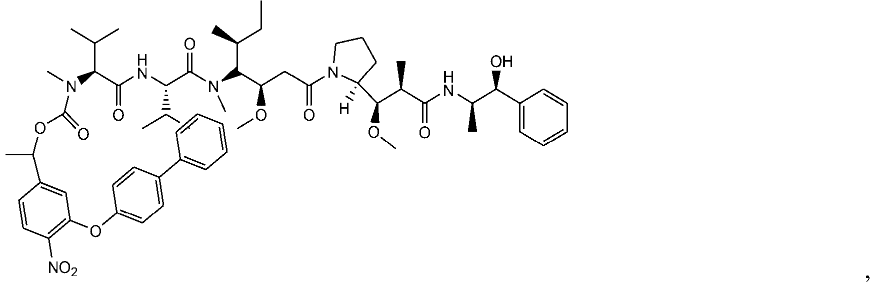

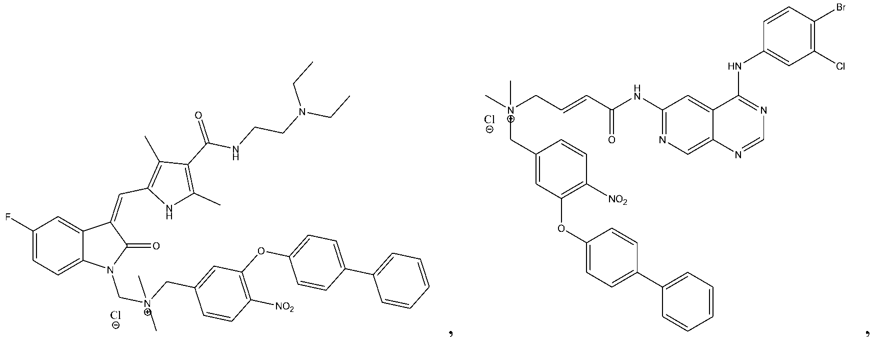

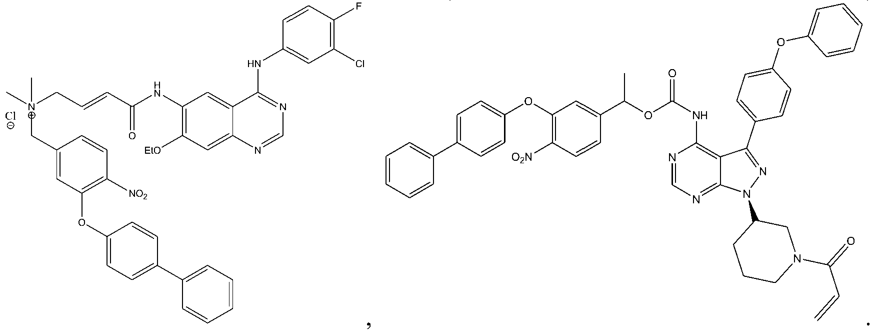

The specific synthetic method of the compound of formula (2) and the corresponding spectral data are disclosed in WO2016161342A1 (corresponding to the Chinese publication text CN108136214A, CN112142692A), which is incorporated herein by reference in their entirety. 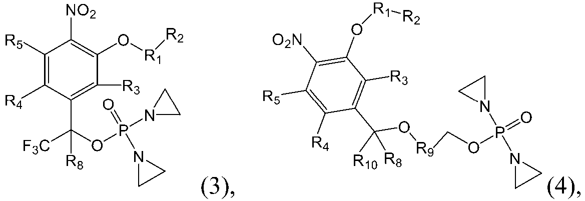 wherein, the definitions of R₁, R₂, R₃, R₄, R₅, R₈, R₉, and R₁₀ are described in the claims of Patent Application PCT/CN2020/089692 with Publication No. WO2020228685A1 (corresponding to Chinese Application No. 2020800358890 with Publication No. CN113853379A), and the synthesis and preparation methods of specific compounds are also described in the above patent application, which is incorporated herein by reference in its entirety. The specific definitions are as follows.
R₁ is C₆-C₁₀ aryl or Z-substituted aryl, 4-15-membered heterocycle or Z-substituted heterocycle, 5-15-membered heteroaryl or Z-substituted heteroaryl, a 7-15-membered fused ring or Z-substituted fused ring;
R₂ is hydrogen, a halogen atom, cyano or isocyano, hydroxy, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, ether having from 1 to 6 carbon atoms or Z-substituted alkoxy having from 1 to 6 carbon atoms, -CONR⁶R⁷, -SO₂NR⁶R⁷, -SO₂R⁶, -OCOO-R⁶, -COOR⁶, -NR⁶COR⁷, -OCOR⁶, -NR⁶SO₂R⁷ or -NR⁶SO₂NR⁶R⁷, or R² together with the atom in the group R¹ to which it is bonded form a 7-15-membered fused ring or Z-substituted fused ring;
R₃ is hydrogen, halogen, cyano or isocyano, hydroxy, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, C₁-C₆ alkoxy or Z-substituted C₁-C₆ alkoxy, -CONR⁶R⁷, -SO₂NR⁶R⁷, -SO₂R⁶, -OCO-R⁶, -OCOO-R⁶, -COOR⁶, -NR⁶COR⁷, -OCOR⁶, or -NR⁶SO₂R⁷;
R₄ and R₅ are each independently hydrogen, a halogen atom, cyano or isocyano, hydroxy, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, C₁-C₆ alkoxy or Z-substituted C₁-C₆ alkoxy, -CONR⁶R⁷, -SO₂NR⁶R⁷, -SO₂R⁶, -OCOO-R⁶, -COOR⁶, -NR⁶COR⁶, -OCOR⁶ or -NR⁶SO₂R⁷, or R⁴ and R⁵ together with the atom in the benzene ring to which they are bonded form a 7-15-membered fused ring or Z-substituted fused ring;
R₆ and R₇ are each independently hydrogen, cyano or isocyano, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, C₁-C₆ alkoxy or Z-substituted C₁-C₆ alkoxy, or R⁶ and R⁷ together with the atom to which they are bonded form 5-7-membered heterocyclyl or Z-substituted 5-7-membered heterocyclyl;
R₈ and R₁₀ are each independently hydrogen, deuterium, aryl or Z-substituted aryl, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, and at least one of R⁸ and R¹⁰ must be hydrogen or deuterium;
R₉ is substituted C₆-C₁₀ aryl which is substituted with at least one fluorine atom or nitro group, substituted 4-15-membered heterocycle which is substituted with at least one fluorine atom or nitro group, or substituted 5-15-membered heteroaryl which is substituted with at least one fluorine atom or nitro group;
the substituent Z is a halogen atom, cyano or isocyano, hydroxy, sulfhydryl, amino, OTs, OMS, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl, an aromatic ring, heterocycle, heteroaromatic ring and fused ring or a substituted aromatic ring, heterocycle, heteroaromatic ring and fused ring, the pattern of substitution being mono- or gem-di-substitution;
in R₉, the substitution in the substituted C₆-C₁₀ aryl, substituted 4-15-membered heterocycle or substituted 5-15-membered heteroaryl is a halogen atom, nitro, cyano or isocyano, hydroxy, amino, C₁-C₃ alkyl or alkoxy, alkenyl, alkynyl, cycloalkyl or benzene ring, substituted benzene ring, C₁-C₃ alkoxy or halogen atom-substituted alkoxy.

The AKR1C3 enzyme-activated prodrug compound of formula (3) is selected from the compounds with the following structural formulae:

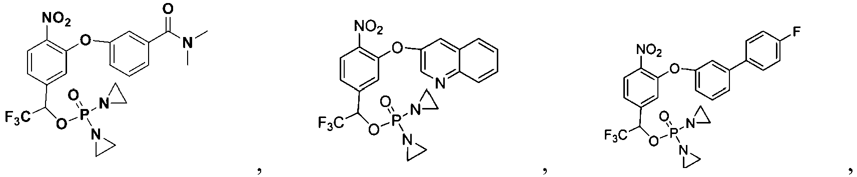

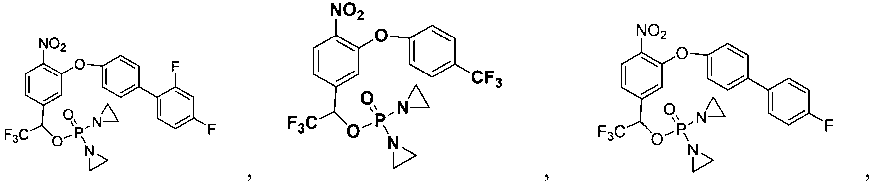

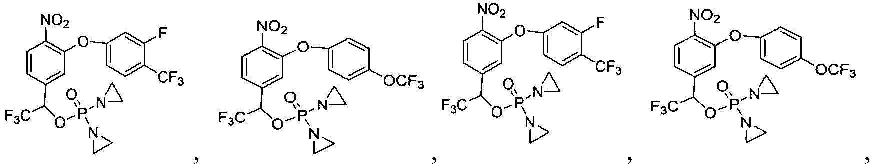

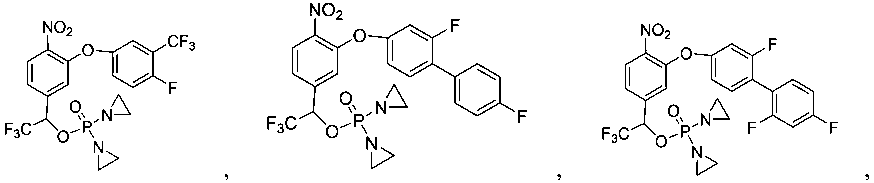

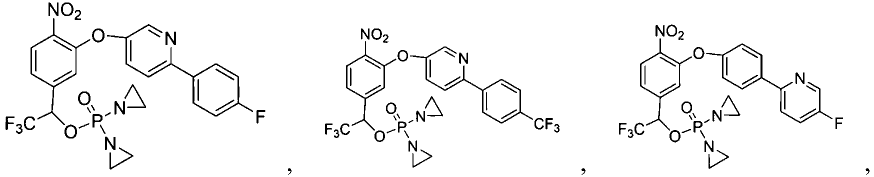

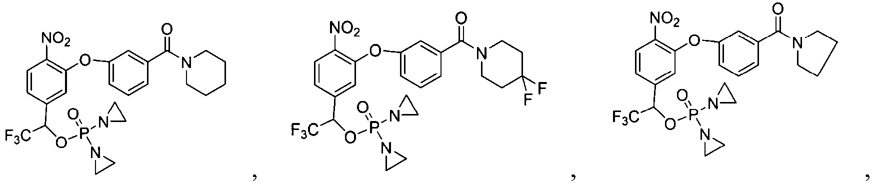

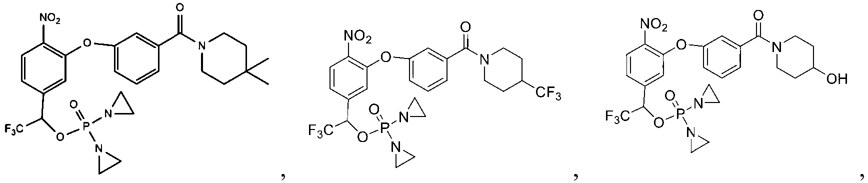

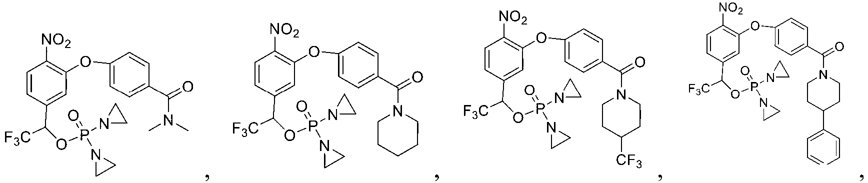

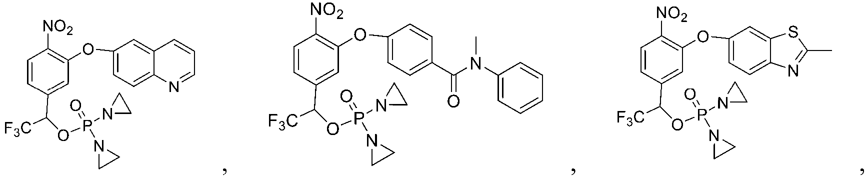

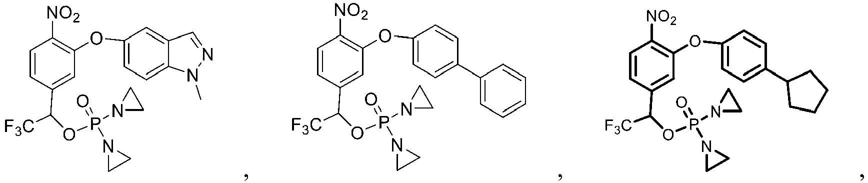

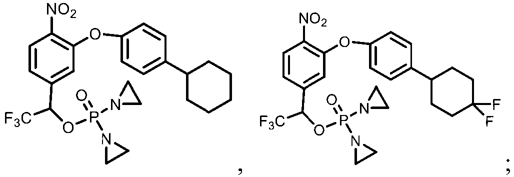

The AKR1C3 enzyme-activated prodrug compound of formula (4) is selected from the compounds with the following structural formulae:

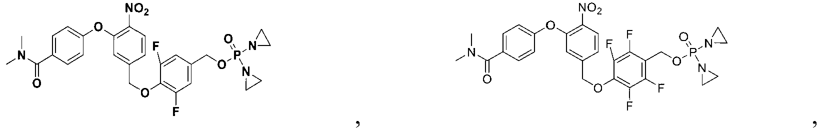

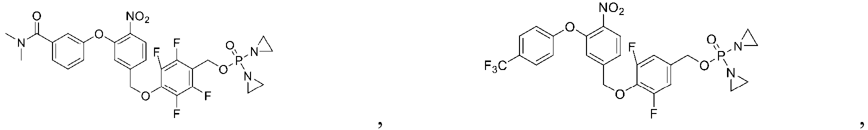

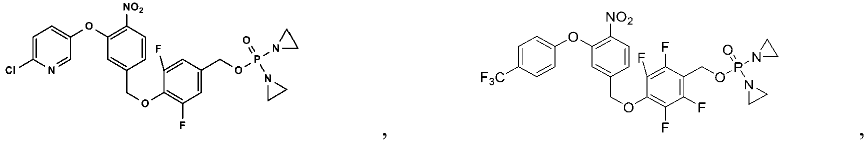

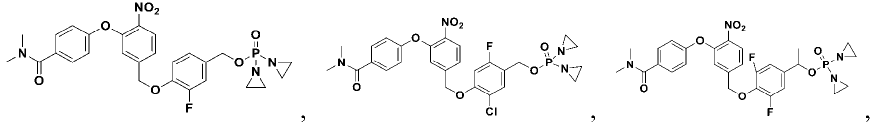

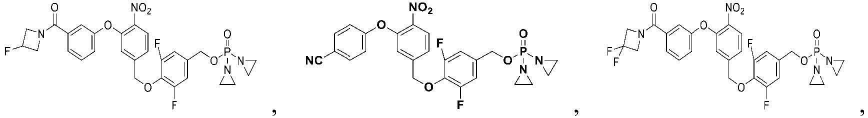

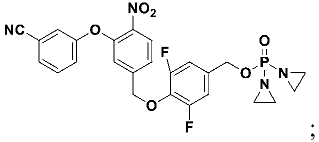

The specific synthetic method of the compounds of formulae (3) and (4), and the corresponding spectral data are disclosed in WO2020228685A1 (corresponding to the Chinese publication text CN113853379A), which is incorporated herein by reference in their entirety. 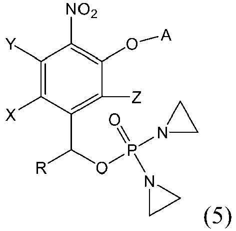 wherein:
A is a substituted or unsubstituted C₆-C₁₀ aryl, a biaryl or substituted biaryl, a 5-15-membered heteroaryl, or -N=CR¹R²; wherein the substituent is selected from the group consisting of halogeno, -CN, -NO₂, -O-(CH₂)-O-, -CO₂H and salt thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are bonded form a 5-7-membered heterocycle;
wherein the alkyl group and the aryl group are each substituted by 1-3 halogeno groups or 1-3 C₁-C₆ alkyl groups;
R¹ and R² are each independently phenyl or methyl;
X, Y and Z are each independently hydrogen or halogeno; and
R is hydrogen or C₁-C₆ alkyl or halogen-substituted alkyl.

The AKR1C3 enzyme-activated prodrug compound of formula (5) is selected from the compounds with the following structural formulae: 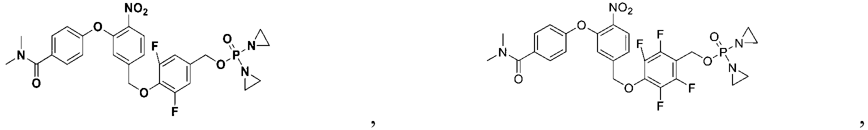 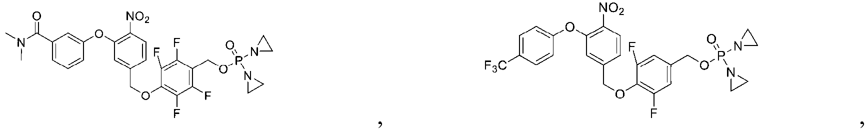 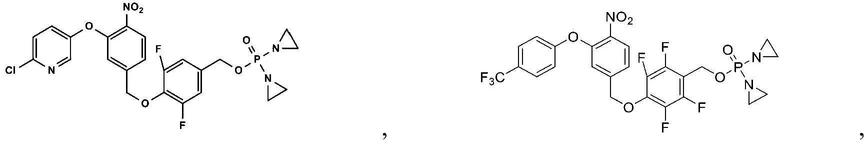 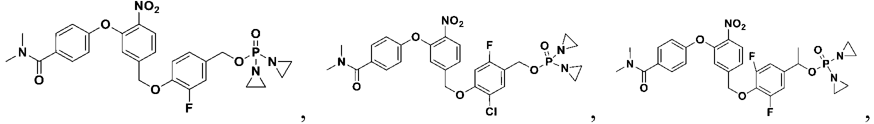 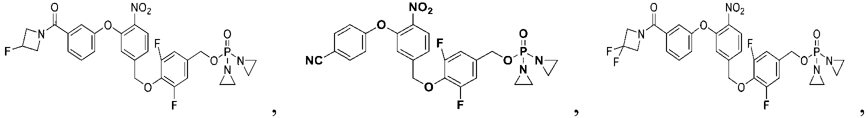 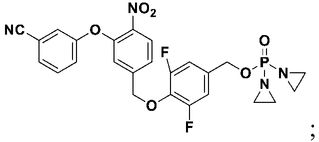 and 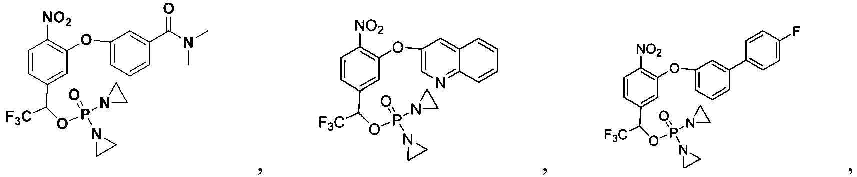 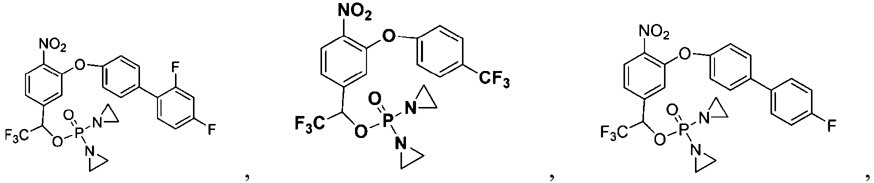 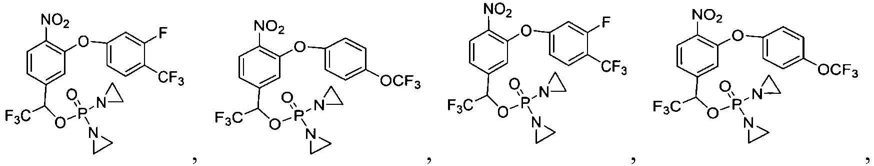 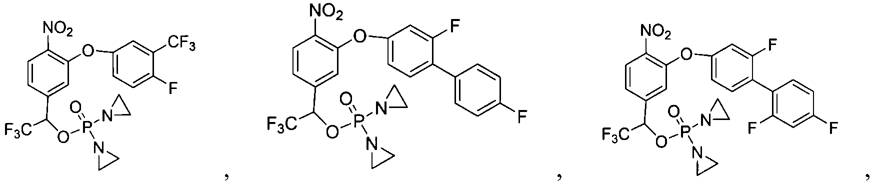 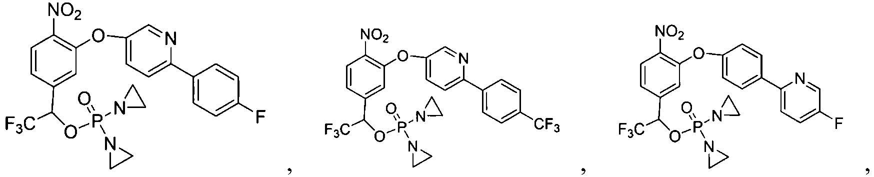 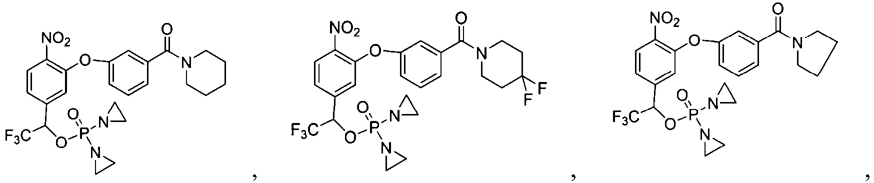 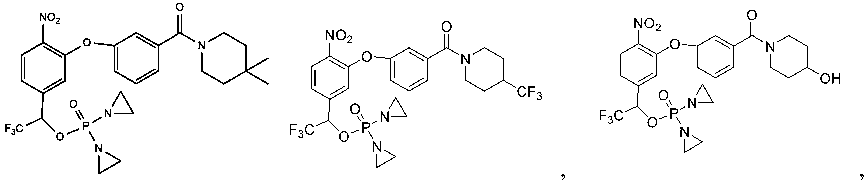 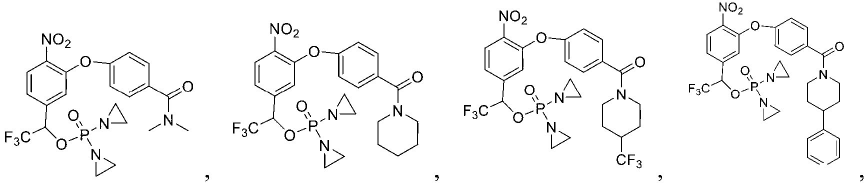 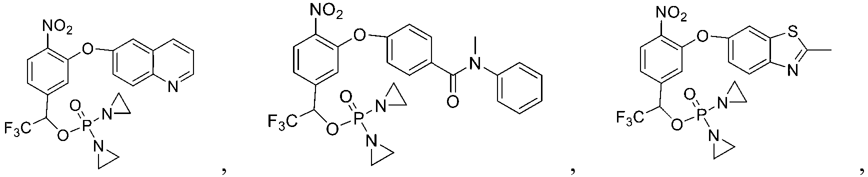 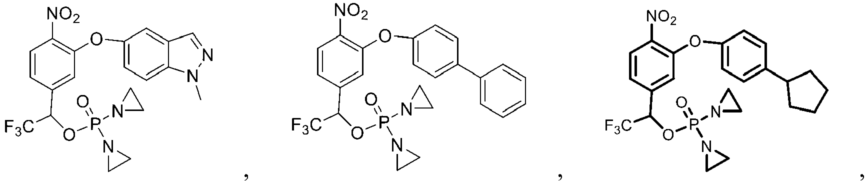 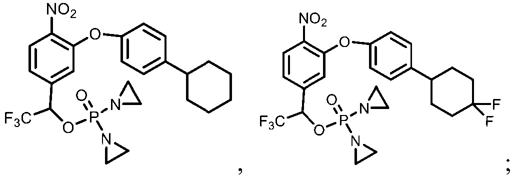 and 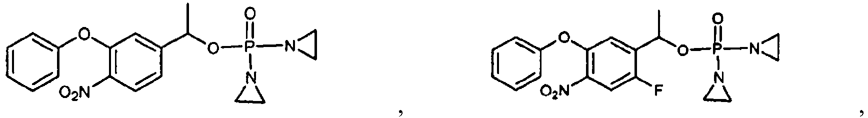 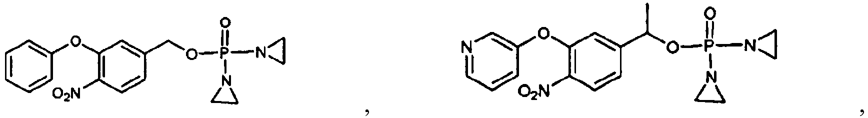 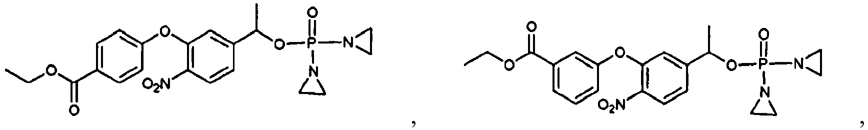 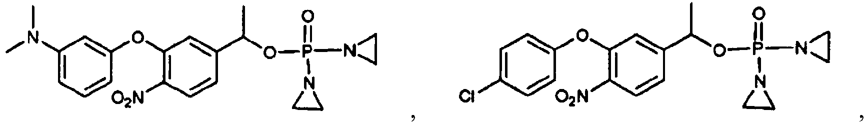 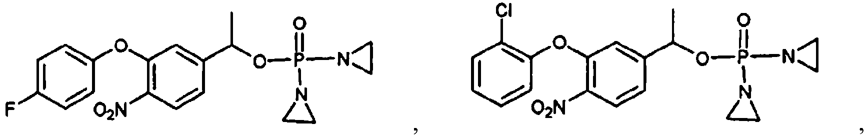 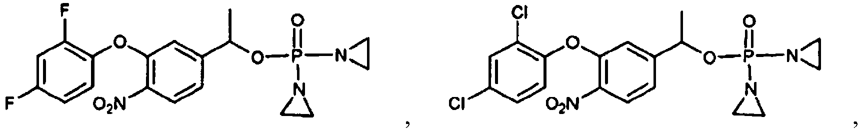 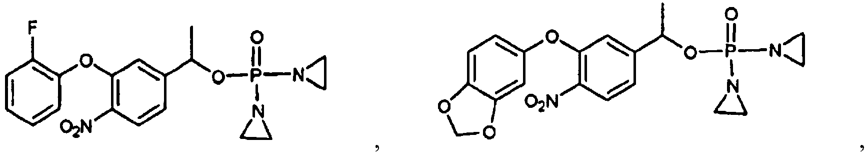 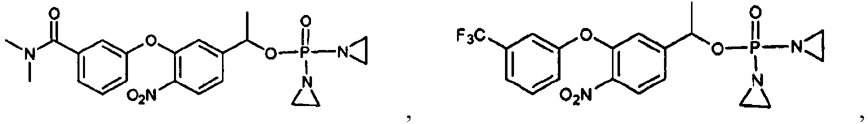 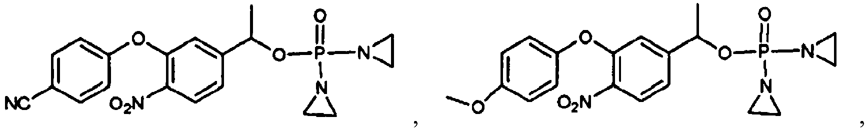 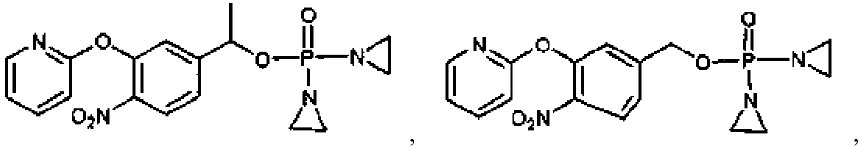 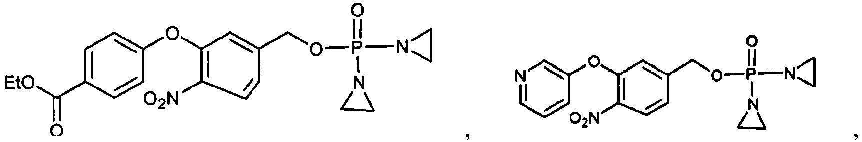 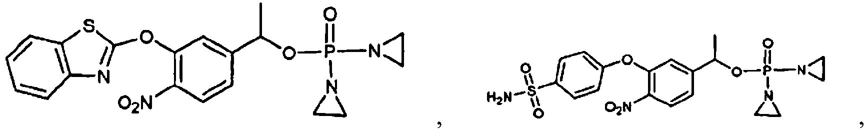 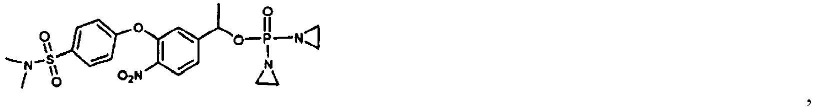 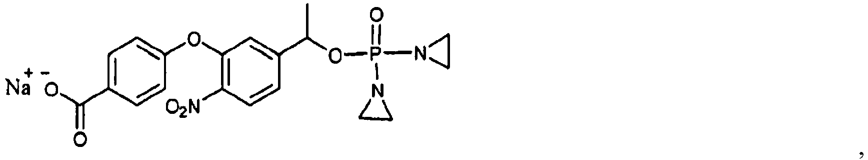 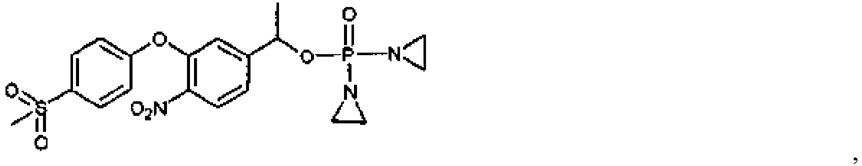 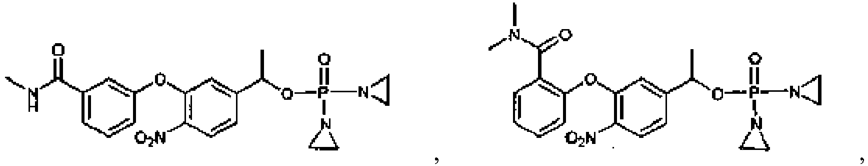 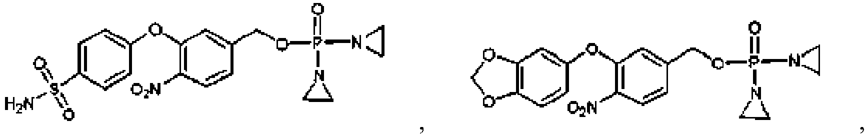 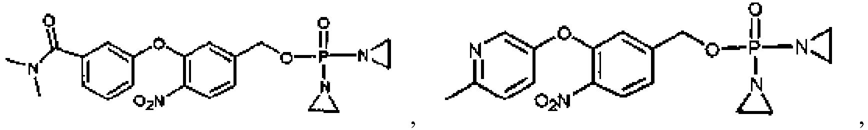 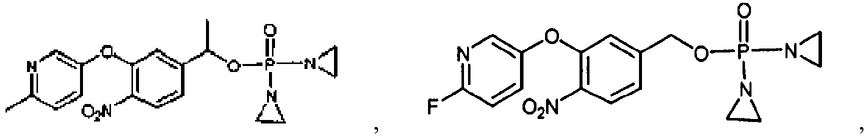 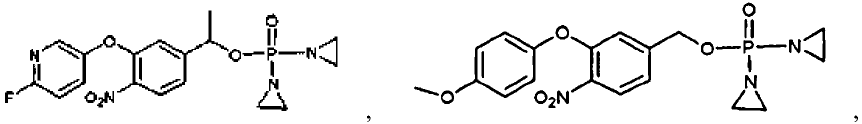 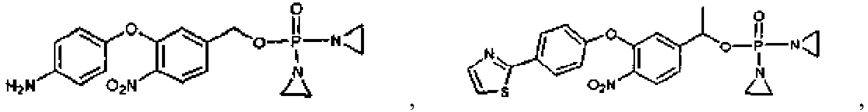 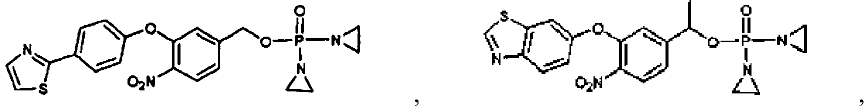 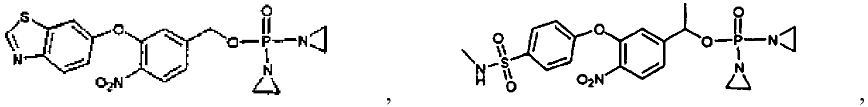 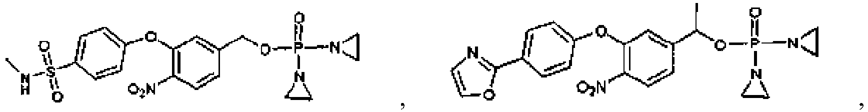 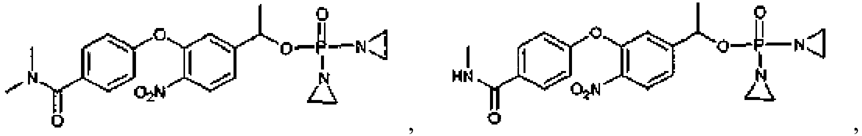 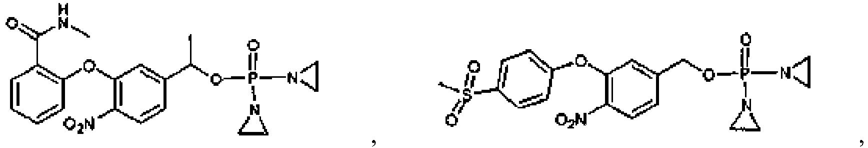 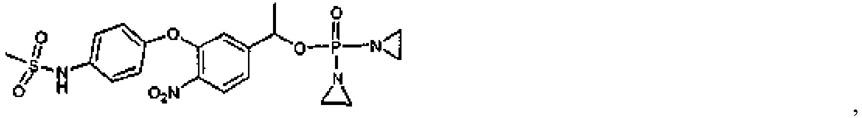 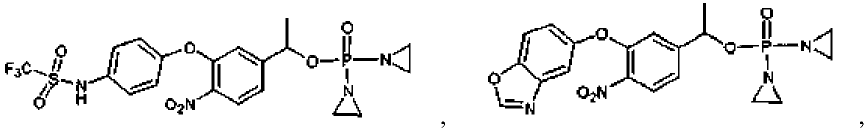 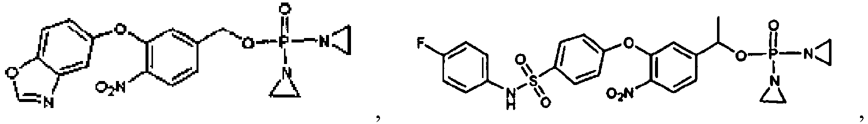 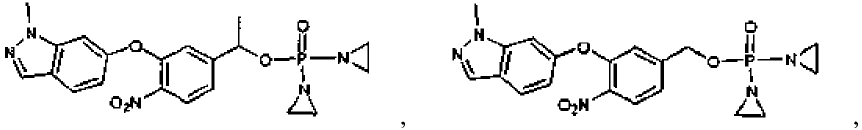 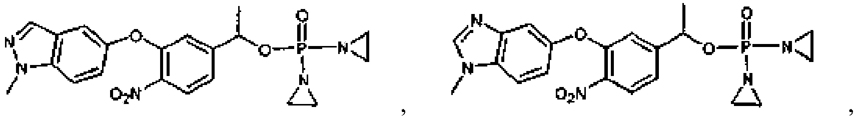 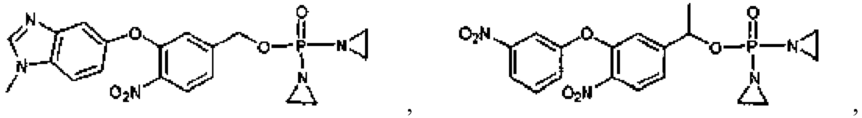 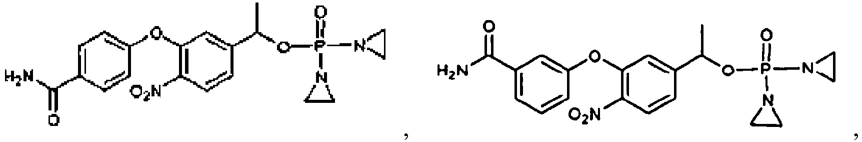 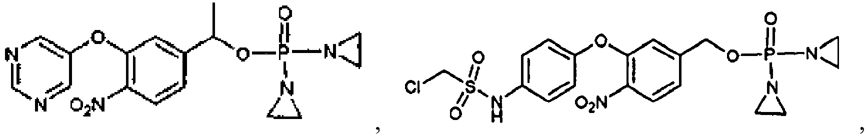 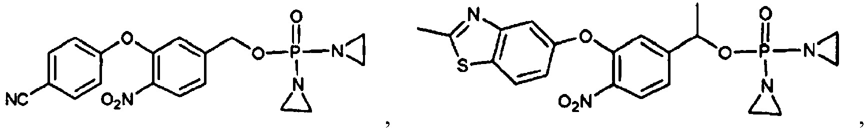 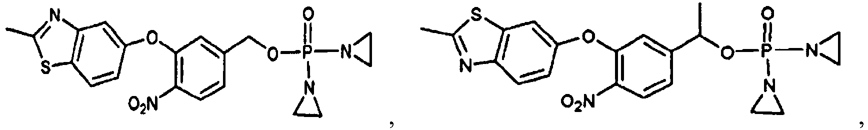 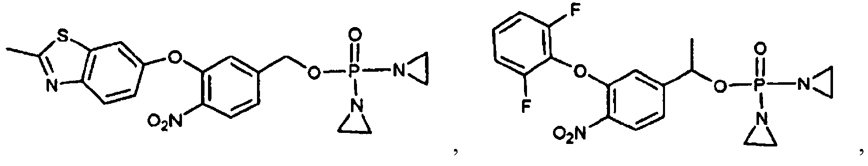 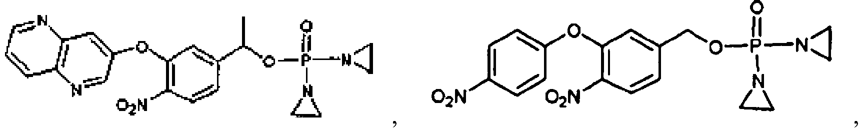 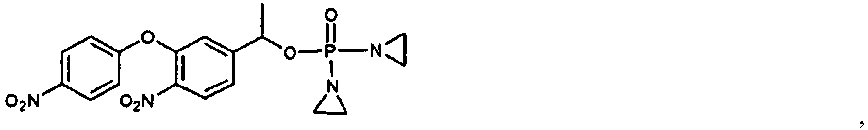 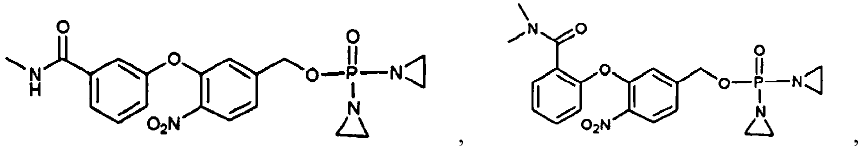 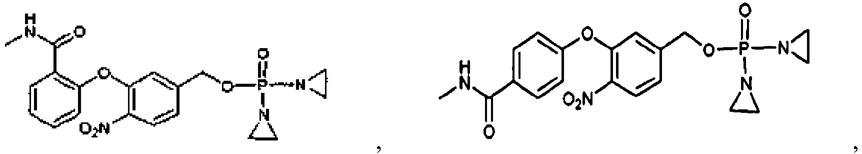 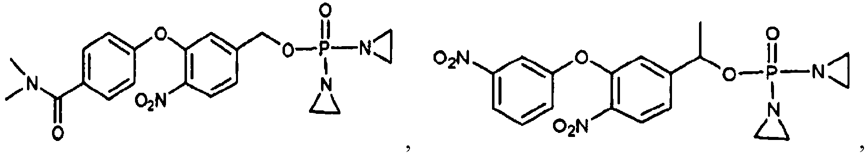 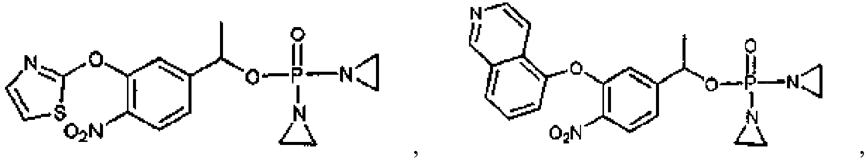 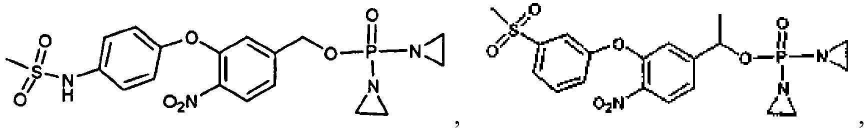 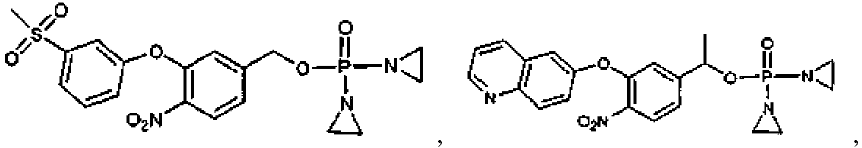 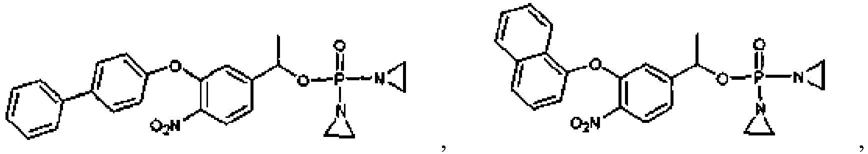 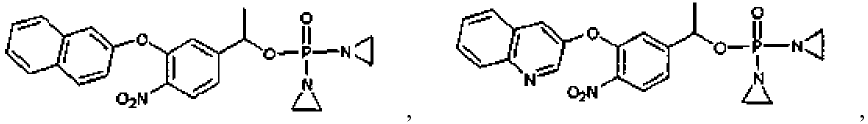 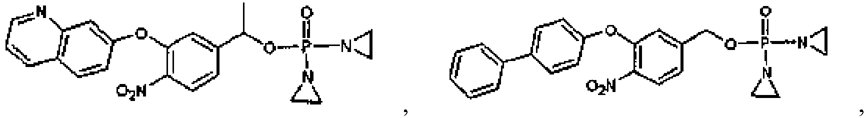 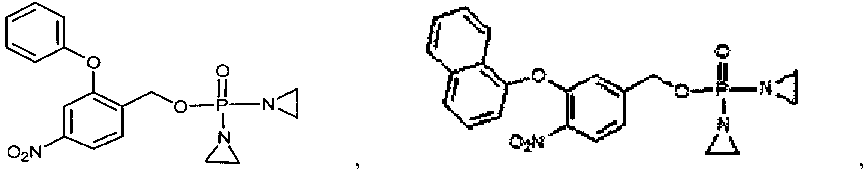 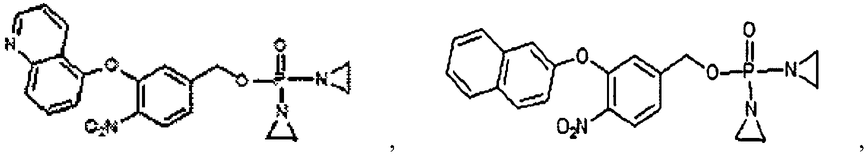 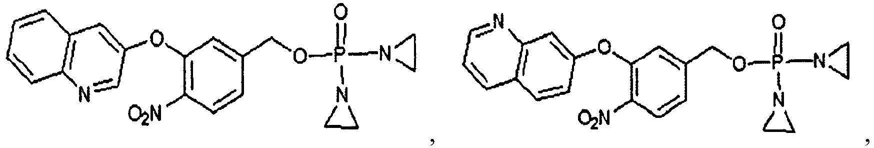 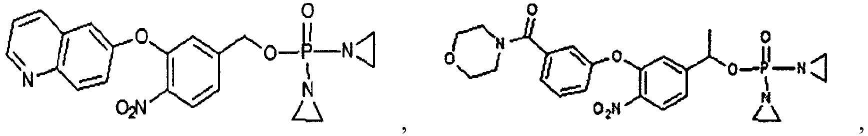 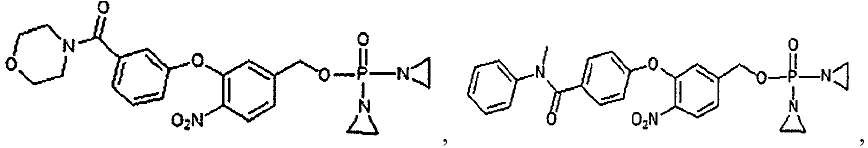 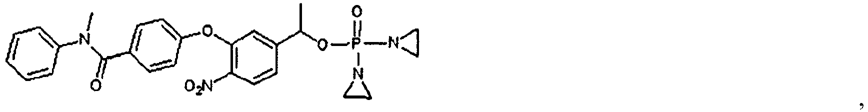 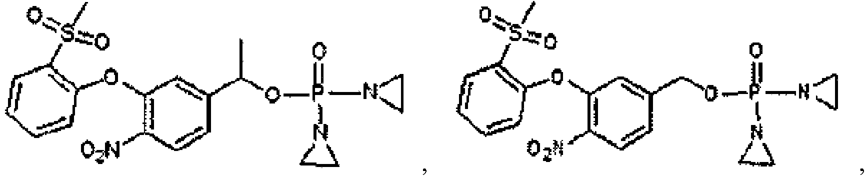 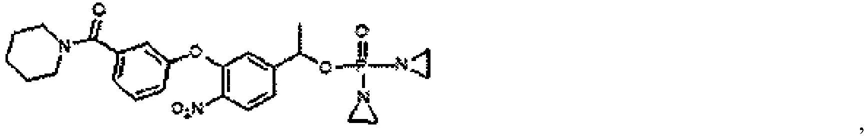 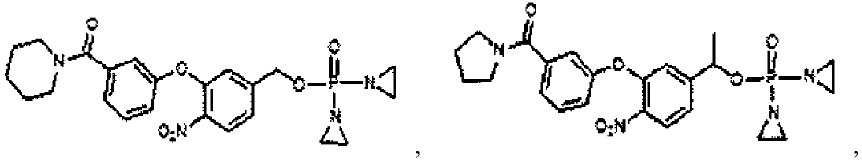 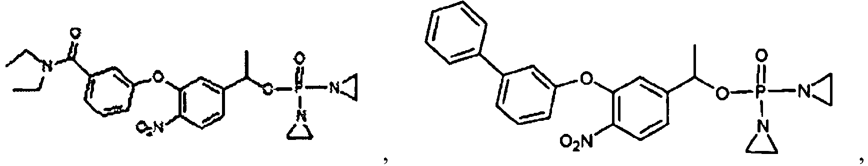 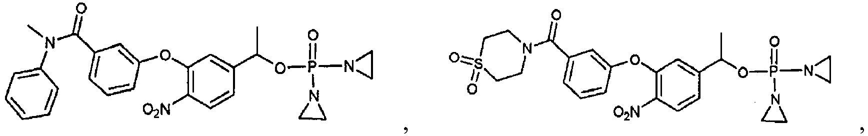 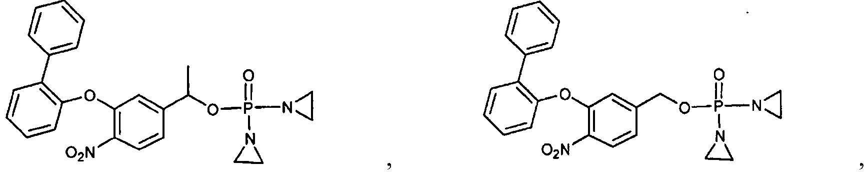 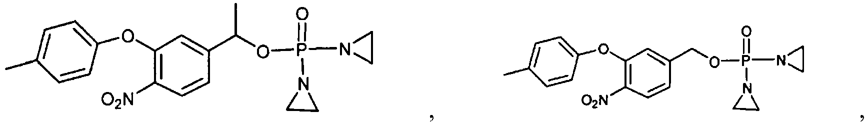 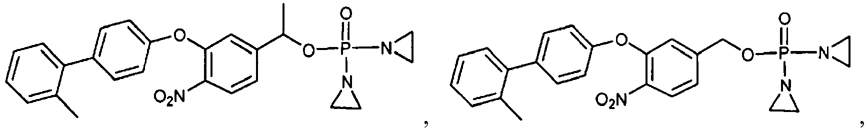 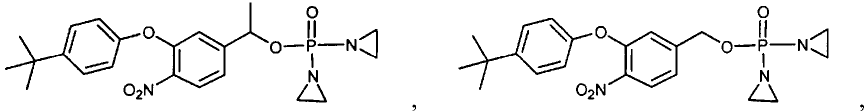 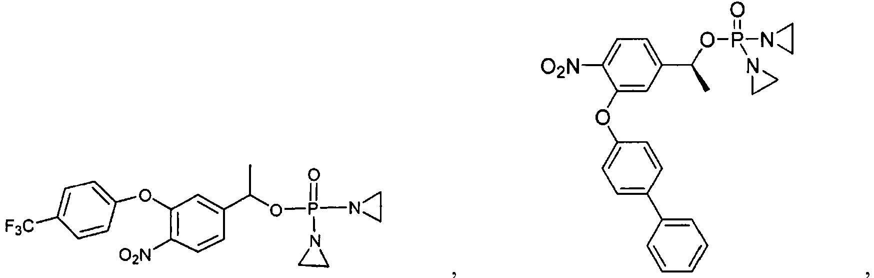 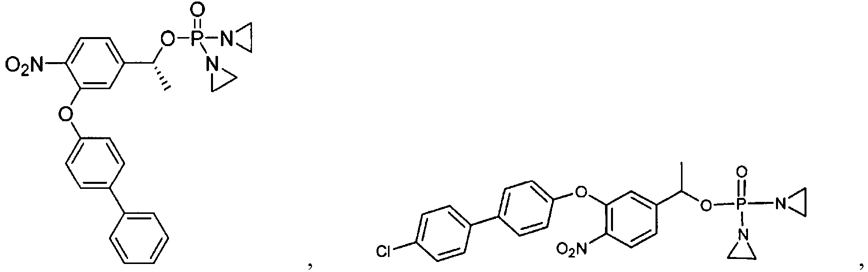 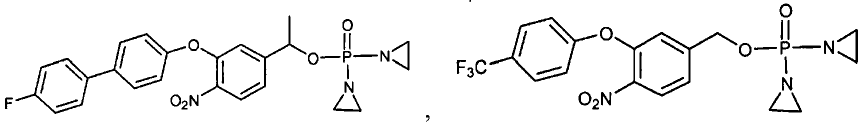 and

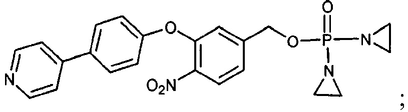

The specific synthetic method of the compound of formula (5) and the corresponding spectral data are disclosed in WO2020228685A1 (corresponding to the Chinese publication text CN113853379A) and WO2016145092A1 (corresponding to the Chinese publication text CN107530556A), which are incorporated herein by reference in their entirety. 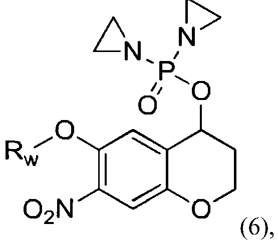 wherein the definition of Rw is described in the claims of Patent Application PCT/CN2020/120281 with Publication No. WO2021068952A1 (corresponding to the Chinese Application No. 202080071652.8 with Publication No. CN114555574A), and the synthesis and preparation methods of specific compounds are also described in the above patent application, which is incorporated herein by reference in its entirety. The specific definitions are as follows.
Rw is 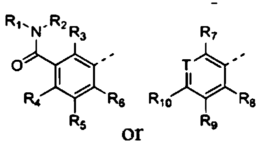
R₁ is H, C₁₋₆ alkyl, C₃₋₆ cycloalkyl, 4-6-membered heterocycloalkyl, 5-6-membered heteroaryl or phenyl, wherein the C₁₋₆ alkyl, C₃₋₆ cycloalkyl, 4-6-membered heterocycloalkyl, 5-6-membered heteroaryl and phenyl are optionally substituted with 1, 2 or 3 R^{a} groups;
each R^{a} is independently H, F, Cl, Br, I, -CN, -OH, C₁₋₃ alkoxy or C₁₋₃ alkyl;
R₂ is H or C₁₋₆ alkyl;
or R₁ and R₂, together with the N atom to which they are bonded, form a 4-6-membered heterocycloalkyl, wherein the 4-6-membered heterocycloalkyl is optionally substituted with 1, 2 or 3 R^{b} groups;
each R^{d} is independently H, F, Cl, Br, I, -CN, -OH, -NH₂, -OCH₃, -OCH₂CH₃, -CH₃ or -CH₂CH₃;
R₃ is H, F, Cl, Br, I, -OH, -NH₂, C₁₋₃ alkoxy or C₁₋₃ alkyl;
or R₂ and R₃ are attached together to make the structural unit 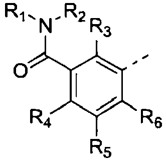 to be 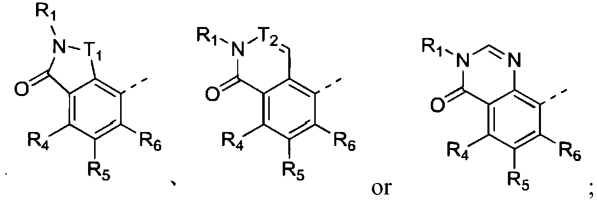
T₁ is -(CR^{c}R^{d})ₘ- or -(CR^{c}R^{d})ₙ-O-;
m is 1, 2 or 3;
n is 1 or 2;
T₂ is N or CH;
R^{c} and R^{d} are each independently H, F, C₁₋₃ alkyl or C₁₋₃ alkoxy;
R₄, R₅ and R₆ are each independently H, F, Cl, Br, I, C₁₋₃ alkyl or C₁₋₃ alkoxy;
T is N or CH;
R₇ and R₈ are each independently H, F, Cl, Br or I;
R₉ and R₁₀ are each independently H, F, Cl, Br, I, -CN; or
the 4-6-membered heterocycloalkyl and 5-6-membered heteroaryl each contain 1, 2, 3 or 4 heteroatoms independently selected from N, -O- and -S-.

The AKR1C3 enzyme-activated prodrug compound of formula (6) is selected from the compounds with the following structural formulae: 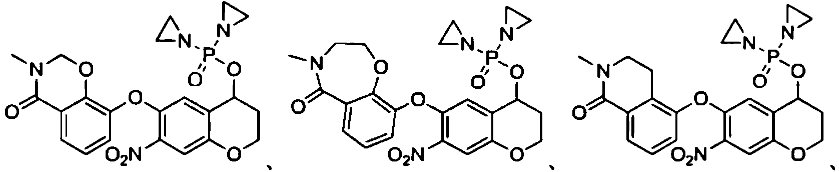 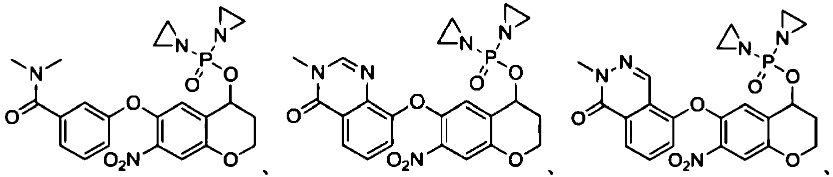 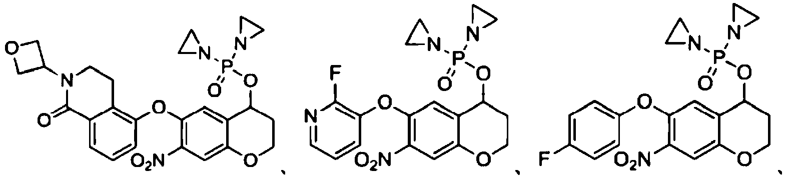 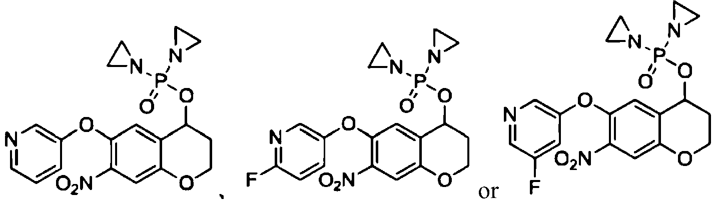 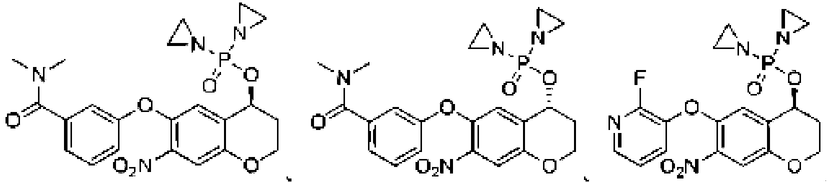 or

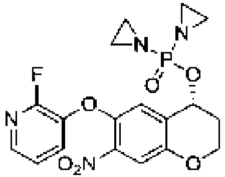

The specific synthetic method of the compound of formula (6) and the corresponding spectral data are disclosed in WO2021068952A1 (corresponding to the Chinese publication text CN114555574A), which is incorporated herein by reference in their entirety. 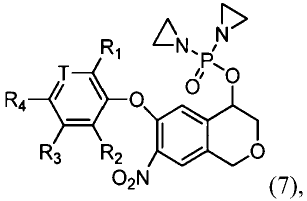 wherein the definitions of R₁, R₂, R₃, R₄, and T are described in the claims of Patent Application PCT/CN2021/118597 with Publication No. WO2022057838A1, and the synthesis and preparation methods of specific compounds are also described in the above patent application, which is incorporated herein by reference in its entirety. The specific definitions are as follows.
T is N or CH;
R₁ and R₂ are each independently H, F, Cl, Br, I, or C₁₋₃ alkyl, wherein the C₁₋₃ alkyl is optionally substituted with 1, 2, or 3 Rₐ groups;
each Rₐ is independently F, Cl, Br, I, -CN, -OH, or -NH₂;
R₃ and R₄ are each independently H, F, Cl, Br, I, CN, C₁₋₃ alkyl, C₁₋₃ alkoxy, 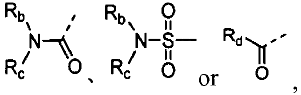 wherein the C₁₋₃ alkyl is optionally substituted with 1, 2, or 3 Rₑ groups;
R_{b} and R_{c} are each independently H, -CH₃, -CH₂CH₃, -(CH₂)₂CH₃, or -CH(CH₃)₂;
R_{d} is -CH₃, -CH₂CH₃, -(CH₂)₂CH₃, or -CH(CH₃)₂;
each Rₑ is independently F, Cl, Br, I, -CN, -OH, or -NH₂.

The AKR1C3 enzyme-activated prodrug compound of formula (7) is selected from the compounds with the following structural formulae:

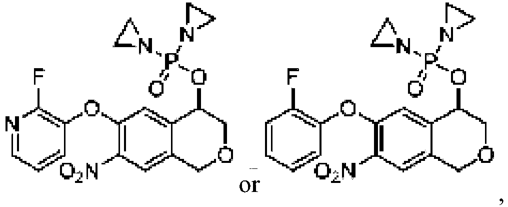

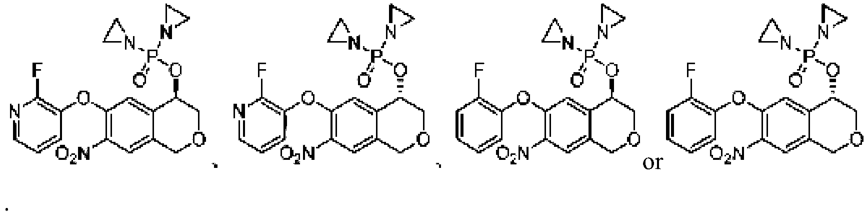

The specific synthetic method of the compound of formula (7) and the corresponding spectral data are disclosed in WO2022057838A1, which is incorporated herein by reference in their entirety. 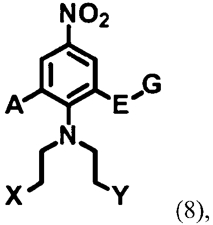 wherein the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A), and the synthesis and preparation methods of specific compounds are also described in the above patent application, which is incorporated herein by reference in its entirety. The specific definitions are as follows.
A is H, C₁-C₆ alkyl, C₁-C₆ alkenyl, C₁-C₆ alkynyl, CFH₂, CF₂H, CF₃, F, Cl, Br, I, OCF₃, COR or CON(R)₂;
E is SO or SO₂;
X is Cl, Br, I or OSO₂R;
Y is Cl, Br, I or OSO₂R;
each R is independently H or C₁-C₆ alkyl;
G is a radical group selected from the group consisting of formulae (B)-(AA): 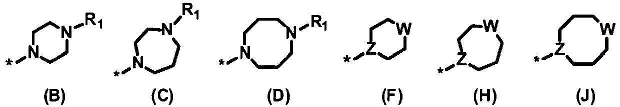 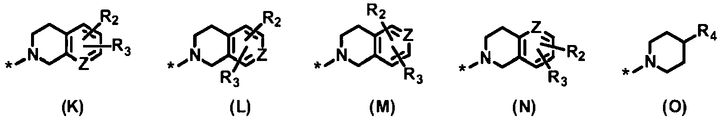 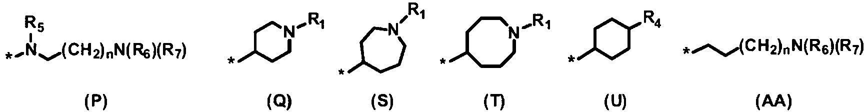 wherein:
   R₁ is H, C₁-C₆ alkyl, CH₂(CH₂)ₙOH, CH₂CH(OH)CH₂OH, phenyl, pyridyl, benzyl, or pyridylmethyl, provided that when R₁ is phenyl, pyridyl, benzyl or pyridylmethyl, R₁ is optionally substituted at any available position with C₁-C₆ alkyl, C₁-C₆ alkenyl, C₁-C₆ alkynyl, OR₆, N(R₆)(R₇), CFH₂, CF₂H, CF₃, F, Cl, Br, I, OCF₃, COR₆, CON(R₆)(R₇), SOR₆, SON(R₆)(R₇), SO₂R₆, SO₂N(R₆)(R₇), CN, or NO₂;
   R₂ and R₃ are each independently H, C₁-C₆ alkyl, C₁-C₆ alkenyl, C₁-C₆ alkynyl, OR₆, N(R₆)(R₇), CFH₂, CF₂H, CF₃, F, Cl, Br, I, OCF₃, COR₆, CON(R₆)(R₇), SOR₆, SON(R₆)(R₇), SO₂R₆, SO₂N(R₆)(R₇), CN or NO₂;
   R₄ is N(R₆)(R₇), OH, OCH₂(CH₂)ₙN(R₆)(R₇) or CH₂(CH₂)ₙN(R₆)(R₇);
   R₅ is H or a C₁-C₆ alkyl group;
   R₆ and R₇ are each independently H or C₁₋₆ alkyl, or R₆ and R₇ together form a substituted or unsubstituted 5-membered or 6-membered heterocycle;
   Z is CH or N;
   W is CH₂, O, S, SO or SO₂;
   n is 0 to 6;
   * represents a point of attachment to formula (I).

The AKR1C3 enzyme-activated prodrug compound of formula (8) is selected from the compounds with the following structural formulae:

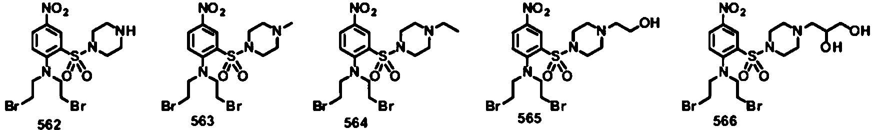

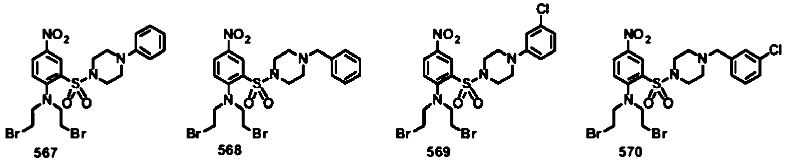

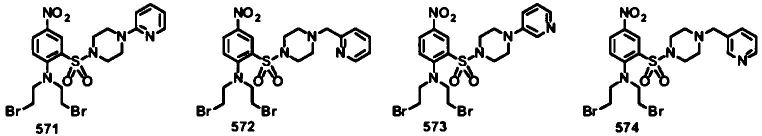

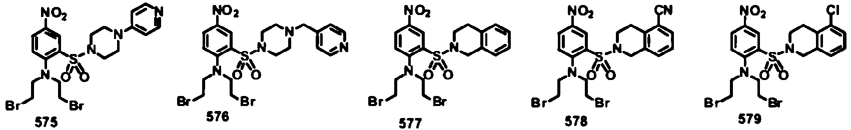

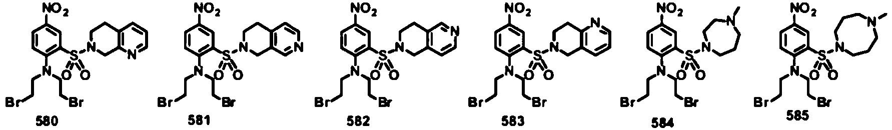

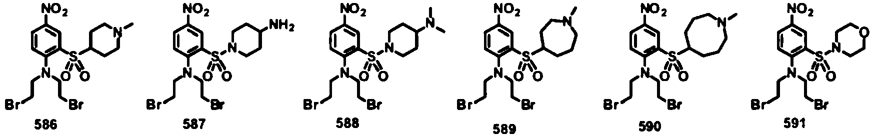

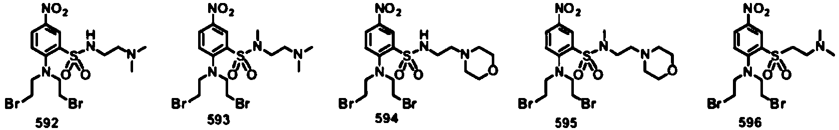

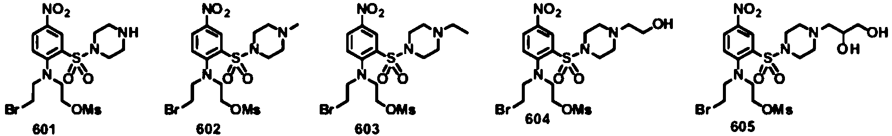

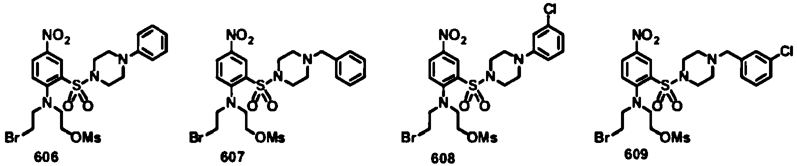

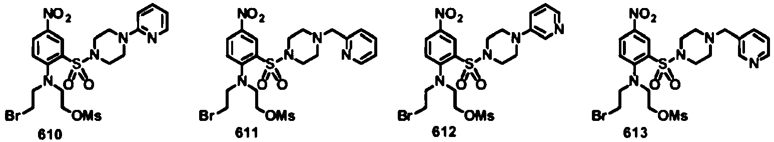

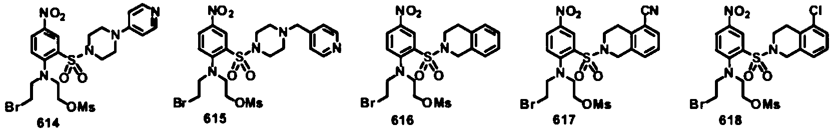

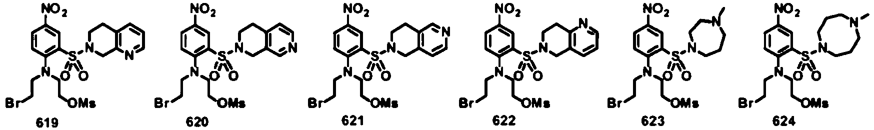

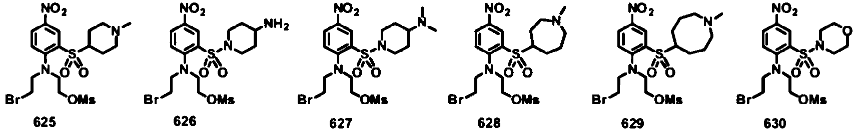

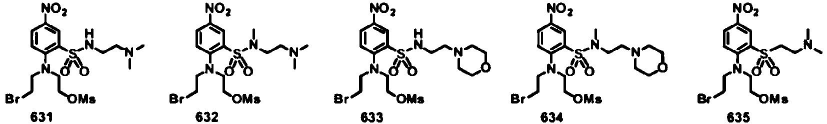

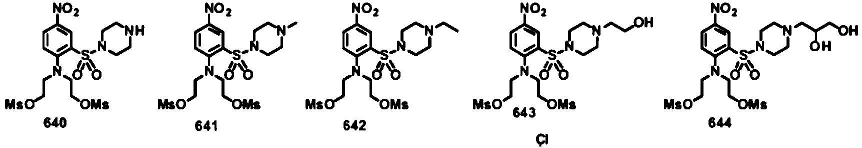

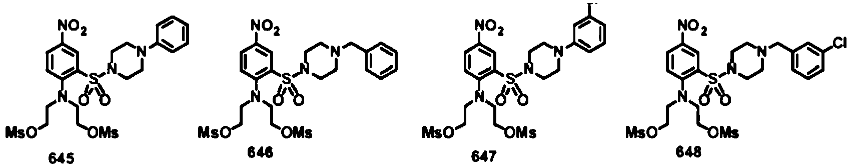

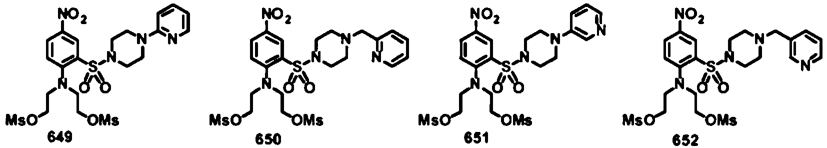

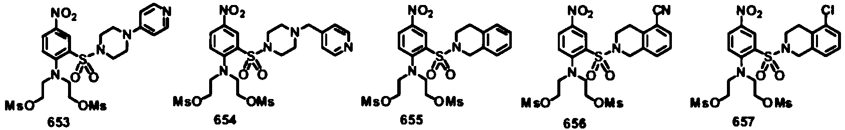

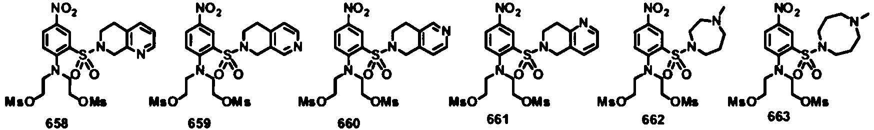

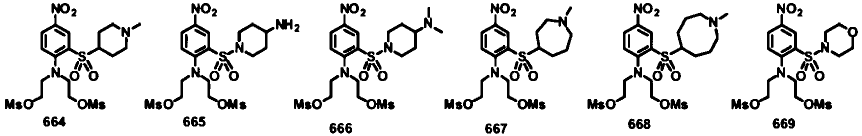

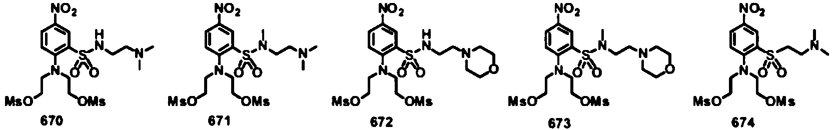

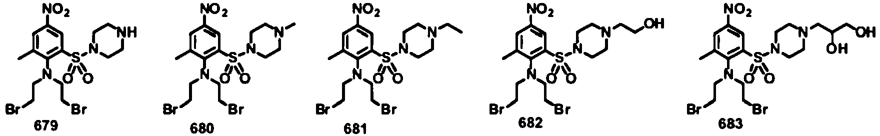

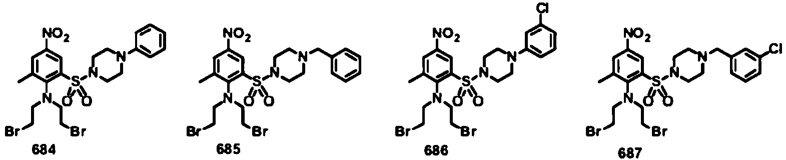

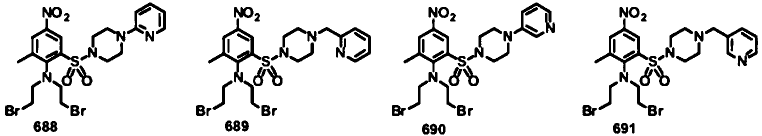

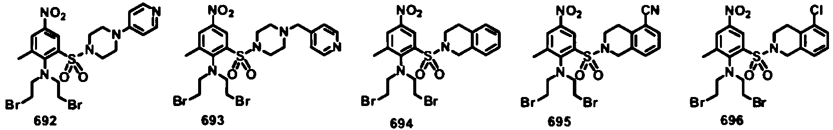

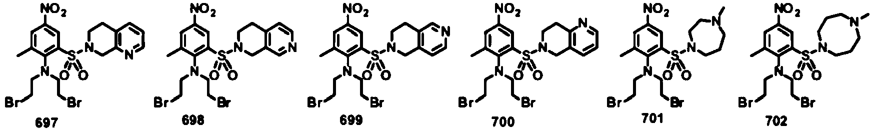

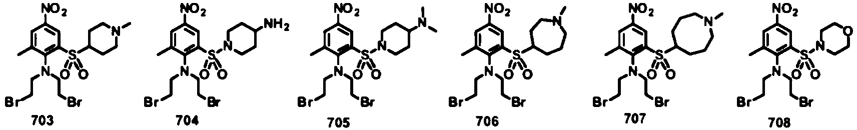

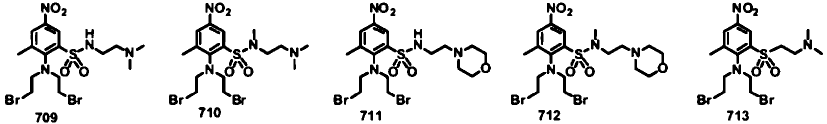

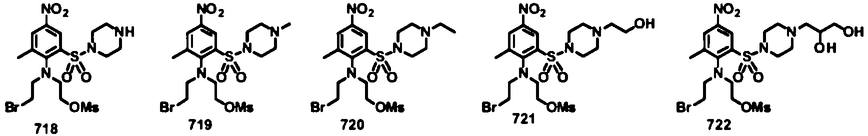

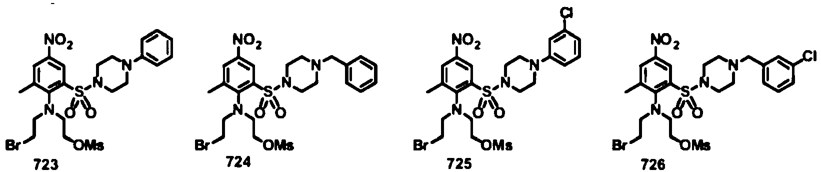

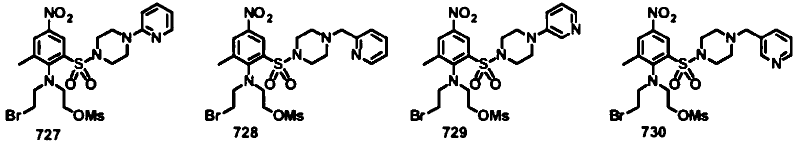

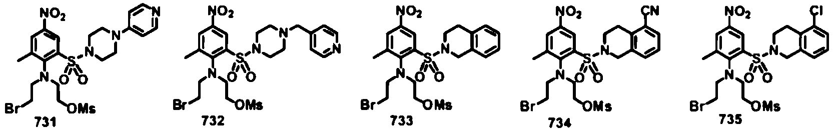

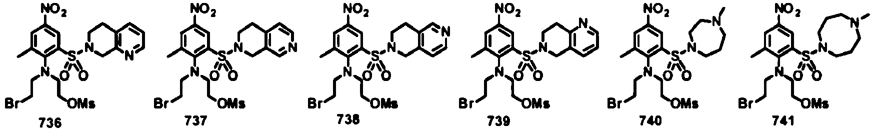

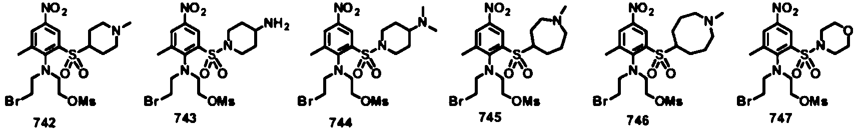

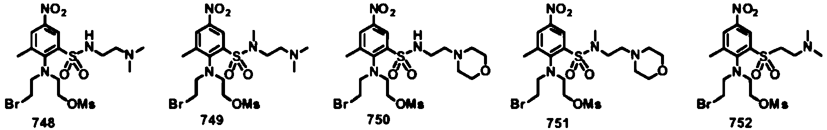

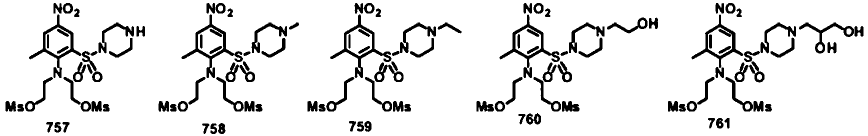

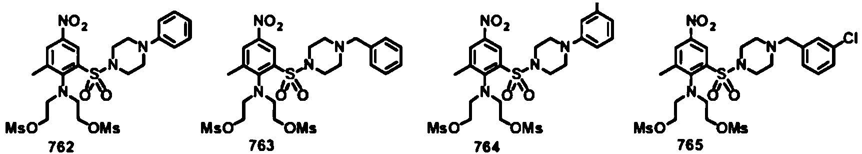

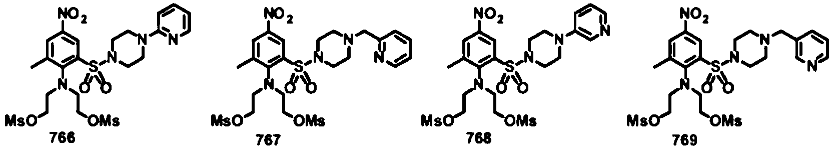

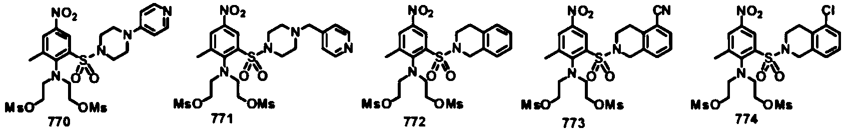

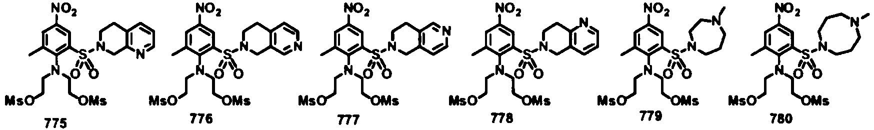

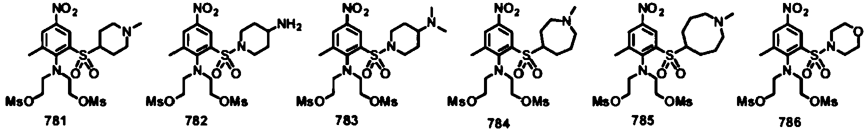

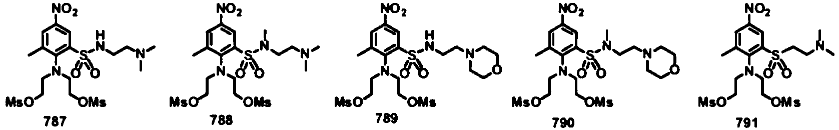

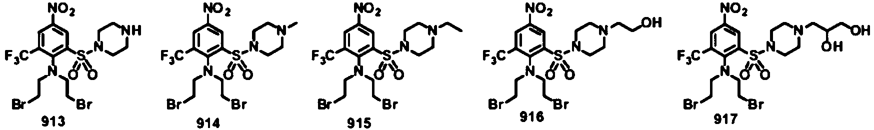

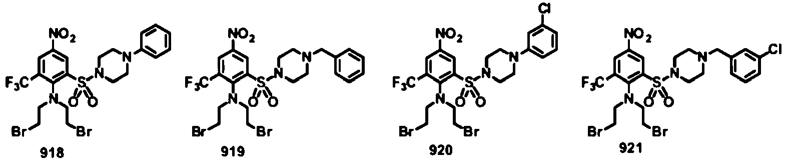

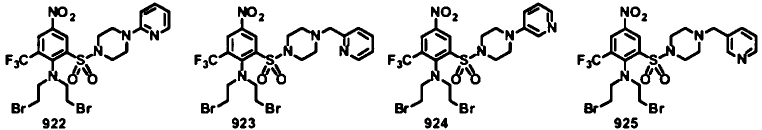

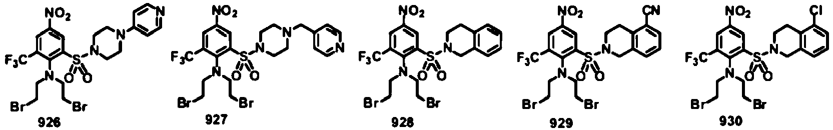

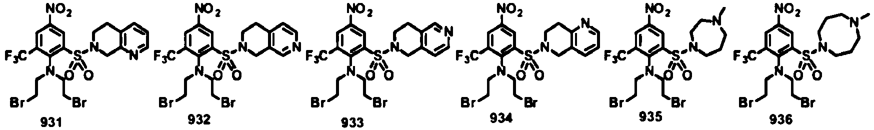

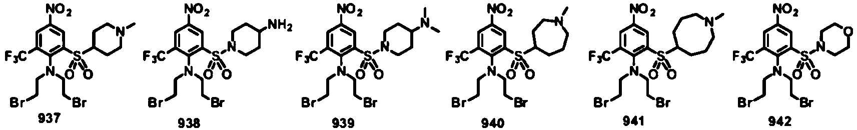

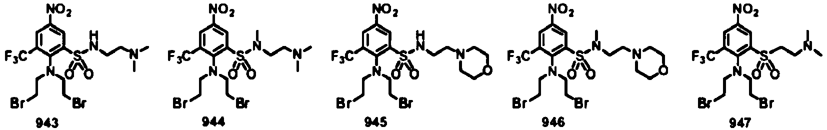

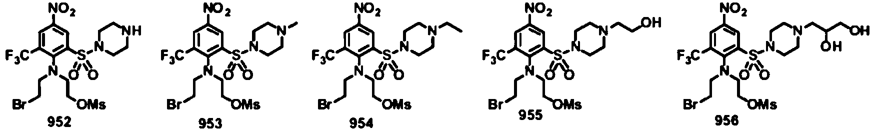

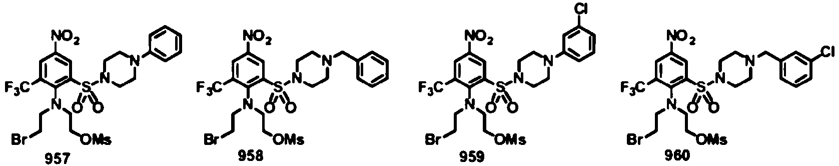

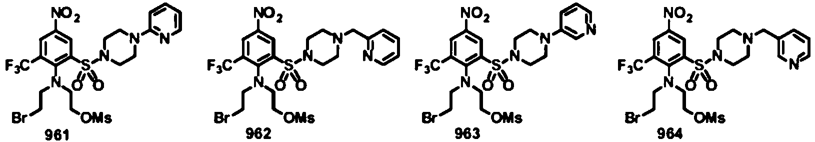

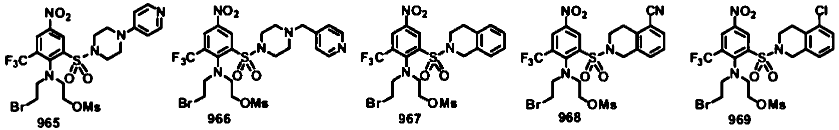

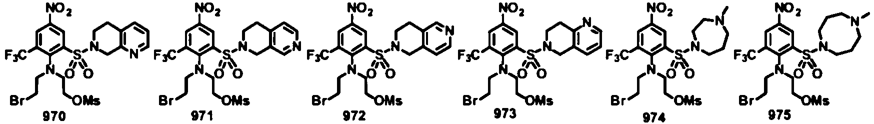

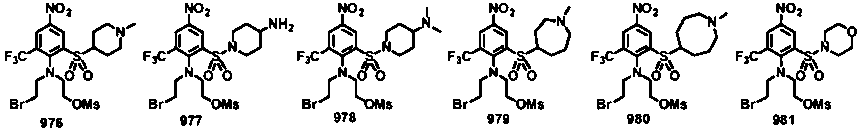

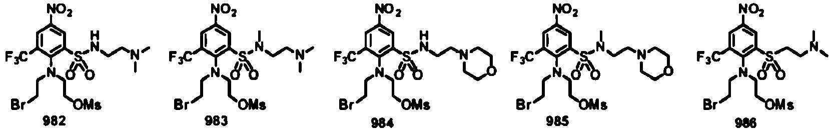

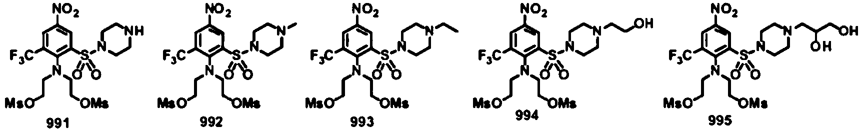

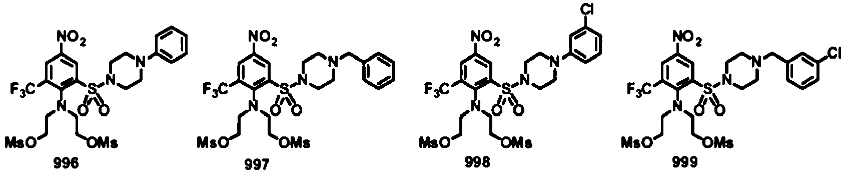

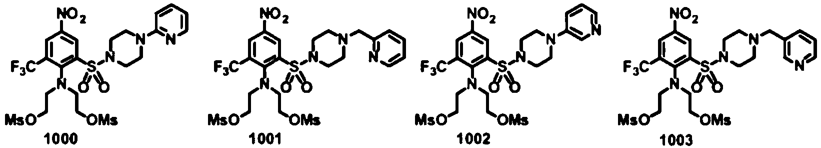

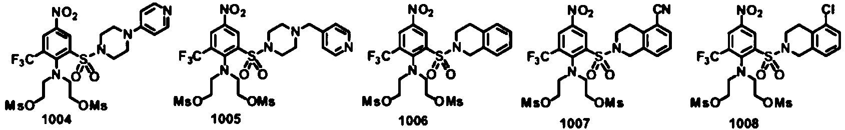

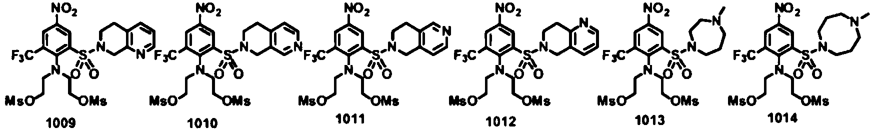

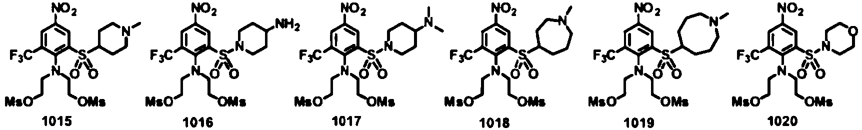

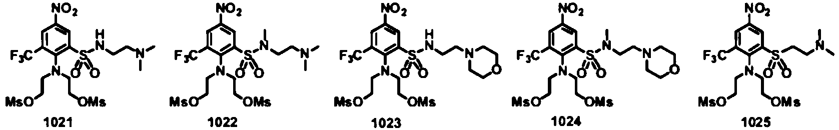

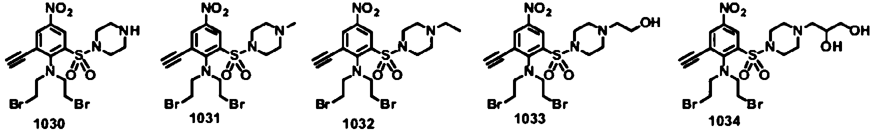

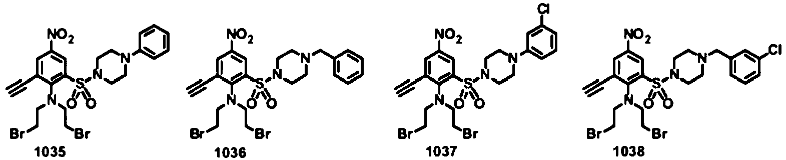

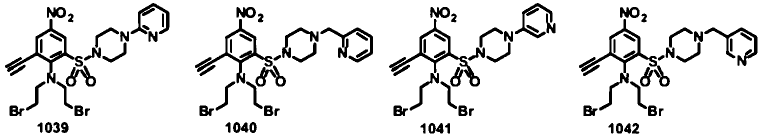

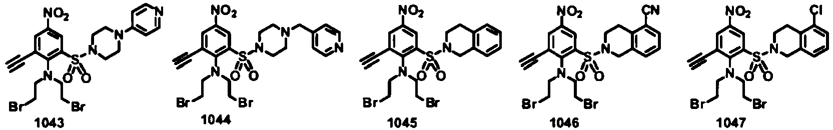

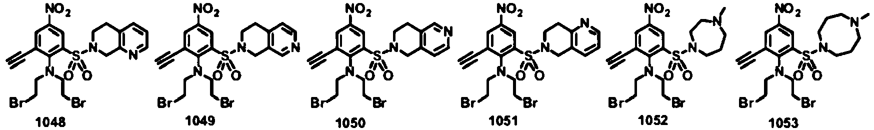

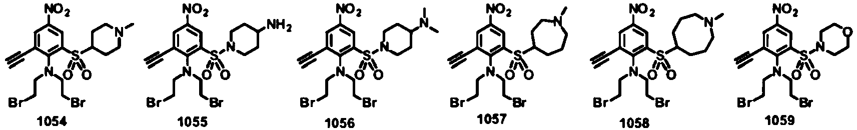

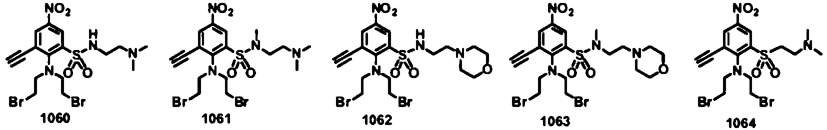

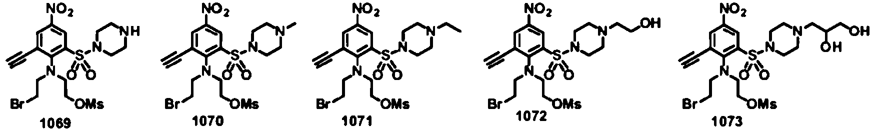

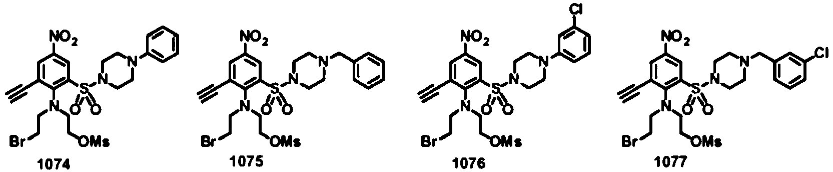

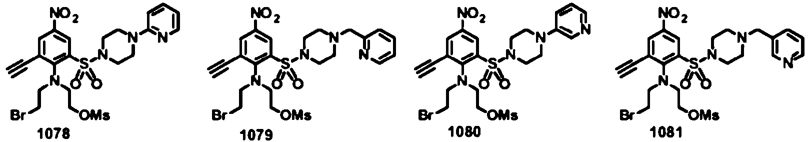

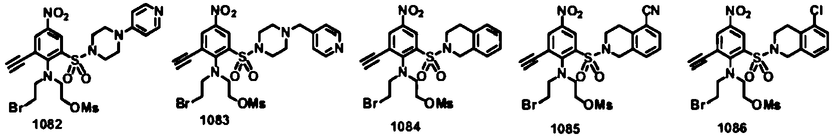

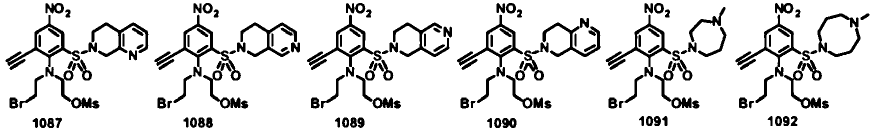

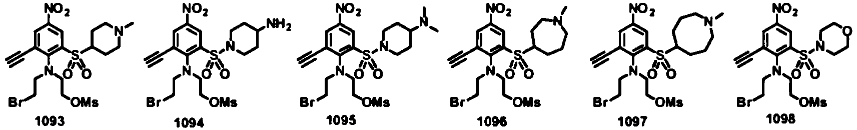

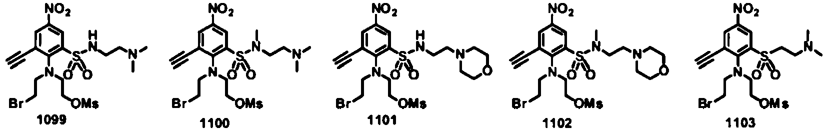

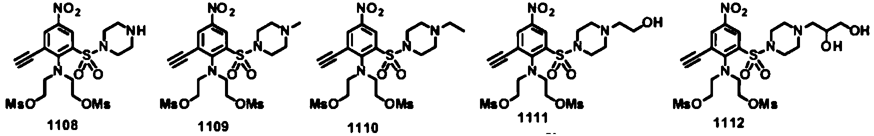

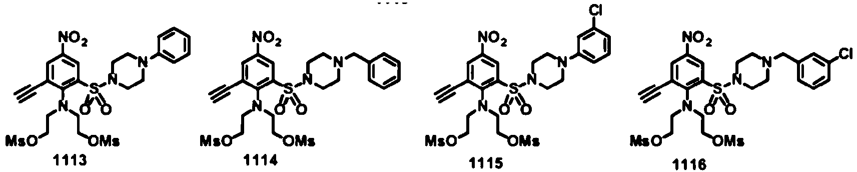

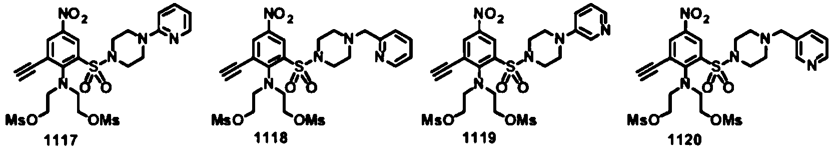

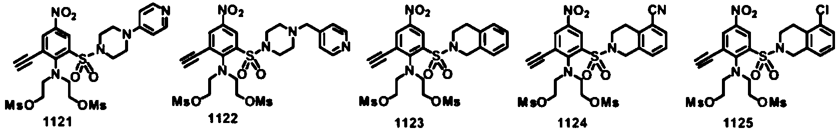

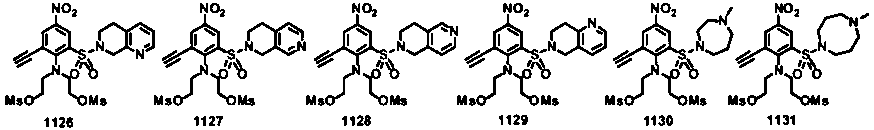

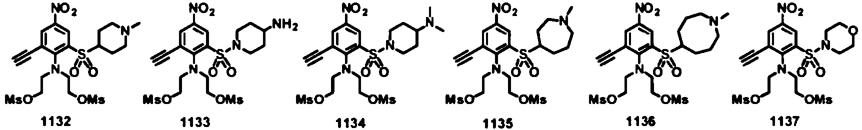

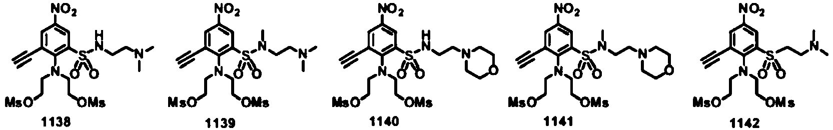

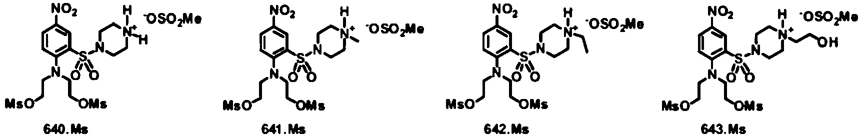

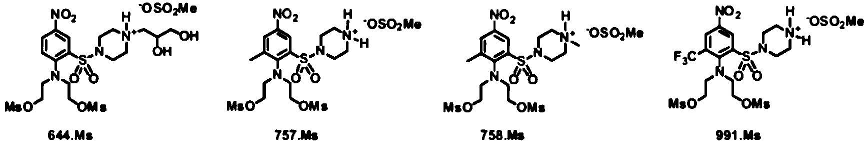

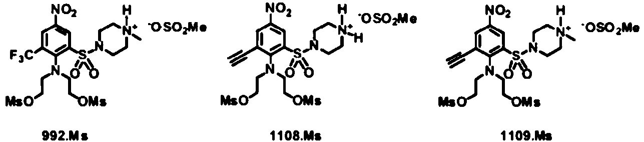

The specific synthesis method and corresponding spectral data of the compound of formula (8) are disclosed in WO2019190331A1 (corresponding to Chinese publication text CN111918864A), which is incorporated herein by reference in its entirety. 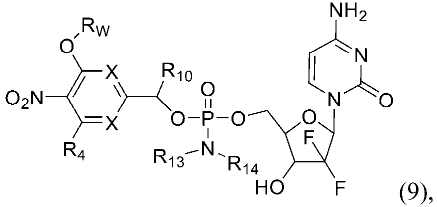 wherein the definitions of R_{w}, X, R₄, R₁₀, R₁₃, and R₁₄ are described in the claims of the Patent Application PCT/CN2022/098082 with Publication No. WO2022258043A1, and the synthesis and preparation methods of specific compounds are also described in the above patent application, which is incorporated herein by reference in its entirety. The specific definitions are as follows:
Each of the two X groups is independently CR¹⁵ or N;
Each of R₁₃ and R₁₄ is independently hydrogen, C₁-C₆ alkyl, cycloalkyl, alkenyl, alkynyl, C₆-C₂₀ aryl, 5-20 membered heterocyclyl; halogen-substituted C₁-C₆ alkyl, cycloalkyl, alkenyl, alkynyl; halogen-substituted C₆-C₂₀ aryl, or halogen-substituted 5-20 membered heterocyclyl, and R₁₃ and R₁₄ are not both hydrogen;
R₁₀ is hydrogen, C₁-C₆ alkyl, cycloalkyl, alkenyl, alkynyl, C₆-C₂₀ aryl, 5-20 membered heterocyclyl; halogen-substituted C₁-C₆ alkyl, cycloalkyl, alkenyl, alkynyl; halogen-substituted C₆-C₂₀ aryl, or halogen-substituted 5-20 membered heterocyclyl;
Alternatively, R₁₀ may be attached to R₁₃ or R₁₄ to form a 5-9 membered ring under the conditions consistent with the aforementioned definitions of R₁₀, R₁₃, and R₁₄;
Each of R₄ and R¹⁵ is independently hydrogen, halogen, C₁-C₆ alkyl, cycloalkyl, alkenyl, alkynyl, alkoxy, cyano, 5-20 membered heterocyclyl, C₆-C₂₀ aryl; or halogen-substituted C₁-C₆ alkyl, cycloalkyl, alkenyl, alkynyl, alkoxy, 5-20 membered heterocyclyl, or C₆-C₂₀ aryl;
Alternatively, R₁₀ and R¹⁵ may form a 4-12 membered cyclic hydrocarbon or heterocycle under the conditions consistent with the aforementioned definitions of R₁₀ and R¹⁵;
R_{W} is 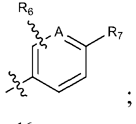
A is CR¹⁶ or N, and the position of A may vary on the ring;
R¹⁶ is hydrogen, C₁-C₆ alkyl, cycloalkyl, alkenyl, alkynyl, C₆-C₂₀ aryl, 5-20 membered heterocyclyl; halogen-substituted C₁-C₆ alkyl, cycloalkyl, alkenyl, alkynyl; halogen-substituted C₆-C₂₀ aryl, or halogen-substituted 5-20 membered heterocyclyl;
R₆ and R₇ satisfy the following conditions:
   Each of R₆ and R₇ is independently hydrogen, halogen, cyano, hydroxyl, C₁-C₆ alkyl, cycloalkyl, alkenyl, alkynyl, alkoxy, 5-20 membered heterocyclyl, C₆-C₂₀ aryl; or halogen-substituted C₁-C₆ alkyl, cycloalkyl, alkenyl, alkynyl, alkoxy, 5-20 membered heterocyclyl, C₆-C₂₀ aryl; or cyano-substituted C₁-C₆ alkyl, cycloalkyl, alkenyl, alkynyl, alkoxy, 5-20 membered heterocyclyl, C₆-C₂₀ aryl; or hydroxyl-substituted C₁-C₆ alkyl, cycloalkyl, alkoxy, 5-20 membered heterocyclyl, C₆-C₂₀ aryl, or -CONR¹¹R¹², or -CH₂NR¹¹R¹²;
   Alternatively, R₆ and R₇ are attached to form a 5-8 membered monocyclic heterocycle or fused heterocycle containing at least one of N, S and O, or two or three of N, S and O;
   Alternatively, R₆ and R₇ are attached to form a 5-8 membered monocyclic heterocycle or fused heterocycle containing at least one of N, S and O, or two or three of N, S and O, and the monocyclic heterocycle or fused heterocycle is substituted with C₁-C₆ alkyl;
   R₆ may be attached to CR¹⁶ to form a 5-9 membered ring, heterocycle, or aromatic heterocycle;
   R¹¹ and R¹² satisfy the following conditions:
      Each of R¹¹ and R¹² is independently C₁-C₆ alkyl or halogen-substituted C₁-C₆ alkyl; or R¹¹ and R¹², under the conditions consistent with the aforementioned definitions, form a 5-7 membered ring with the N in -CONR¹¹R¹² or the N in -CH₂NR¹¹R¹².

The AKR1C3 enzyme-activated prodrug compounds of formula (9) are selected from compounds with the following structural formulae:

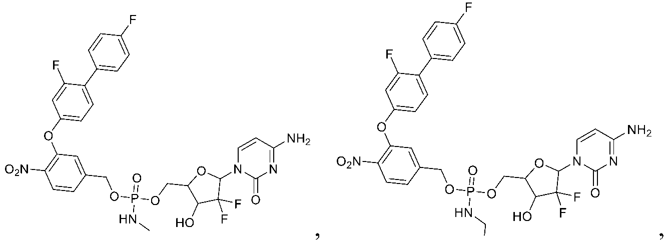

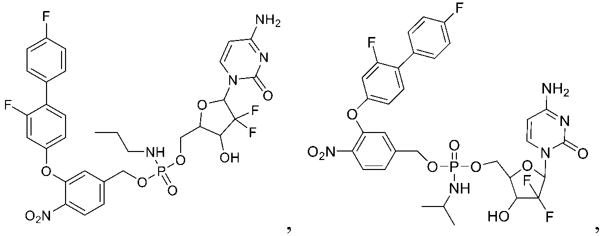

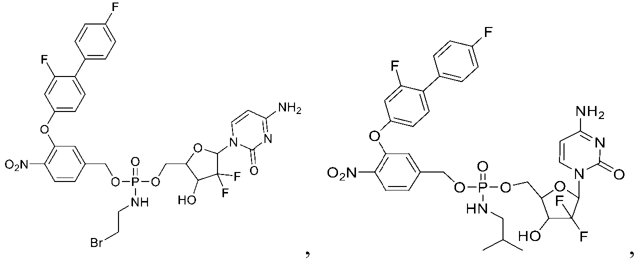

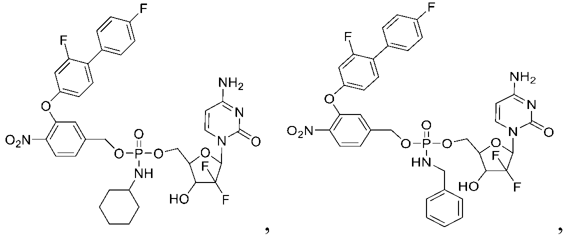

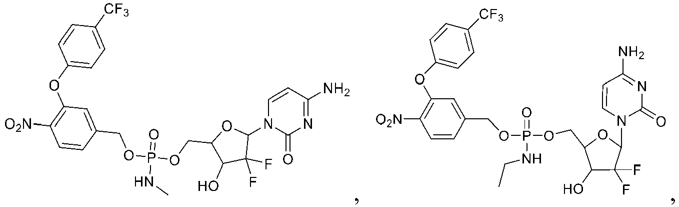

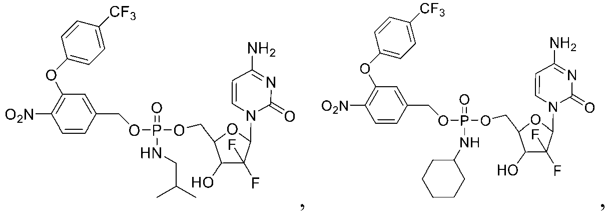

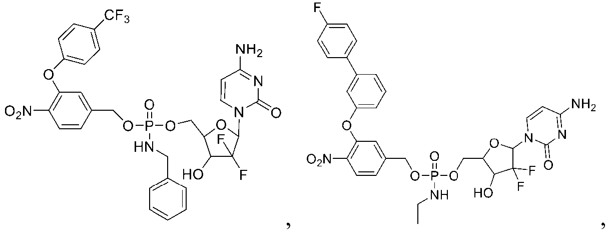

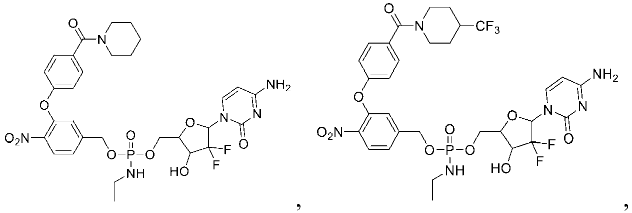

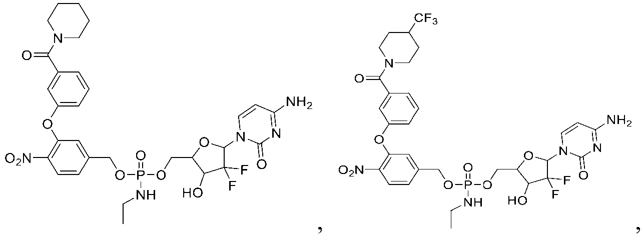

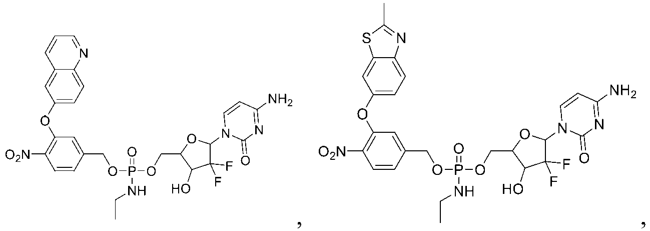

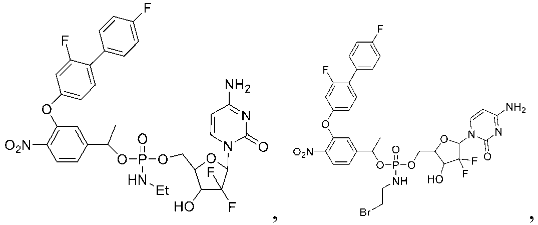

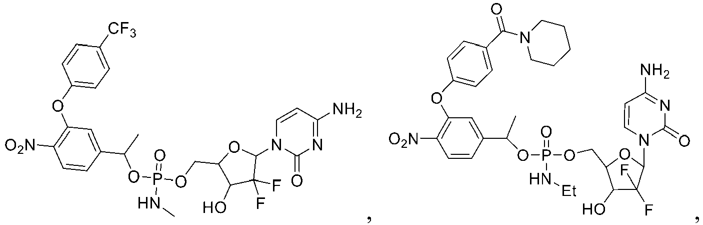

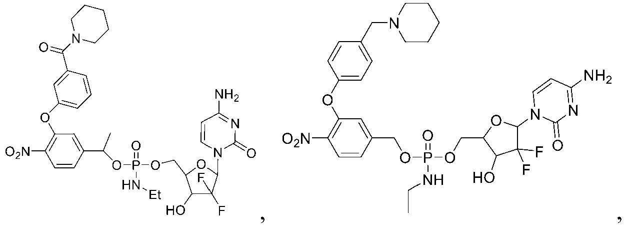

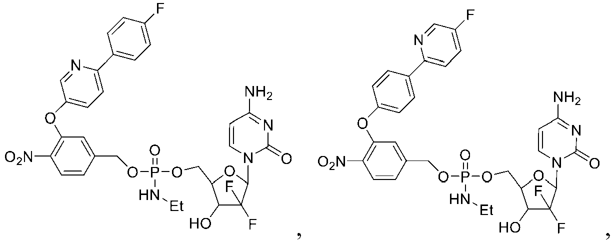

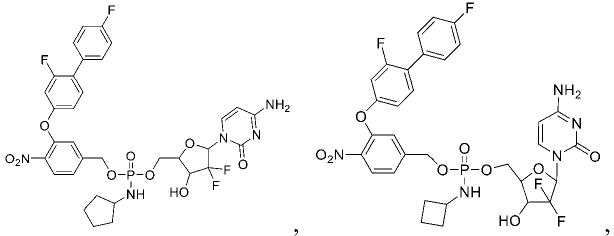

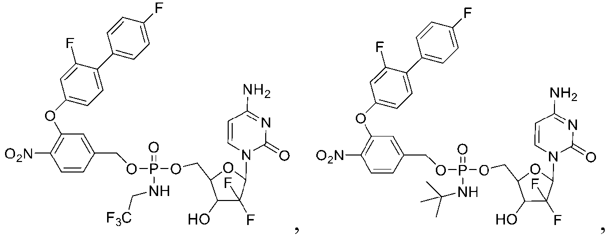

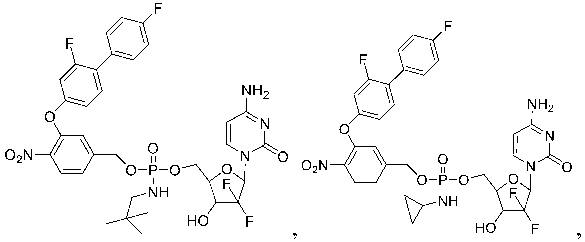

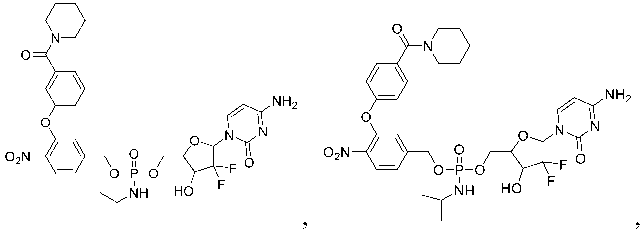

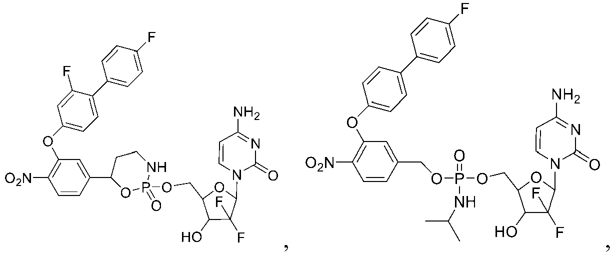

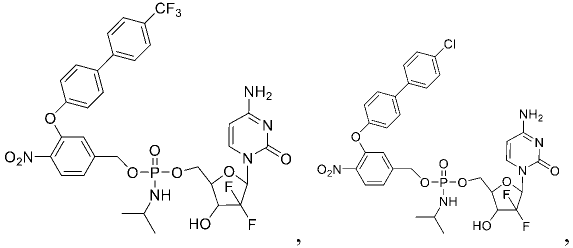

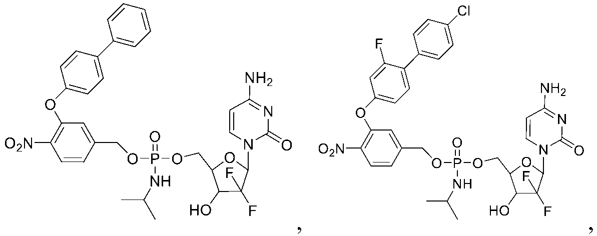

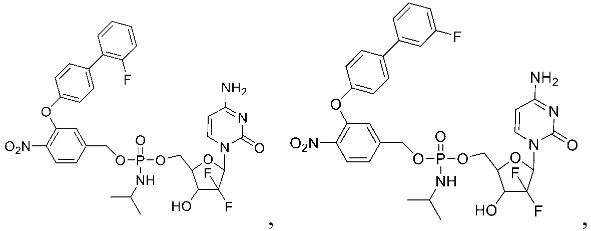

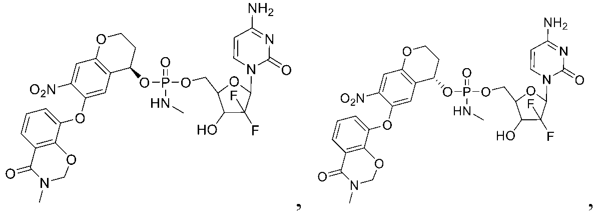

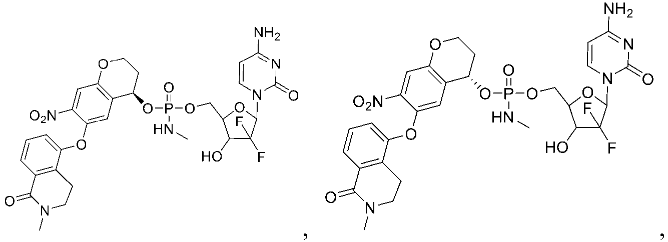

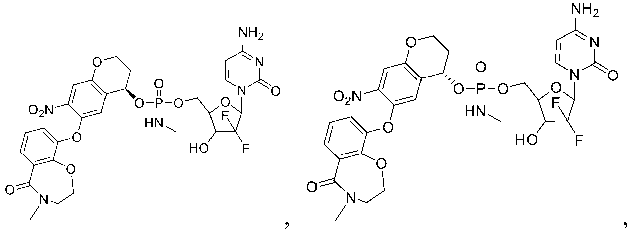

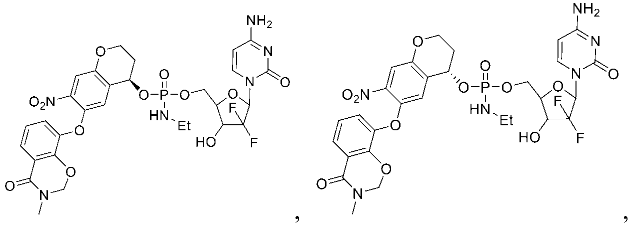

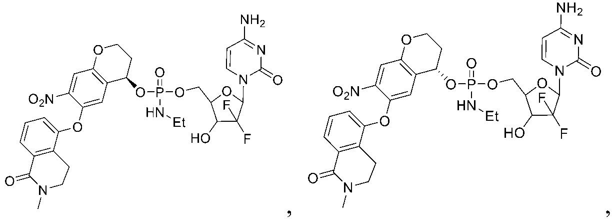

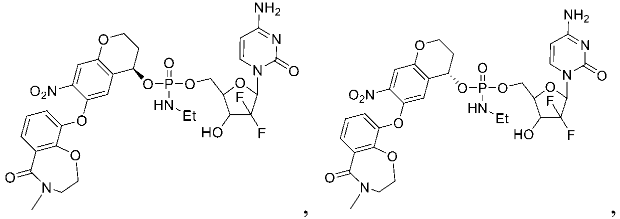

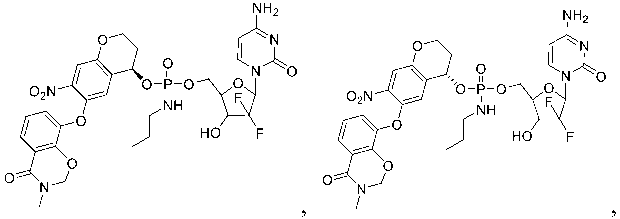

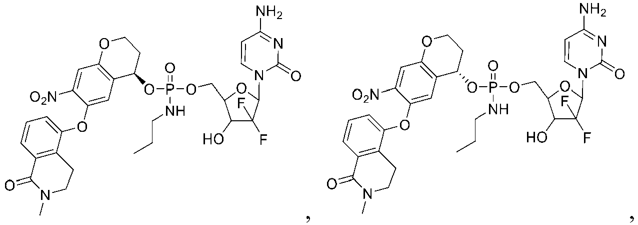

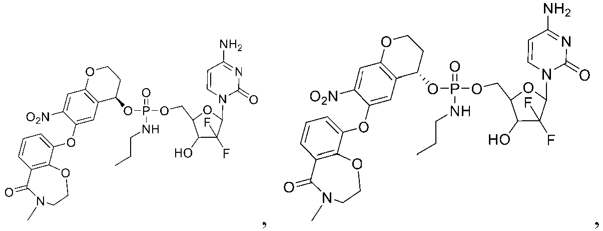

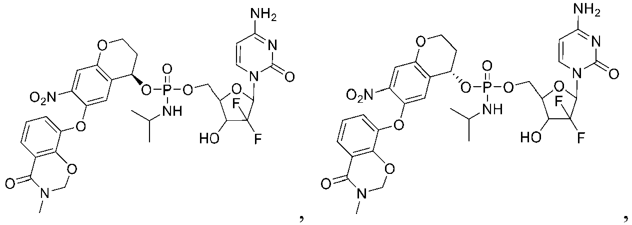

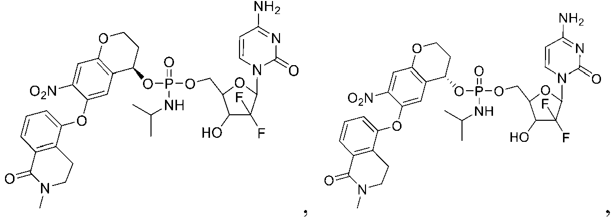

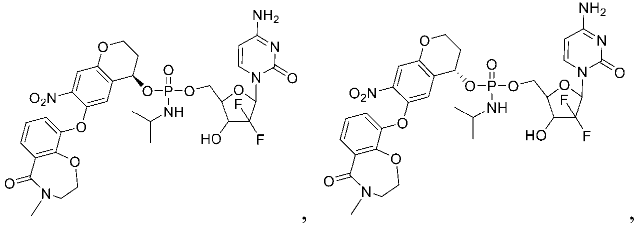

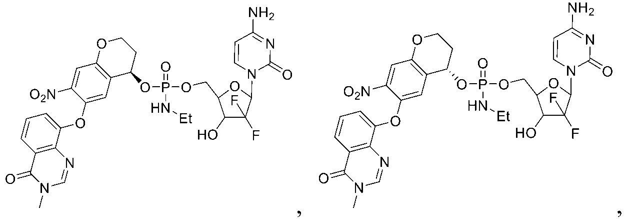

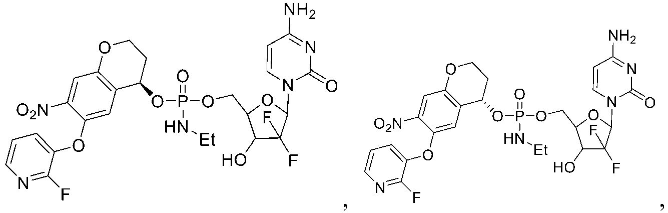

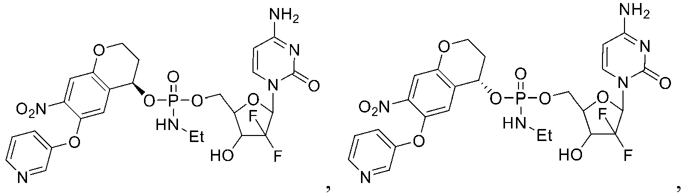

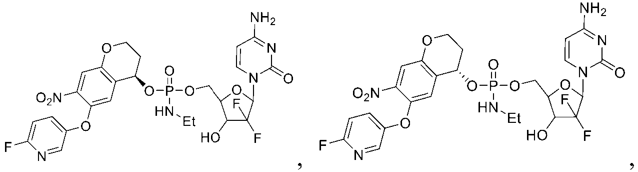

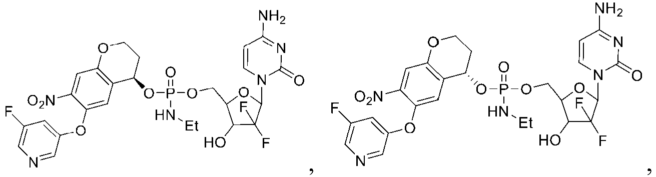

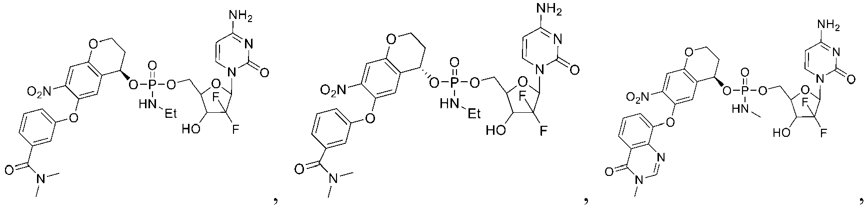

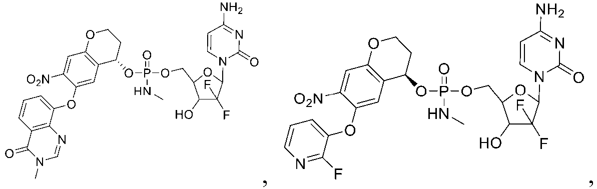

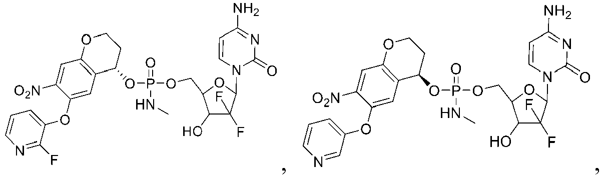

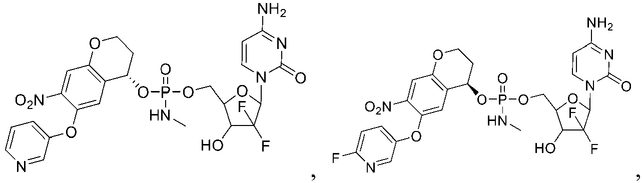

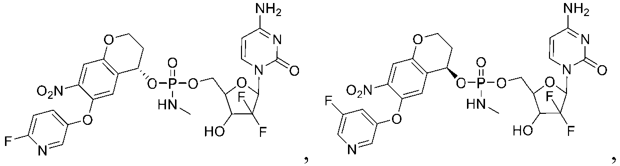

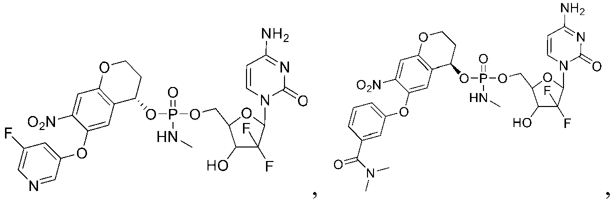

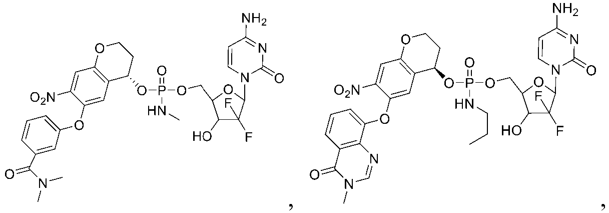

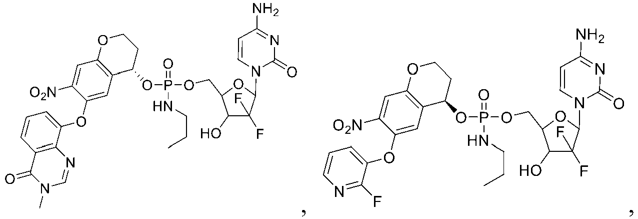

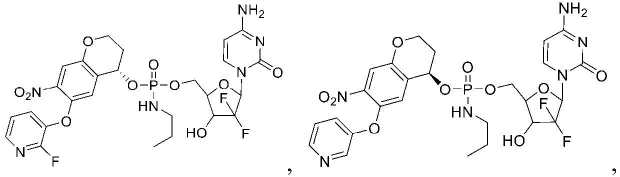

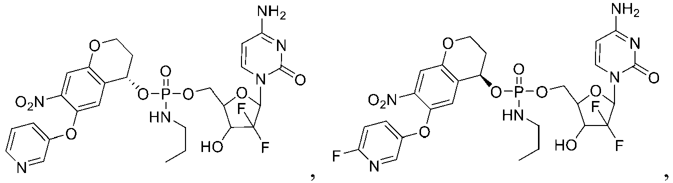

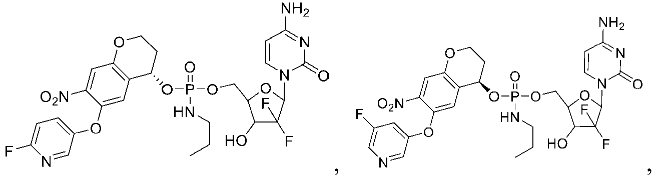

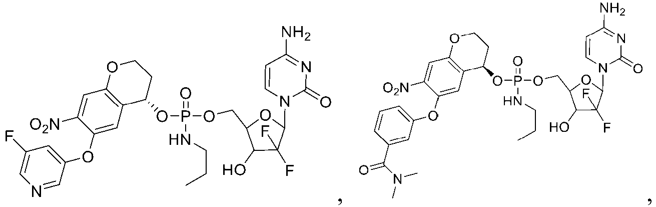

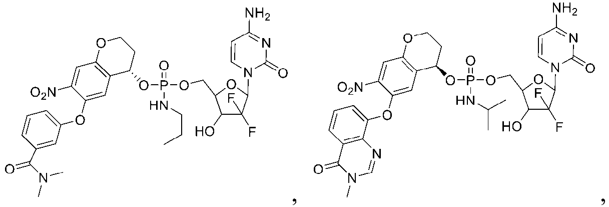

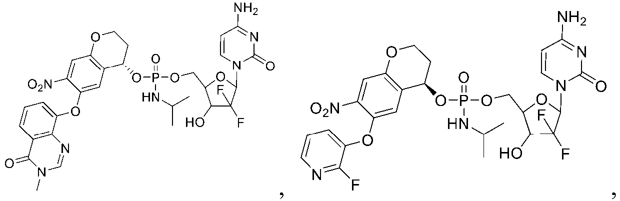

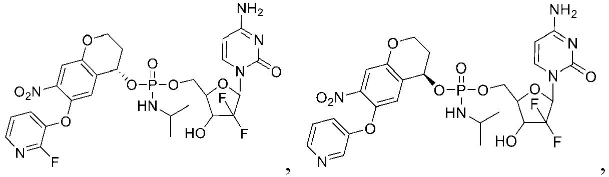

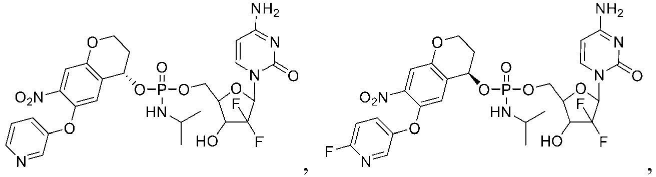

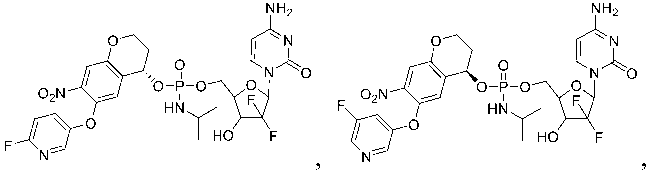

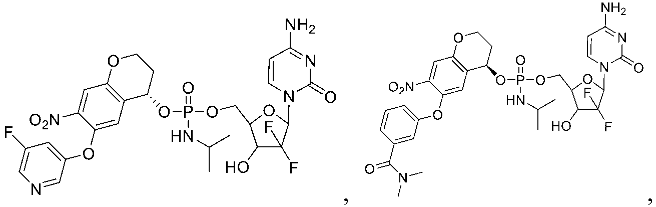

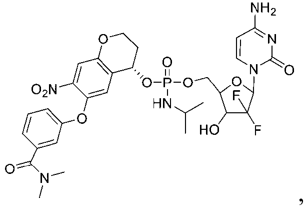

The specific synthesis method and corresponding spectral data of the compound of formula (9) are disclosed in WO2022258043A1, which is incorporated herein by reference in its entirety. 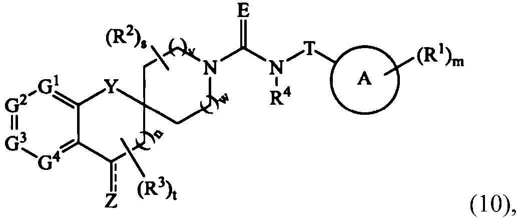 wherein the definitions of R₁, R₂, R₃, R₄, G₁, G₂, G₃, G₄, E, T, Y, Z, m, n, s, t, v, w, and ring A are described in the claims of the Patent Application CN202210585771.6 with Publication No. CN115403579A, and the synthesis and preparation methods of specific compounds are also described in the above patent application, which is incorporated herein by reference in its entirety. The specific definitions are as follows:
G¹, G², G³, or G⁴ are the same or different, and each is independently CR⁵ or N atom;
Each R⁵ is the same or different, and each is independently selected from a hydrogen atom, halogen, alkyl, alkenyl, alkynyl, alkoxy, hydroxyl, cyano, amino, nitro, -NR^{a}R^{b}, -C(O)NR^{a}R^{b}, cycloalkyl, heterocyclyl, aryl, and heteroaryl, wherein the alkyl, alkenyl, alkynyl, alkoxy, cycloalkyl, heterocyclyl, aryl, and heteroaryl are each independently optionally substituted with one or more substituents selected from halogen, alkyl, haloalkyl, alkoxy, haloalkoxy, hydroxyalkyl, hydroxyl, oxo, cyano, amino, nitro, cycloalkyl, heterocyclyl, aryl, and heteroaryl;
Y is selected from -(C(R^{y2}R^{y3}))_{f}-NR^{y1}-, -(C(R^{y2}R^{y3}))_{g}-O-, -(C(R^{y2}R^{y3}))ₕ-S-, -(C(R^{y2}R^{y3}))ₕ-S(O)-, -(C(R^{y2}R^{y3}))ₕ-S(O)₂-, -C(R^{y2}R^{y3})-, -NR^{y1}-(C(R^{y2}R^{y3}))_{f}-, -O-(C(R^{y2}R^{y3}))_{g}-, -S-(C(R^{y2}R^{y3}))ₕ-, -S(O)-(C(R^{y2}R^{y3}))ₕ-, and -S(O)₂-(C(R^{y2}R^{y3}))ₕ-;
R^{y1} is selected from a hydrogen atom, alkyl, haloalkyl, hydroxyalkyl, cycloalkyl, and heterocyclyl;
R^{y2} and R^{y3} are the same or different, and each is independently selected from a hydrogen atom, halogen, alkyl, haloalkyl, hydroxyalkyl, cycloalkyl, and heterocyclyl;
Alternatively, R^{y2} and R^{y3} together form =O;
Z is O or OH;
-̅ -̅ -̅ -̅ -̅ is a single bond or a double bond; when -̅ -̅ -̅ -̅ -̅ is a single bond, Z is OH; when
-̅ -̅ -̅ -̅ -̅ is a double bond, Z is O;
E is selected from NH, an O atom, and a S atom;
T is selected from -C(R^{T1}R^{T2})-, -NR^{T3}-, or -O-;
R^{T1} and R^{T2} are the same or different, and each of R^{T1} and R^{T2} is independently selected from a hydrogen atom, a deuterium atom, halogen, alkyl, haloalkyl, hydroxyalkyl, cycloalkyl, and heterocyclyl;
Alternatively, R^{T1} and R^{T2} together with the carbon atom to which they are attached form cycloalkyl or heterocyclyl, wherein the cycloalkyl or heterocyclyl is each independently optionally substituted with one or more substituents selected from halogen, alkyl, and hydroxyl;
R^{T3} is selected from a hydrogen atom, alkyl, haloalkyl, hydroxyalkyl, cycloalkyl, and heterocyclyl;
Ring A is a 6- to 10-membered aryl or a 5- to 10-membered heteroaryl;
Each R¹ is the same or different, and each R¹ is independently selected from a hydrogen atom, a deuterium atom, halogen, alkyl, alkenyl, alkynyl, alkoxy, hydroxyl, cyano, -NR^{a}R^{b}, -C(O)NR^{a}R^{b}, -S(O)NR^{a}R^{b}, -S(O)2NR^{a}R^{b}, -S(O)R^{c}, -S(O)₂R^{c}, -B(OR^{d})₂, nitro, cycloalkyl, heterocyclyl, aryl, and heteroaryl, wherein the alkyl, alkenyl, alkynyl, alkoxy, cycloalkyl, heterocyclyl, aryl, and heteroaryl are each independently optionally substituted with one or more substituents selected from halogen, alkyl, haloalkyl, alkoxy, haloalkoxy, hydroxyalkyl, hydroxyl, oxo, cyano, -NR^{a}R^{b}, -C(O)NR^{a}R^{b}, -S(O)NR^{a}R^{b}, -S(O)₂NR^{a}R^{b}, -S(O)R^{c}, -S(O)₂R^{c}, -B(OR^{d})₂, nitro, cycloalkyl, heterocyclyl, aryl, and heteroaryl;
R^{a} and R^{b} are the same or different, and each of R^{a} and R^{b} is independently selected from a hydrogen atom, alkyl, haloalkyl, hydroxyl, hydroxyalkyl, -C(O)R^{e}, cycloalkyl, and heterocyclyl; alternatively, R^{a} and R^{b} together with the nitrogen atom to which they are attached form cycloalkyl or heterocyclyl, wherein the cycloalkyl or heterocyclyl is optionally substituted with one or more substituents selected from halogen, alkyl, haloalkyl, alkoxy, haloalkoxy, hydroxyalkyl, hydroxyl, cyano, amino, nitro, cycloalkyl, heterocyclyl, aryl, and heteroaryl;
R^{c} is selected from alkyl, cycloalkyl, heterocyclyl, aryl, and heteroaryl, wherein the alkyl, cycloalkyl, heterocyclyl, aryl, and heteroaryl are optionally substituted with one or more substituents selected from halogen, alkyl, haloalkyl, alkoxy, haloalkoxy, hydroxyalkyl, hydroxyl, cyano, amino, nitro, cycloalkyl, heterocyclyl, aryl, and heteroaryl;
R^{d} is a hydrogen atom or C₁₋₆ alkyl;
R^{e} is selected from alkyl, alkoxy, cycloalkyl, heterocyclyl, aryl, and heteroaryl, wherein the alkyl, alkoxy, cycloalkyl, heterocyclyl, aryl, and heteroaryl are optionally substituted with one or more substituents selected from halogen, alkyl, haloalkyl, alkoxy, haloalkoxy, hydroxyalkyl, hydroxyl, cyano, amino, nitro, cycloalkyl, heterocyclyl, aryl, and heteroaryl;
Each R² is the same or different, and each R² is independently selected from a hydrogen atom, a deuterium atom, halogen, alkyl, haloalkyl, alkoxy, haloalkoxy, hydroxyalkyl, oxo, hydroxyl, cyano, amino, nitro, cycloalkyl, heterocyclyl, aryl, and heteroaryl;
Each R³ is the same or different, and each R³ is independently selected from a hydrogen atom, a deuterium atom, halogen, alkyl, haloalkyl, alkoxy, haloalkoxy, hydroxyalkyl, oxo, hydroxyl, cyano, amino, nitro, cycloalkyl, heterocyclyl, aryl, and heteroaryl;
R⁴ is selected from a hydrogen atom, alkyl, haloalkyl, hydroxyl, and hydroxyalkyl; n is 0, 1, 2, or 3;
v is 0, 1, or 2;
w is 0, 1, or 2;
f is 0, 1, or 2;
g is 0, 1, or 2;
h is 0, 1, or 2;
m is 0, 1, 2, 3, 4, or 5;
s is 0, 1, 2, 3, 4, 5, 6, 7, or 8;
t is 0, 1, 2, 3, 4, 5, or 6;
provided that
when Y is an -O- atom and E is an O atom, ring A is phenyl or a 5- to 6-membered heteroaryl, and G³ is CR⁵ or a N atom, and R⁵ is not a hydrogen atom;
when Y is an -O- atom and E is a S atom, ring A is phenyl or a 5- to 6-membered heteroaryl;
when G¹, G², G³, and G⁴ are all CR⁵, Y is NR^{y1}, n, v, and w are all 1, and E is an O atom, 1) T is not CH₂ or CD₂; 2) at least one of R² or R³ is a deuterium atom; 3) R⁴ is selected from alkyl, haloalkyl, hydroxyl, and hydroxyalkyl; 4) one of R¹ is a 3- to 8-membered cycloalkyl or a 5- to 8-membered heterocyclyl, wherein the 3- to 8-membered cycloalkyl or 5- to 8-membered heterocyclyl is optionally substituted with one or more substituents selected from halogen, alkyl, haloalkyl, alkoxy, haloalkoxy, hydroxyalkyl, hydroxyl, oxo, cyano, amino, nitro, cycloalkyl, heterocyclyl, aryl, and heteroaryl; 5) ring A is R^{d} being a hydrogen atom or C₁₋₆ alkyl.

The AKR1C3 enzyme-activated KARS inhibitor prodrug compounds of formula (10) are selected from compounds with the following structural formulae:

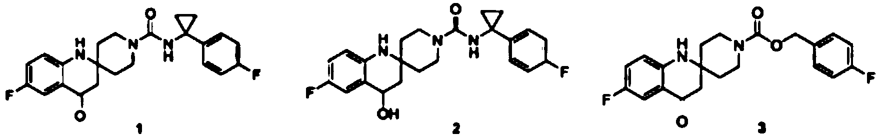

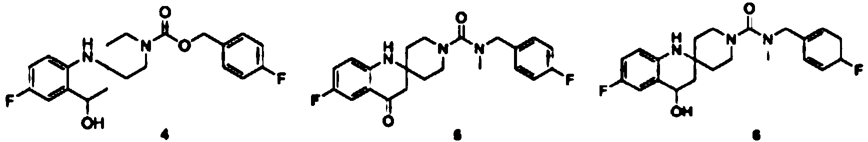

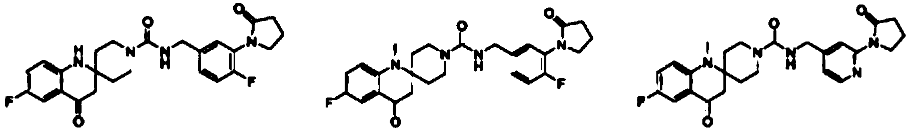

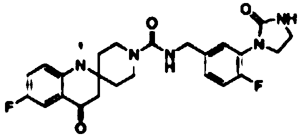

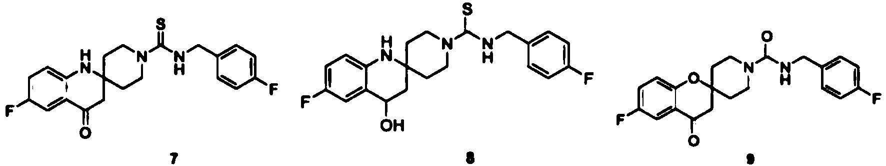

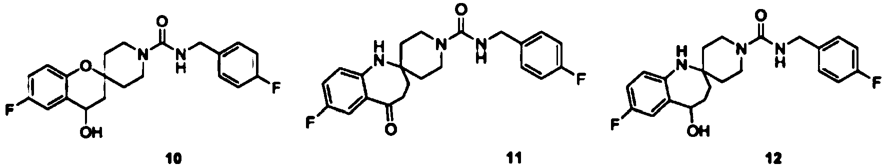

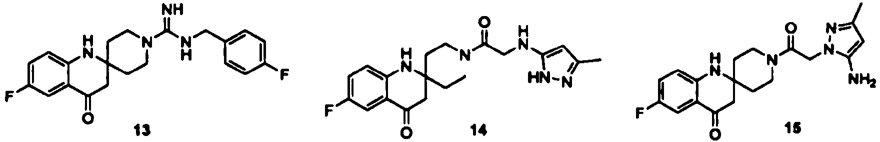

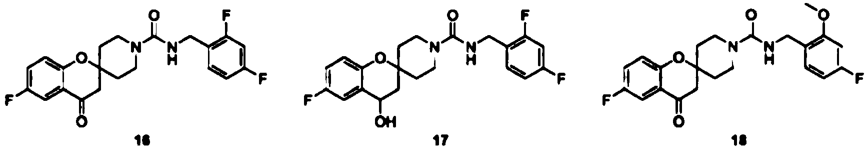

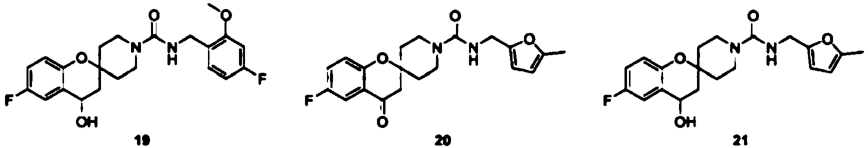

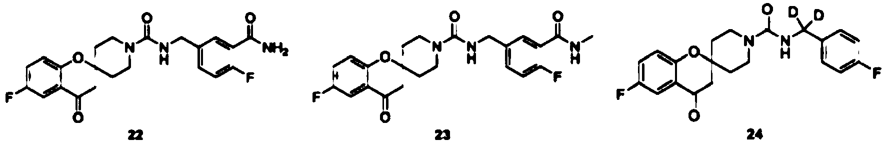

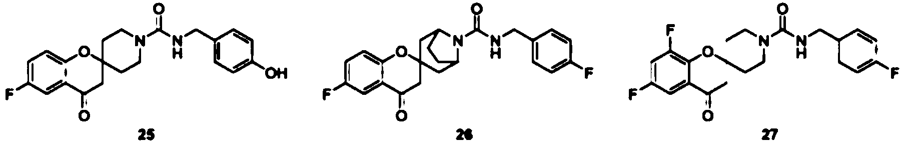

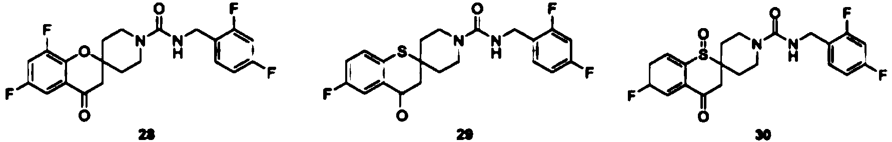

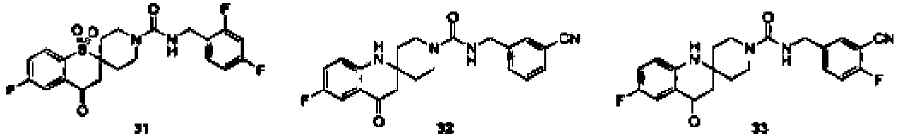

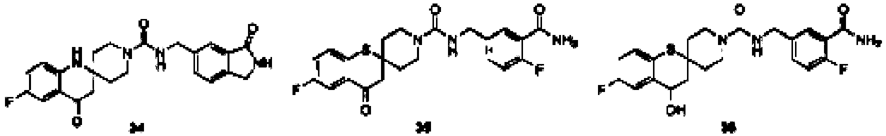

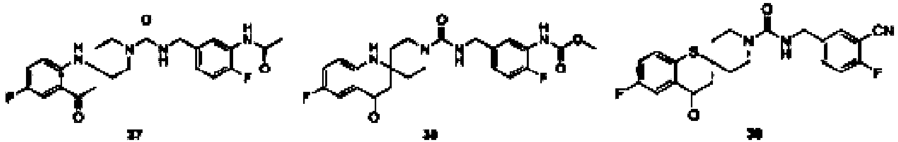

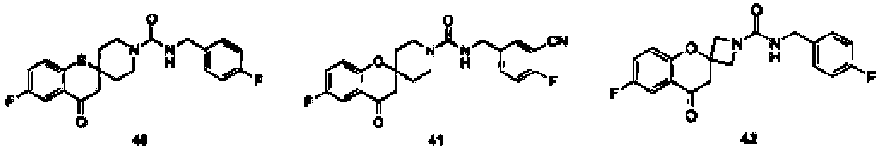

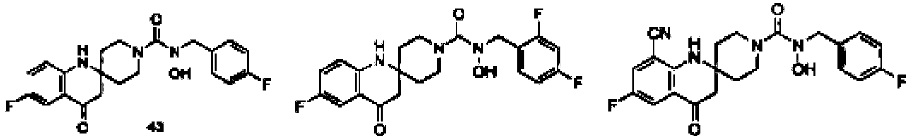

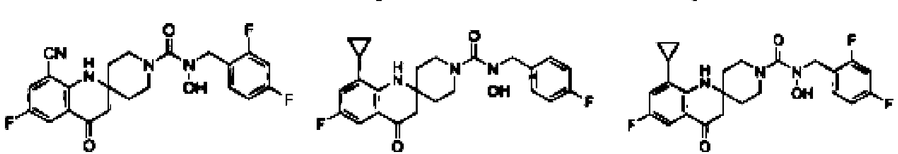

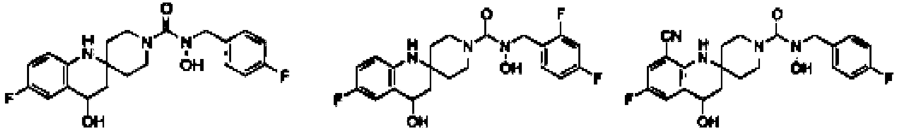

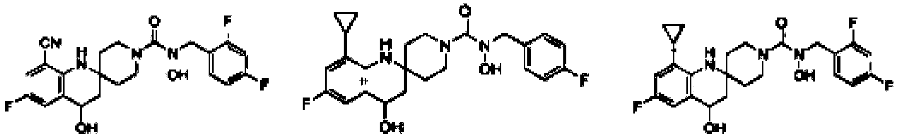

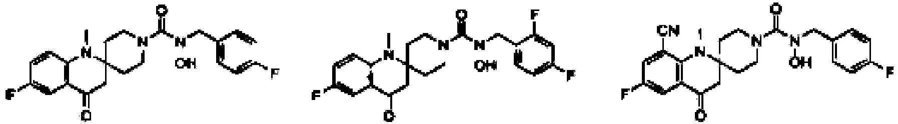

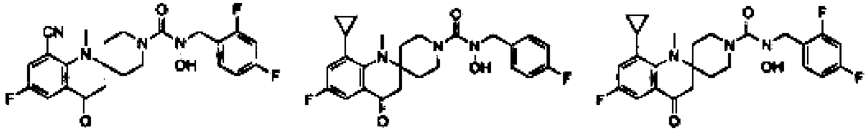

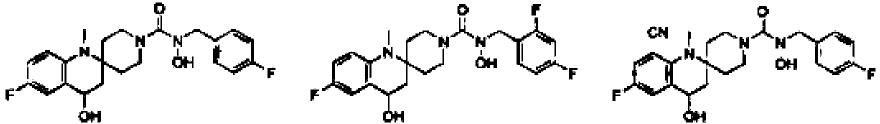

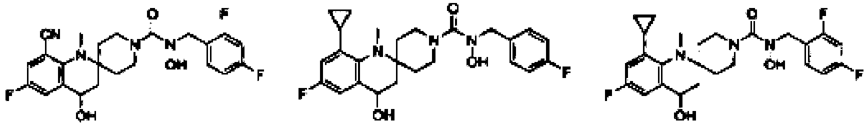

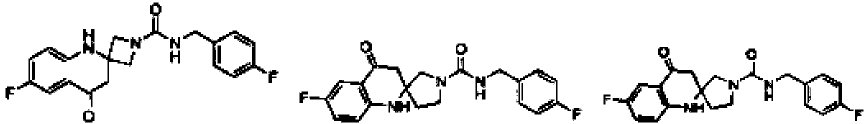

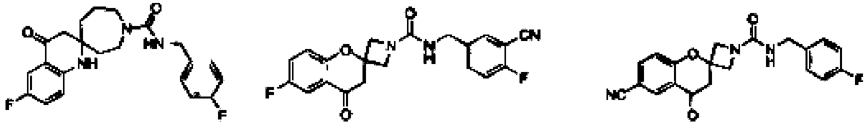

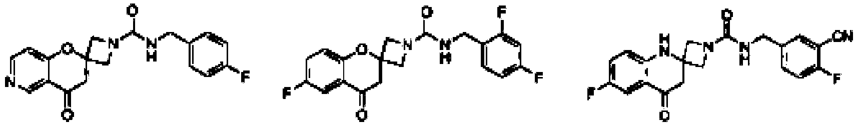

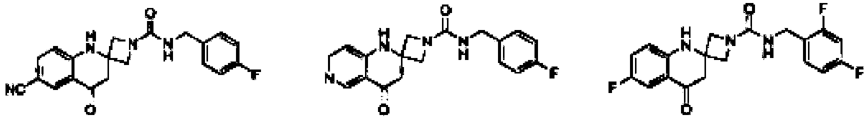

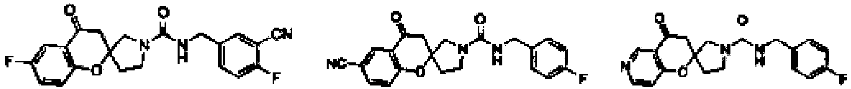

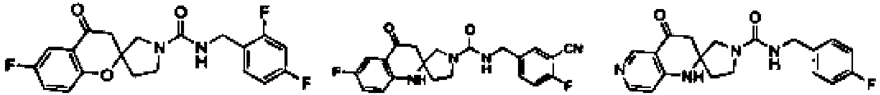

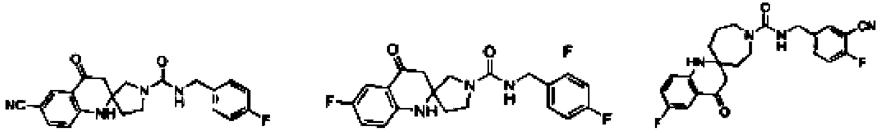

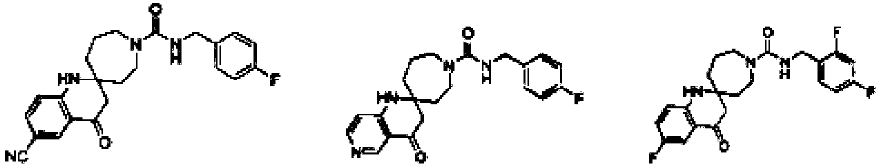

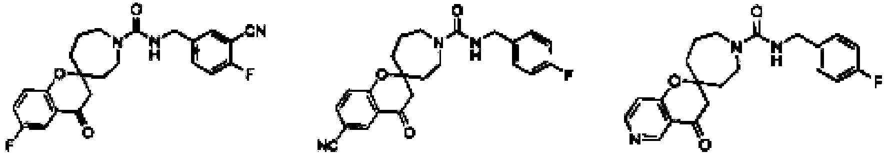

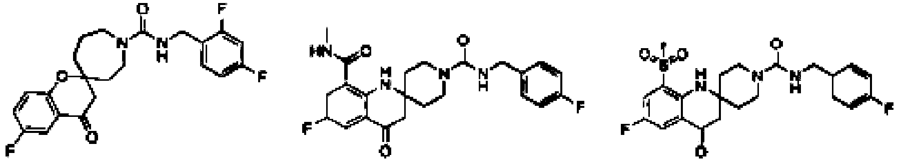

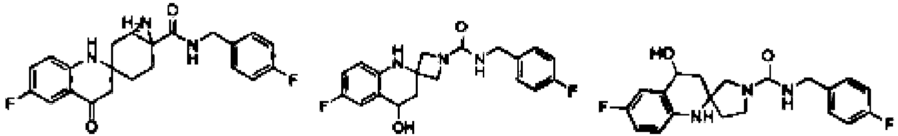

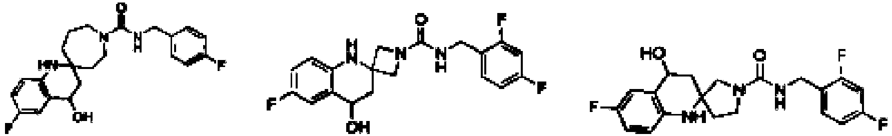

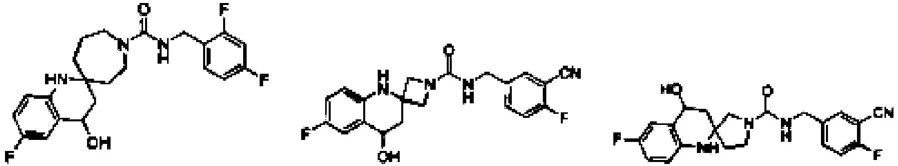

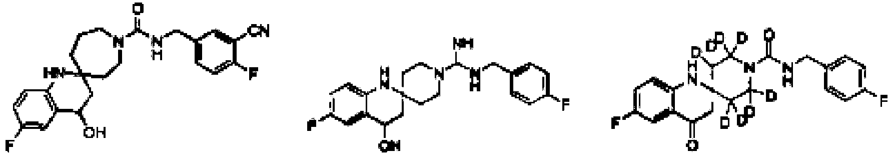

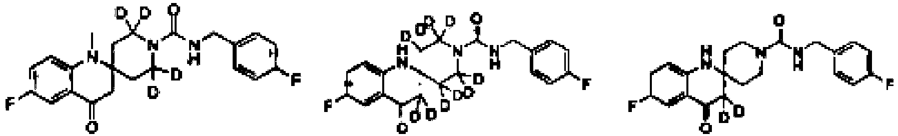

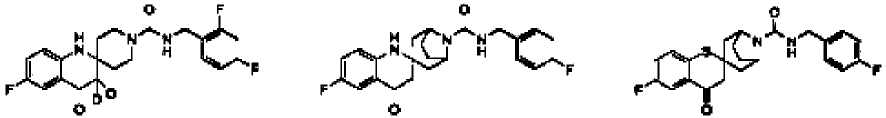

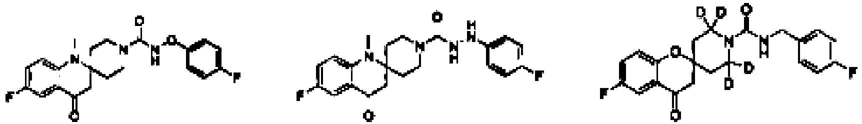

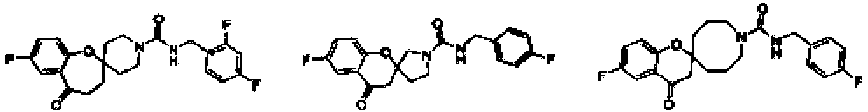

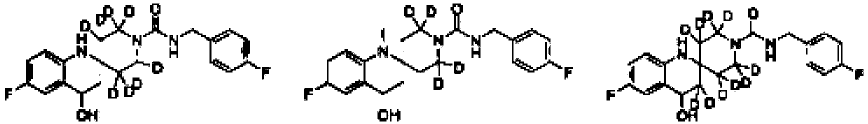

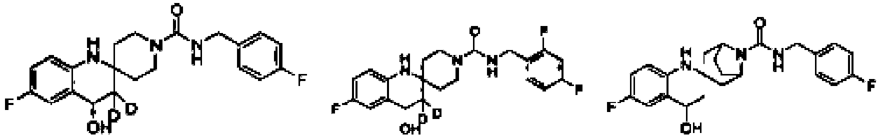

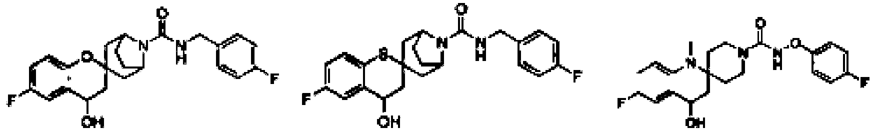

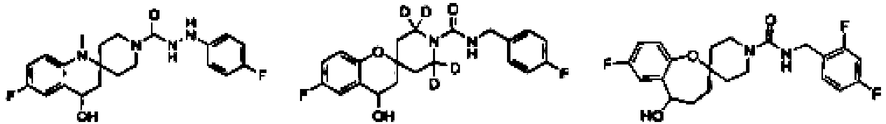

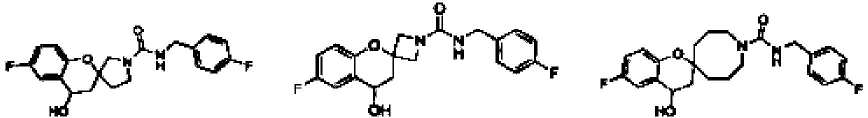

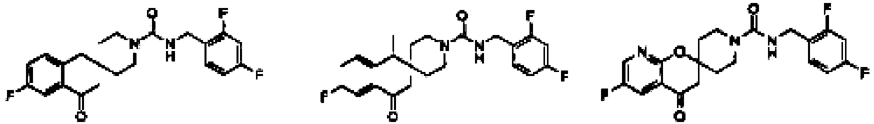

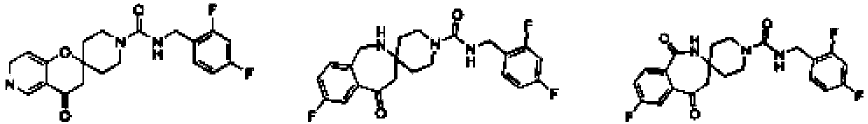

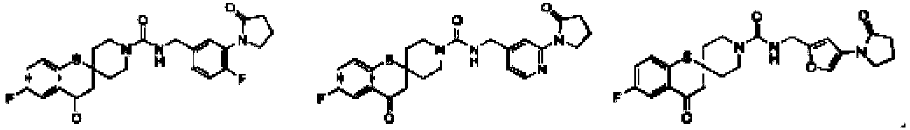

The specific synthesis method and corresponding spectral data of the compound of formula (10) are disclosed in the Chinese publication text CN115403579A, which is incorporated herein by reference in its entirety. 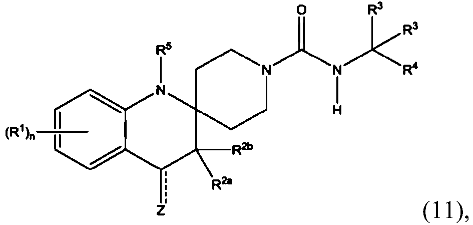 wherein the definitions of R¹, R^{2a}, R^{2b}, R³, R⁴, R⁵, n, and Z are described in the claims of the Patent Application PCT/IB2020/057285 with Publication No. WO2021005586A1 (corresponding to Chinese Application No. CN202080053804.1 with Publication No. CN114206870A), and the synthesis and preparation methods of specific compounds are also described in the above patent application, which is incorporated herein by reference in its entirety. The specific definitions are as follows:
----- is a single bond or a double bond;
Z is OH when -̅ -̅ -̅ -̅ -̅ is a single bond; or O when -̅ -̅ -̅ -̅ -̅ is a double bond;
Each R¹ is independently selected from the group consisting of (C₁-C₆)alkyl, (C₁-C₆)alkoxy, (C₀-C₄)alkylN(R⁸)₂, and halogeno;
Each of R^{2a} and R^{2b} is independently selected from the group consisting of H, (C₁-C₆)alkyl, and halogeno;
Each R³ is independently selected from the group consisting of H and halogeno;
R⁴ is selected from the group consisting of aryl, 5- to 6-membered heteroaryl containing 1, 2, 3, or 4 heteroatoms independently selected from N, O, and S; and 9-to 10-membered fused bicyclic heteroaryl containing 1, 2, 3, or 4 heteroatoms independently selected from N, O, and S; wherein any of the foregoing is optionally substituted with one or more R⁶;
R⁵ is selected from the group consisting of H; (C₁-C₆)alkyl; (C₂-C₆)alkenyl; (C₀-C₄)alkylOR⁸; (C₁-C₄)alkyl(C₃-C₁₀)cycloalkyl; halo(C₁-C₆)alkyl; (C₂-C₃)alkynyl; (C₁-C₄)alkylN(R¹⁰)₂;
Each R⁶ is independently selected from the group consisting of halogeno; (C₁-C₆)alkyl; (C₁-C₆)alkoxy; halo(C₁-C₆)alkyl; OH; aryl; 3- to 6-membered heterocycle; 5- to 6-membered heteroaryl; (C₀-C₄)alkylS(O)ₘ(C₁-C₆)alkyl; halo(C₁-C₆)alkoxy; (C₀-C₄)alkylS(O)ₘN(R⁸)₂; (C₀-C₄)alkylN(R⁸)₂; (C₀-C₄)alkyl(CO)OR⁷; N(R⁸)S(O)ₘ(C₁-C₆)alkyl; N(R⁸)S(O)ₘ(C₃-C₆)cycloalkyl; OP(O)(OH)₂; (C₀-C₃)alkyl(CO)NHR¹¹; (C₀-C₃)alkylOR⁷; and (C₃-C₁₀)cycloalkyl; wherein each R⁶, when not being halogeno, OH, or OP(O)(OH)₂, is optionally substituted with one to three R⁹; or two adjacent R⁶ together with the atom to which they are bonded form a 5- to 7-membered heterocycle or (C₅-C₈)cycloalkyl;
Each of R⁷ and R⁸ is independently selected from the group consisting of H or (C₁-C₆)alkyl optionally substituted with one to three R⁹;
Each R⁹ is independently selected from the group consisting of halogeno; -OH; amino; (C₁-C₄)alkylamino; di(C₁-C₄)alkylamino; OP(O)(OH)₂; (C₁-C₆)alkyl; (C₁-C₃)alkynyl; (C₁-C₆)alkoxy; halo(C₁-C₆)alkyl; (C₀-C₄)alkylS(O)ₘ(C₁-C₆)alkyl; halo(C₁-C₆)alkoxy; 3- to 6-membered heterocycle optionally substituted with oxo (=O); (C₀-C₄)alkylS(O)ₘN(R¹⁰)₂; (C₀-C₄)alkyl(CO)R¹⁰; (C₀-C₄)alkyl(CO)OR¹⁰; (C₀-C₄)alkylNR¹⁰S(O)ₘ(C₁-C₆)alkyl; (C₀-C₄)alkylOR¹⁰; (C₀-C₄)alkylN(R¹⁰)₂; (C₀-C₄)alkylCN; (C₀-C₄)alkylN(R¹⁰)₂; and (C₀-C₄)alkyl(CO)N(R¹⁰)₂;
Each R¹⁰ is independently selected from the group consisting of H; (C₁-C₆)alkyl; or 3-to 6-membered heterocycle, wherein the 3- to 6-membered heterocycle is optionally substituted with one or more substituents selected from (C₁-C₆)alkyl; and oxo (=O);
Each R¹¹ is independently selected from the group consisting of H; 4- to 6-membered heterocycle optionally substituted with one to four R¹²; (C₃-C₆)cycloalkyl optionally substituted with one to four R¹²; (C₀-C₃)alkyl(C₃-C₆)cycloalkyl(C₁-C₃)alkyl optionally substituted with halogeno; CH₂-aryl optionally substituted with one to three R¹²; (C₁-C₆)alkyl; (C₂-C₆)alkenyl; or (C₂-C₆)alkynyl; wherein each of the (C₁-C₆)alkyl; (C₂-C₆)alkenyl; and (C₂-C₆)alkynyl is optionally substituted with one or more R¹³;
Each R¹² is independently selected from the group consisting of OH; (C₁-C₃)alkoxy; NH₂; or (C₁-C₃)alkyl optionally substituted with one or more OH;
Each R¹³ is independently selected from the group consisting of halogeno; OH; amino; (C₁-C₄)alkylamino; di(C₁-C₄)alkylamino; (C₁-C₃)alkoxy; and C(O)-(C₃-C₈)cycloalkyl;
m is 0, 1, or 2; and
n is 0, 1, or 2.

The AKR1C3 enzyme-activated KARS inhibitor prodrug compounds of formula (11) are selected from compounds with the following structural formulae:

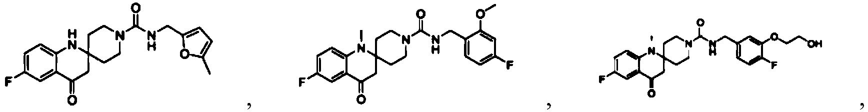

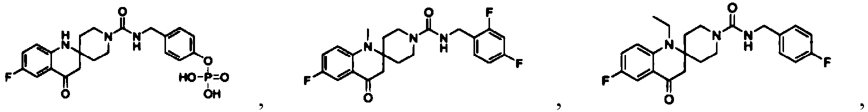

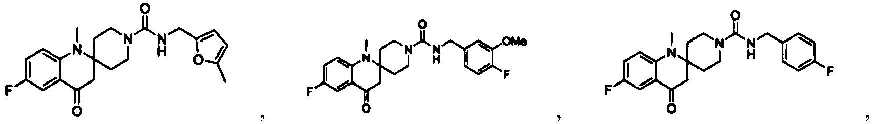

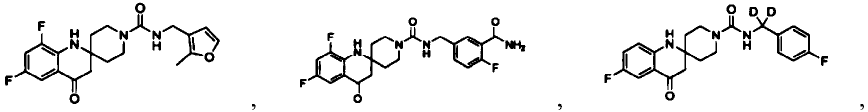

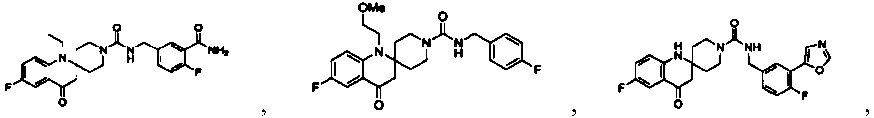

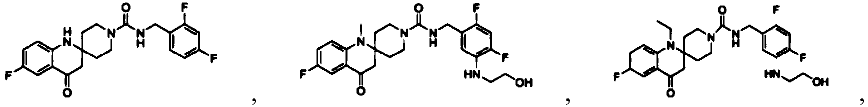

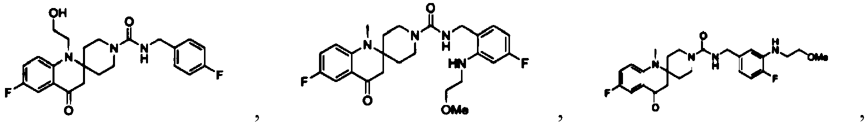

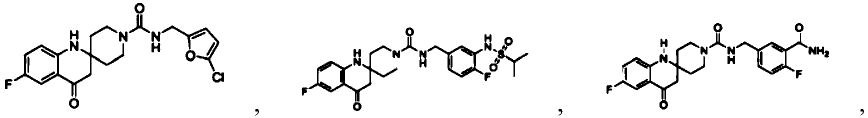

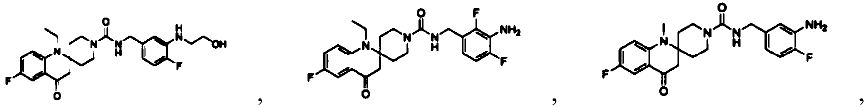

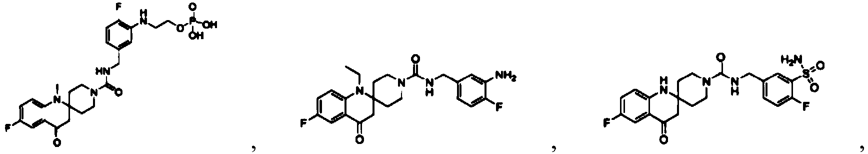

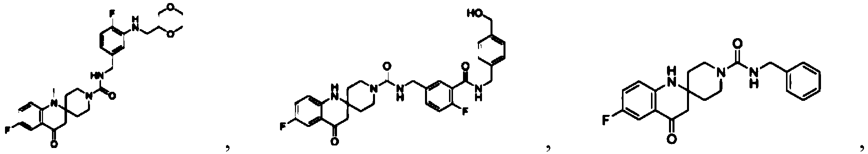

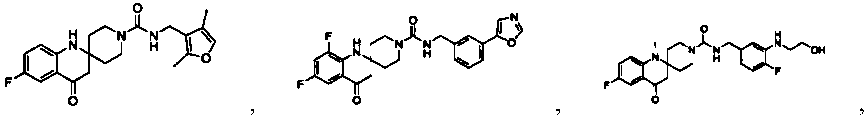

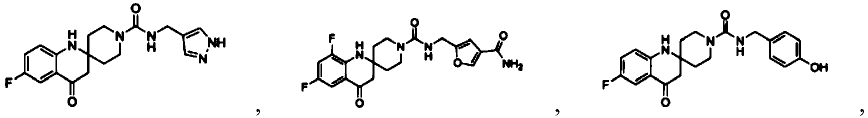

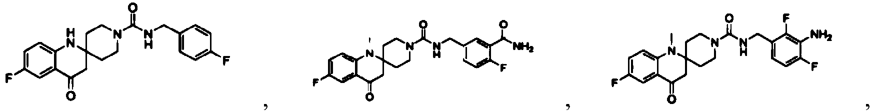

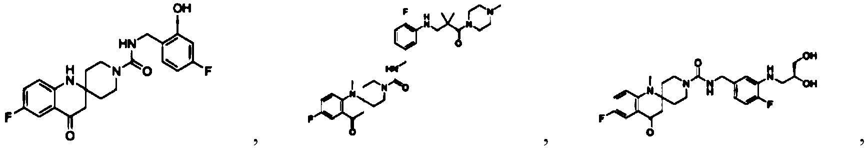

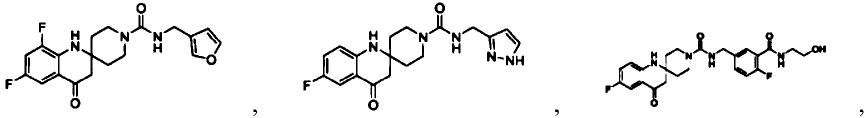

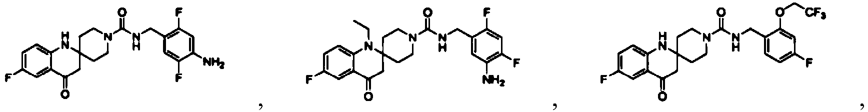

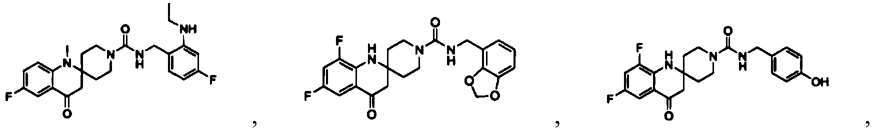

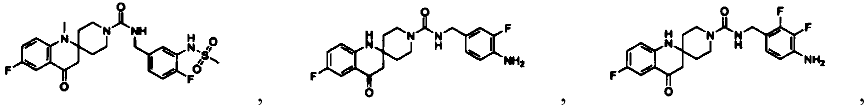

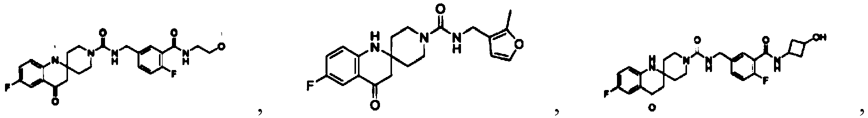

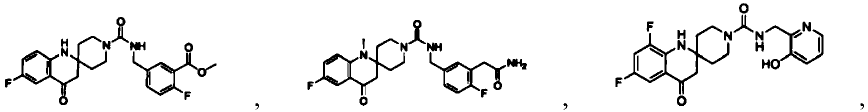

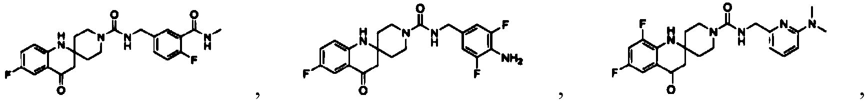

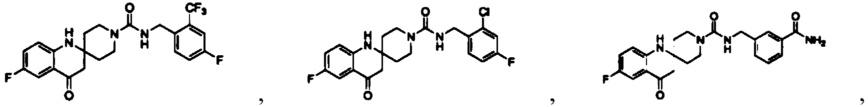

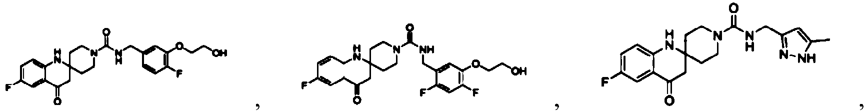

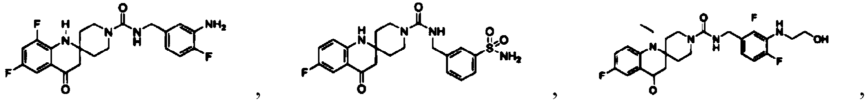

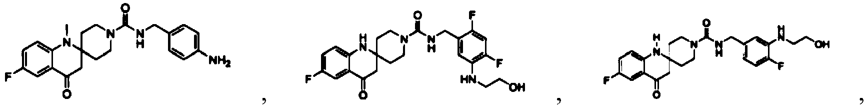

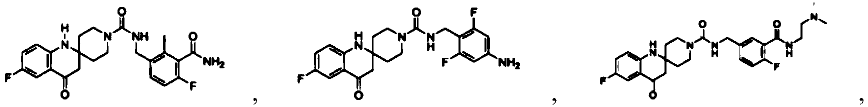

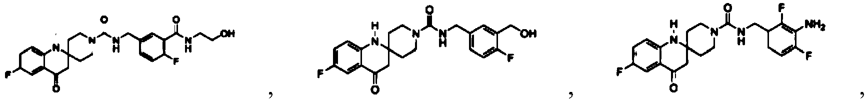

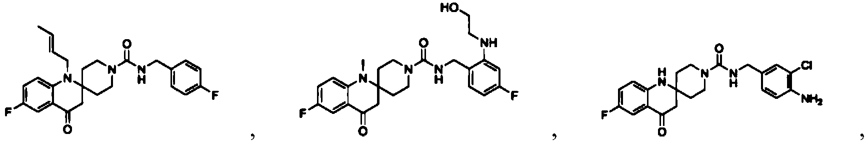

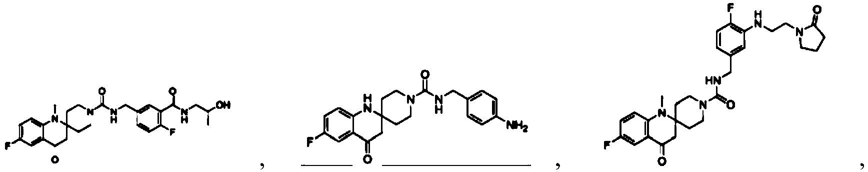

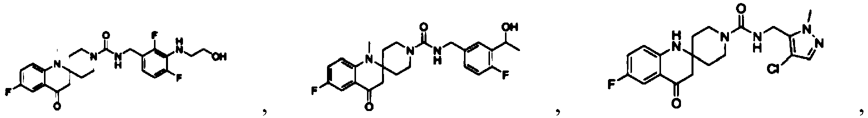

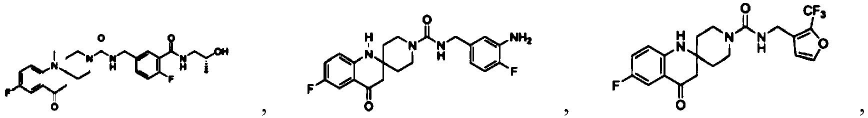

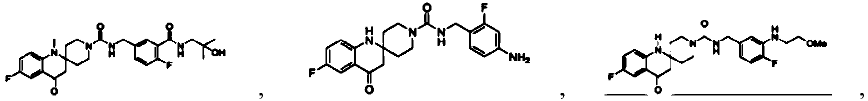

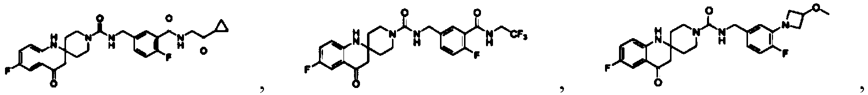

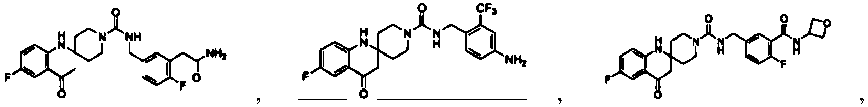

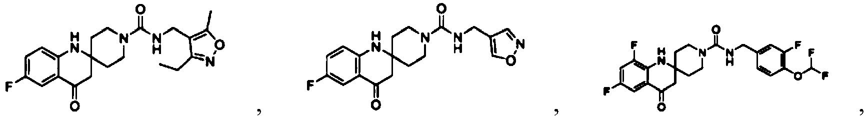

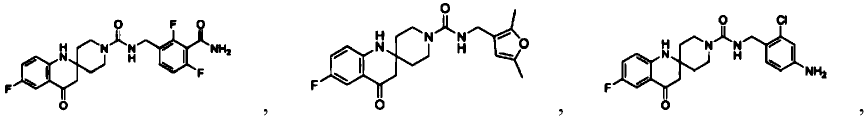

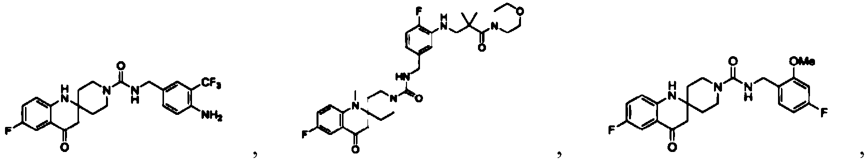

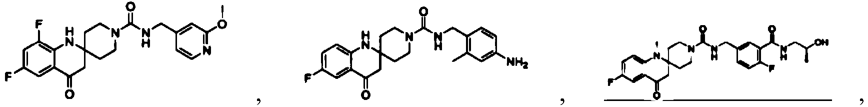

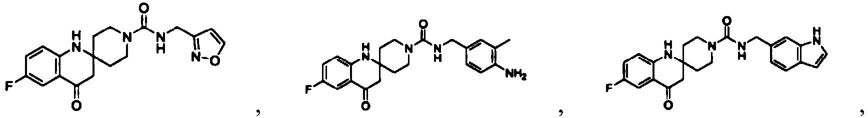

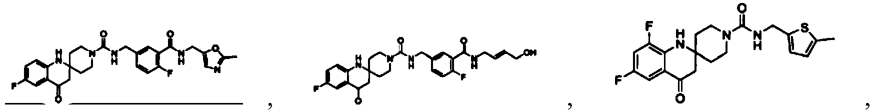

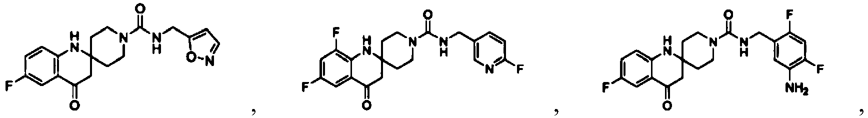

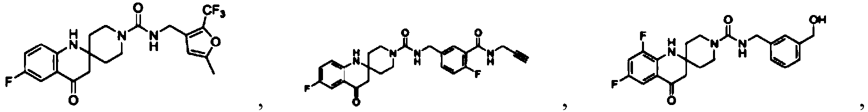

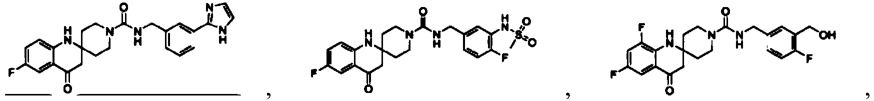

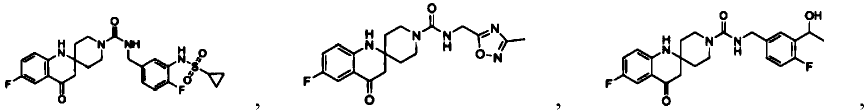

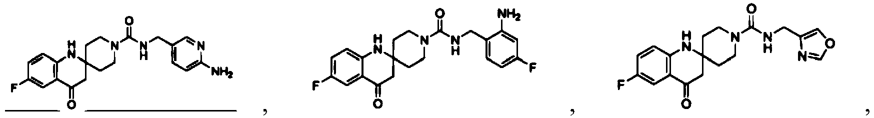

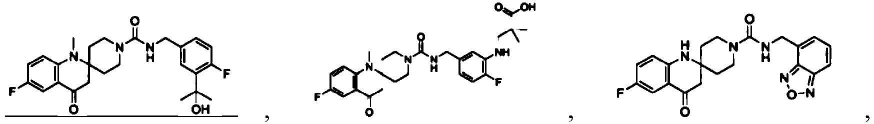

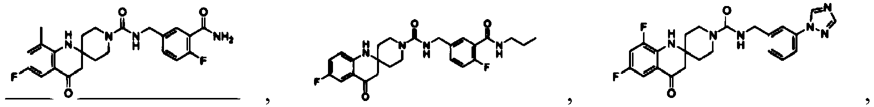

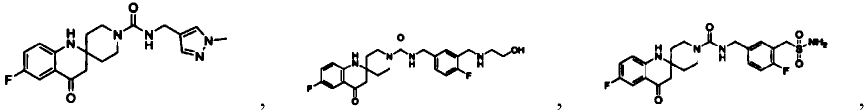

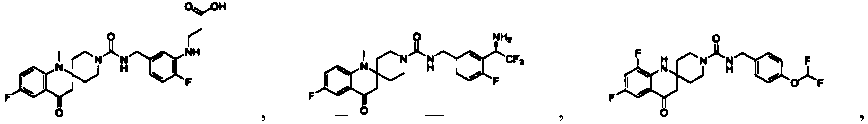

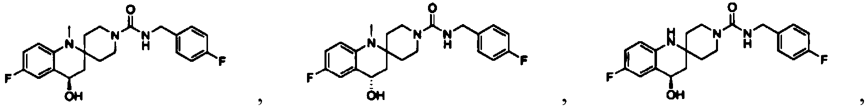

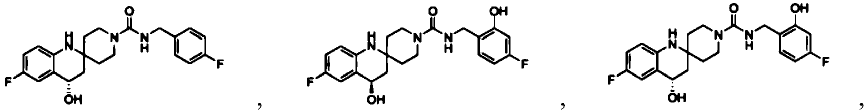

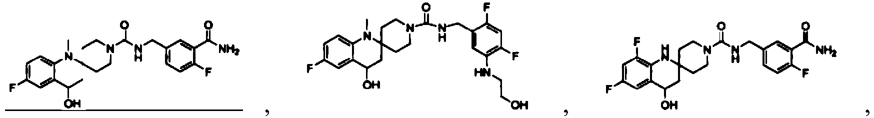

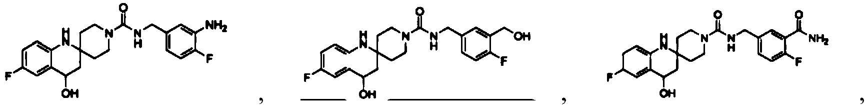

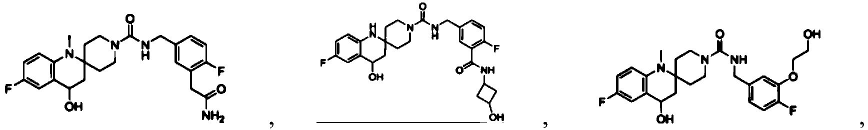

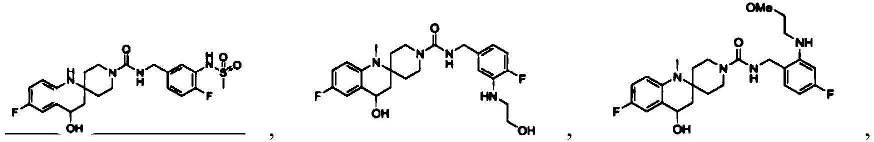

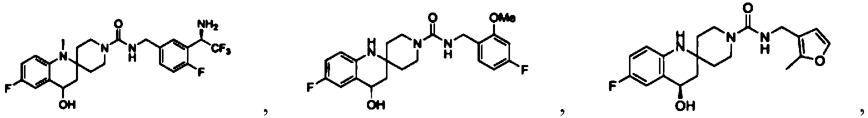

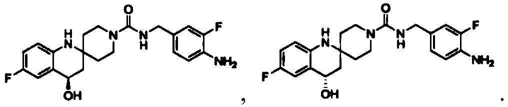

The specific synthesis method and corresponding spectral data of the compound of formula (11) are disclosed in WO2021005586A1 (corresponding to Chinese publication text CN114206870A), which is incorporated herein by reference in its entirety. 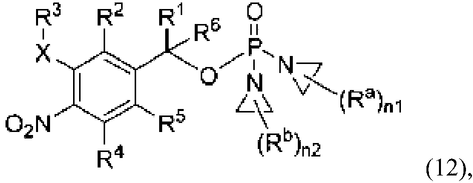 or a pharmaceutically acceptable salt thereof,
wherein the definitions of R¹, R², R³, R⁴, R⁵, R⁶, R^{a}, R^{b}, n1, and n2 are described in the claims of the Patent Application PCT/CN2023/123253 with Publication No. WO2024078392A1, and the synthesis and preparation methods of specific compounds are also described in the above patent application, which is incorporated herein by reference in its entirety. The specific definitions are as follows:
wherein:
(2) R¹ and R², together with the inserted atom, are attached to form an optionally substituted 4- to 8-membered carbocycle or heterocycle, and R⁴ and R⁵ are as defined in (1); or
(3) R¹ and R⁵, together with the inserted atom, are attached to form an optionally substituted 4- to 8-membered carbocycle or heterocycle, and R² and R⁴ are as defined in (1); or
(4) R⁴ and R⁵, together with the inserted atom, are attached to form an optionally substituted 4- to 8-membered carbocycle or heterocycle, and R¹ and R² are as defined in (1) or (2); or
(1) R¹ is hydrogen, deuterium, optionally substituted C₁₋₄ alkyl, optionally substituted C₂₋₄ alkenyl, or optionally substituted C₂₋₄ alkynyl;
Each of R², R⁴, and R⁵ is independently hydrogen, halogen (e.g., F), optionally substituted C₁₋₄ alkyl, optionally substituted C₂₋₄ alkenyl, optionally substituted C₂₋₄ alkynyl, optionally substituted C₁₋₄ alkoxy, or optionally substituted 3- to 5-membered ring;
wherein:
   X is O, S, NR¹⁰, optionally substituted C₁₋₄ alkylene, or optionally substituted C₁₋₄ heteroalkylene, wherein R¹⁰ is hydrogen, optionally substituted C₁₋₄ alkyl, optionally substituted 3- to 6-membered ring, or a nitrogen-protecting group;
   R³ is hydrogen, optionally substituted C₁₋₄ alkyl, or optionally substituted 3- to 10-membered ring;
   R⁶ is hydrogen, deuterium, optionally substituted C₁₋₄ alkyl, optionally substituted C₂₋₄ alkenyl, or optionally substituted C₂₋₄ alkynyl;
   Each of the integers n1 and n2 is independently 0, 1, 2, 3, or 4;
   Each occurrence of R^{a} and R^{b} is independently optionally substituted C₁₋₄ alkyl or optionally substituted C₁₋₄ heteroalkylene; or two instances of R^{a} or two instances of R^{b}, together with the inserted atoms, are attached to form an optionally substituted 3-to 6-membered ring, and any remaining instances of R^{a} and/or R^{b} are as defined above.

The AKR1C3 enzyme-activated compounds of formula (12) are selected from compounds 1-569 in tables 1-15 of the Patent Application PCT/CN2023/123253 with Publication No. WO2024078392A1, and the structures of some compounds are listed as follows:

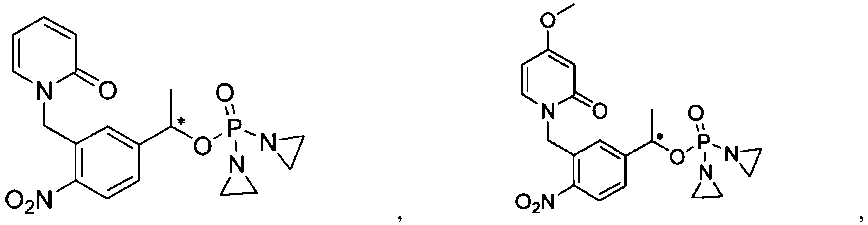

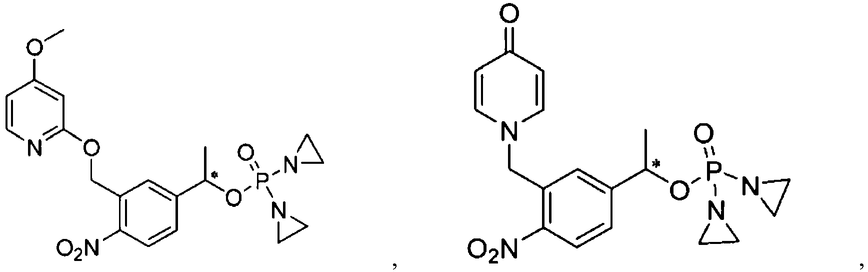

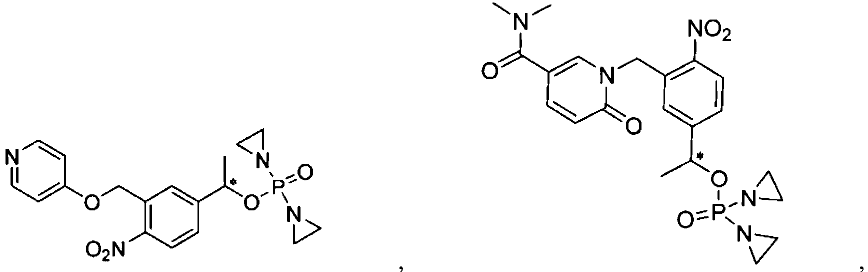

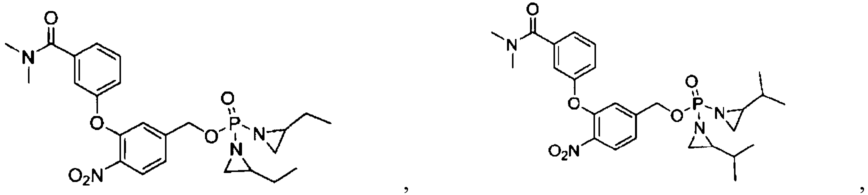

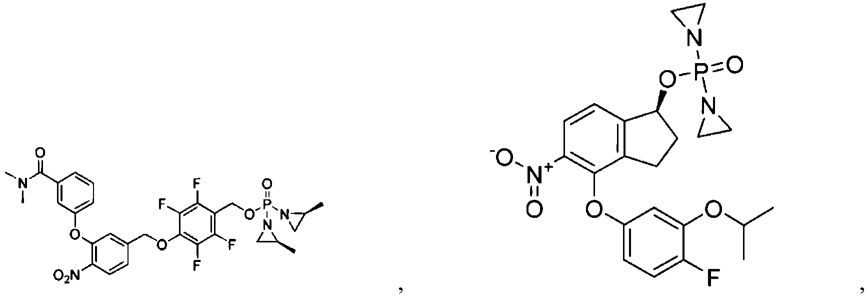

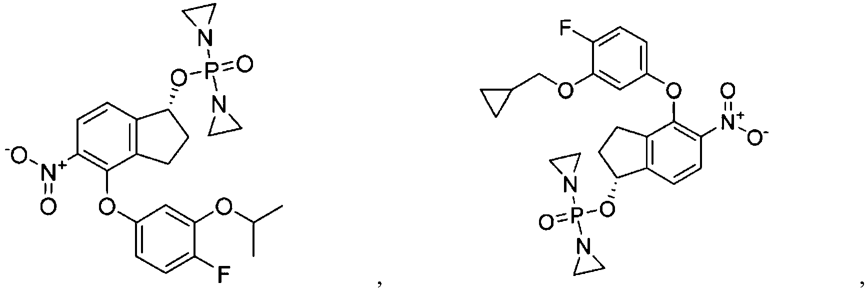

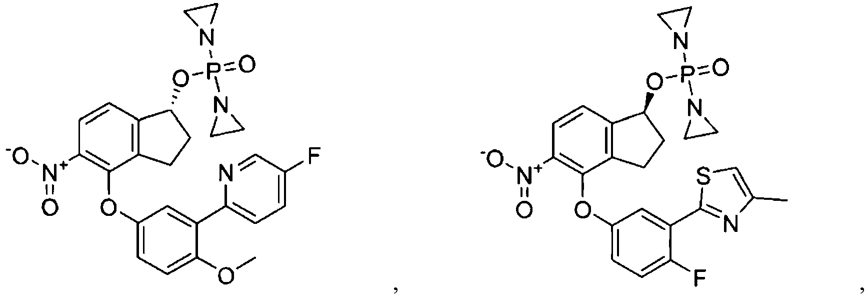

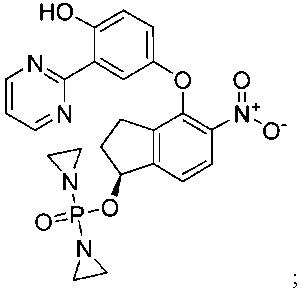

The specific synthesis methods and corresponding spectral data of compounds 1-569 of formula (12) are disclosed in WO2024078392A1, which is incorporated herein by reference in its entirety.

When the AKR1C3 enzyme-activated prodrug is selected from compounds of formula (1), (3)-(8), (12), or a pharmaceutically acceptable salt, solvate, or isotopic isomer thereof,
the tumor or cancer tissue of the patient is detected to have homology-dependent recombination repair deficiency (HRD), or the patient is detected to have homology-dependent recombination repair deficiency (HRD).

Having homology-dependent recombination repair deficiency must be met any one or both of the following conditions:
mutation occurs in one or more genes related to homology-dependent recombination repair (HRR) process;
genomic scar (GS) score reaches a predetermined value.

Cellular DNA damage can be divided into DNA single strand break (SSB) and DNA double strand break (DSB). Among them, SSB is mainly repaired by poly ADP-ribose polymerase (PARP) through nucleotide excision repair (NER), base excision repair (BER), and mismatch repair (MMR); DSB is repaired through pathways such as homologous recombination repair (HRR), Non-homologous End Joining (NHEJ), and Microhomology-mediated end joining (MMEJ).

Homology-dependent recombination repair (HRR) is a high-fidelity repair method and one of the core DNA damage repair methods. It mainly occurs in the S and G2 phases of the cell cycle, and is a DNA repair mechanism that maintains genomic integrity and ensures the high-fidelity transmission of genetic information. This process involves many genes, including ATM, ATR, BARD1, BLM, BRCA1, BRCA2, BRIP1, CDK12, CHEK1, CHEK2, FAAP20, FAN1, FANCE, FANCL, FANCM, MRE11A, NBN, PALB2, POLQ, PPP2R2A, RAD51B, RAD51C, RAD51D, RAD54L, RBBP8, SLX4, XRCC2 and other genes.

Homologous recombination repair deficiency (HRD) is a biological event caused by various factors such as genetic and epigenetic inactivation of genes in the homology-dependent recombination repair pathway, and is widely present in various tumors. When DNA is damaged, if it cannot be normally repaired through the homology-dependent recombination repair (HRR) pathway, it is homology-dependent recombination repair deficiency (HRD).

HRD can lead to the phenomenon of "genomic scars", including Loss of Heterozygosity (LOH), Telomeric Allelic Imbalance (TAI), Large-Scale State Transition (LST), etc.

When double-strand damage and homology-dependent recombination repair deficiency (HRD) occur simultaneously, cells will initiate non-high-fidelity repair methods for double-strand damage repair, such as Non-homologous End Joining (NHEJ) and Microhomology-mediated End Joining (MMEJ). This will lead to gene insertions/deletions and other errors, resulting in high instability on a genome-wide scale, which is "Genomic Scar (GS)". Therefore, the phenomenon of genomic instability caused by HRD is called the phenomenon of "Genomic Scar (GS)".

Genomic scars mainly include three types: Loss of Heterozygosity (LOH), Telomeric Allelic Imbalance (TAI), and Large-Scale State Transition (LST). Through the combined detection of the three types, the status of genomic instability can be maximized, thereby reversely deducing whether HRD exists in cells.

In summary, homology-dependent recombination repair mutation (HRRm) is the cause of homology-dependent recombination repair deficiency (HRD), while Genomic Scar (GS) is the manifestation of HRD: homology-dependent recombination repair mutation (HRRm) will lead to homology-dependent recombination repair deficiency (HRD), thereby showing Genomic Scar (GS).

Therefore, homology-dependent recombination repair deficiency (HRD) can be determined based on one or both of the following conditions: detecting mutation in one or more genes related to the homology-dependent recombination repair (HRR) process, and/or the Genomic Scar (GS) score reaching a predetermined value.

However, since DNA double strand break (DSB) mainly relies on homology-dependent recombination repair (HRR) and can also be repaired through Non-homologous End Joining (NHEJ) and Microhomology-mediated End Joining (MMEJ) pathways, the detection of HRRm alone does not necessarily result in HRD 100%; and since mutations in genes of repair pathways such as Non-homologous End Joining (NHEJ) and Microhomology-mediated End Joining (MMEJ) may also lead to the occurrence of GS, the detection of GS alone does not necessarily 100% determine that the GS is related to HRRm.

Therefore, the combined detection of HRRm and GS will more comprehensively evaluate the status of HRD.

Two detection kits have been approved: FoundationFocus CDx BRCA LOH Assay and Myriad Genetics myChoice^{®} HRD. These two HRD detection products designed based on Western populations have been clinically validated and approved by the U.S. Food and Drug Administration (FDA), while no HRD companion diagnostic products have been launched in China.

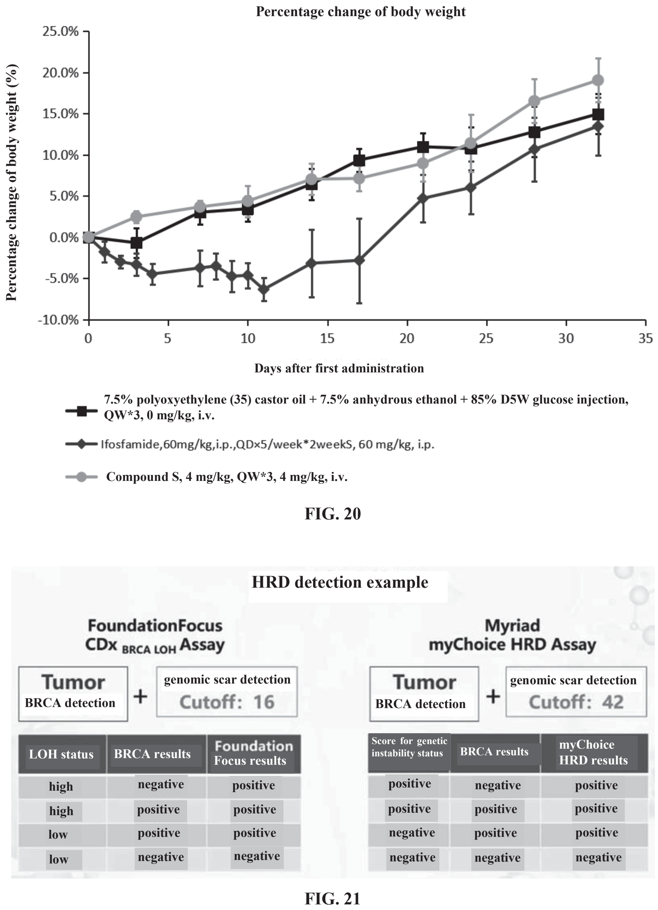

The details of the two HRD detection kits are shown in FIG. 21 (this figure is sourced from the academic report "From BRCA to HRD Detection" by Professor Ouyang Nengtai at the Academic Seminar of the Gynecological Oncology Molecular Diagnosis and Treatment Professional Committee of the Chinese Southern Oncology Clinical Research Association on January 27, 2021).

When detecting HRD, it is necessary to simultaneously detect whether mutation occur in one or more genes related to the homology-dependent recombination repair (HRR) process, and whether the Genomic Scar (GS) score reaches a predetermined value. HRD positivity is determined only when both meet certain conditions.

The HRRm genes detected by the two HRD detection kits approved by the U.S. FDA are BRCA. In fact, other genes related to the homology-dependent recombination repair process can also be selected from ATM, ATR, BARD1, BLM, BRCA1, BRCA2, BRIP1, CDK12, CHEK1, CHEK2, FAAP20, FAN1, FANCE, FANCL, FANCM, MRE11A, NBN, PALB2, POLQ, PPP2R2A, RAD51B, RAD51C, RAD51D, RAD54L, RBBP8, SLX4, XRCC2.

The status of genomic scar detection also varies. FoundationFocus CDx BRCA LOH Assay detects LOH status, while Myriad Genetics myChoice^{®} HRD detects the comprehensive status of LOH, TAI, and LST.

The aforementioned compounds (1), (3)-(8), and (12) are activated by the AKR1C3 enzyme highly expressed in tumor cells *in vivo* to release DNA alkylating agents to exert their effects:
The DNA alkylating agent released after AKR1C3 enzyme activation of compounds (1), (3)-(7), and (12) is 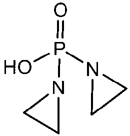 (AST-2660);
The DNA alkylating agent released after AKR1C3 enzyme activation of compound (8) is 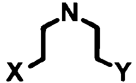 (a nitrogen mustard analogue);

The cytotoxic compounds of these drugs after metabolic activation are all DNA alkylating agents, which act on DNA double strands to cause cross-linking, thereby leading to DNA double strand break (DSB). In other words, the final effect of the aforementioned compounds (1)-(8) and (12) is to induce DNA double-strand damage and further kill cancer cells.

DSB is mainly repaired through the homology-dependent recombination repair (HRR) pathway. When DNA is damaged, if it cannot be normally repaired through the homology-dependent recombination repair (HRR) pathway, it is homology-dependent recombination repair deficiency (HRD).

Therefore, the aforementioned compounds should have more excellent therapeutic effects on tumor models with homology-dependent recombination repair deficiency (HRD) compared with non-mutated tumor models, that is, AKR1C3-activated DNA alkylating agent prodrugs have more significant therapeutic effects on cancer and tumor patients with HRD positivity. *In vitro* cell proliferation inhibitory experiments and animal model experiments also support this.

Regarding the drug or formulation described herein, the prepared drugs contain the indicated compounds or salts or solvates thereof in a specific dosage range, and/or the prepared drugs are administered in a specific dosage form and specific mode of administration.

The drug or formulation described herein may also contain pharmaceutically acceptable excipients or carriers. The drugs can be any dosage form for clinical administration, such as tablets, suppositories, dispersible tablets, enteric-coated tablets, chewable tablets, orally disintegrating tablets, capsules, sugar-coated tablets, granules, dry powders, oral solutions, small injection for injection, freeze-dried powder injection for injection, or large-volume injections. Depending on the specific dosage form and the mode of administration, the pharmaceutically acceptable excipients or carriers in the drugs may include one or more of the following: diluents, solubilizers, disintegrants, suspending agents, lubricants, binders, fillers, flavoring agents, sweeteners, antioxidants, surfactants, preservatives, coating agents, and pigments, etc.

"Administering" or "administration of" a drug shall achieve a "therapeutically effective amount". "Therapeutically effective amount" of a drug refers to an amount of a drug that, when administered to a patient with cancer, will have the intended therapeutic effect (e.g., alleviation, amelioration, palliation, or elimination of one or more clinical manifestations of cancer in the patient). A therapeutic effect does not necessarily occur by administration of one dose, and may occur only after administration of a series of doses. Thus, a therapeutically effective amount may be administered in one or more administrations.

"Administering" or "administration of" or "use of" a drug to a patient refers to direct administration, which may be administered to a patient by a medical professional or may be self-administered, and/or indirect administration, which may be the act of prescribing a drug. For example, a physician who instructs a patient to self-administer a drug and/or provides a patient with a prescription for a drug is administering the drug to the patient.

"Treatment" or "treatment of a patient" is to administer, use or administer a therapeutic effective amount of a drug to a patient in relation to the present invention.

"Treating", "treatment of" or "therapy of" a condition or a patient refers to taking steps to obtain a beneficial or desired result (including clinical results). For purposes of the present invention, beneficial or desired clinical results include, but are not limited to, alleviation or amelioration of one or more symptoms of cancer; diminishment of extent of disease; delay or slowing of disease progression; amelioration, remission, or stabilization of disease status; or other beneficial results. In some cases, treatment of cancer can result in partial response or stable disease.

"Tumor cells" or "cancer cells" refers to tumor cells/cancer cells of any appropriate species, e.g., mammalian such as murine, canine, feline, equine or human.

The subject of "treating cancer or tumor" refers to any appropriate species: mammalian, fish, such as mammalian, such as murine, canine, feline, equine or human. Preferably, the patient is a mammal, more preferably a human.

"Patient" and "individual" are used interchangeably and refer to a mammal in need of treatment for cancer. Typically, the patient is a human. In certain embodiments, a "patient" or "individual" may refer to a non-human mammal, such as a non-human primate, dog, cat, rabbit, pig, mouse, or rat used for screening, characterizing, and evaluating drugs and therapies.

"Prodrug" refers to a compound that, after administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor. A prodrug may be synthesized using reactants other than the corresponding drug.

The salt is a basic salt or an acid salt, and the solvate is a hydrate or an alcoholate.

The compounds also include the salt forms of the structures of formula I and formula II, that is, the present invention provides pharmaceutically acceptable salts of the compounds. The salts may be basic salts, including salts formed by the compounds with inorganic bases (e.g., alkali metal hydroxides, alkaline earth metal hydroxides, etc.) or with organic bases (e.g., monoethanolamine, diethanolamine, triethanolamine, etc.). Alternatively, the salts may be acid salts, including salts formed by the compounds with inorganic acids (e.g., hydrochloric acid, hydrobromic acid, hydroiodic acid, nitric acid, perchloric acid, sulfuric acid, phosphoric acid, etc.) or with organic acids (e.g., methanesulfonic acid, trifluoromethanesulfonic acid, ethanesulfonic acid, benzenesulfonic acid, p-toluenesulfonic acid, fumaric acid, oxalic acid, maleic acid, citric acid, etc.). The selection and preparation of acceptable salts, solvates, etc., of the compounds are well-known technology in the art.

The compounds described herein can also be used in the form of solvates, which are hydrates, alcoholates, etc., and alcoholates include ethanolates.

The concept of isotopic variants or isomers in the present invention shall prevail as the concept of isotopic variants and chiral isomers in the Patent Application PCT/US2016/062114 with Publication No. WO2017087428, corresponding to Chinese Application No. 2016800446081 with Publication No. CN108290911A.

For terms not specifically explained or illustrated in the text of this application, reference shall be made to textbooks of medicinal chemistry, organic chemistry, biochemistry, and pharmacology.

### Brief description of the drawings

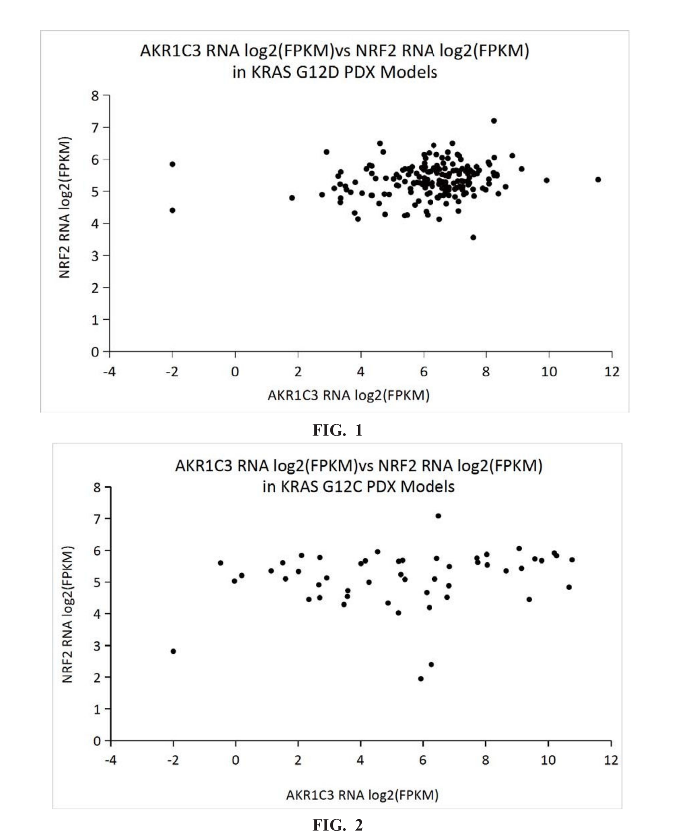

FIG. 1 is a diagram showing the corresponding relationship between the RNA expression level of AKR1C3 and the RNA expression level of NRF2 in a model with KRAS-G12D mutation.
FIG. 2 is a diagram showing the corresponding relationship between the RNA expression level of AKR1C3 and the RNA expression level of NRF2 in a model with KRAS-G12C mutation.

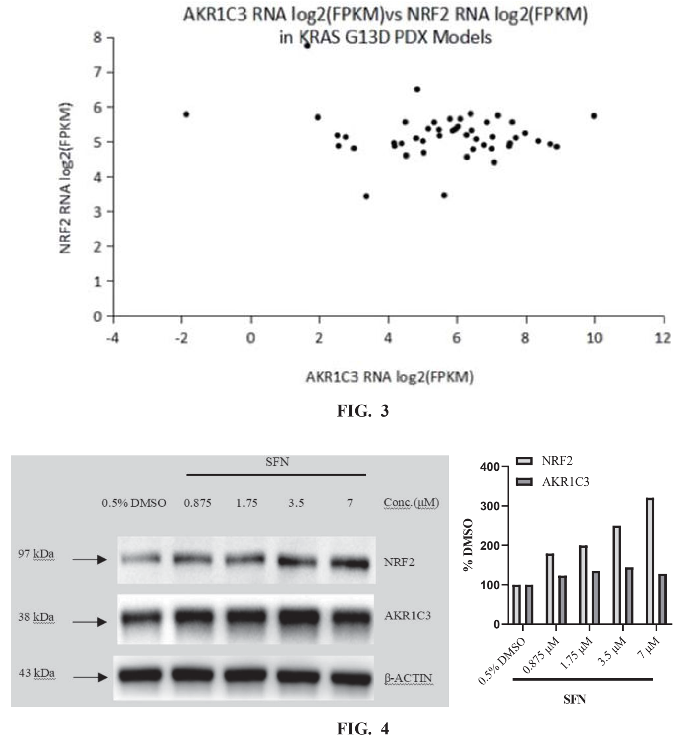

FIG. 3 is a diagram showing the corresponding relationship between the RNA expression level of AKR1C3 and the RNA expression level of NRF2 in a model with KRAS-G13D mutation.
FIG. 4 shows the results of Western Blot assay of target proteins after cell lysis under different concentrations of SFN treatment. Among them, the left panel is the Western Blot image under different concentrations of SFN treatment, and the right panel is a bar graph of the protein band density analysis of NRF2 and AKR1C3. In each concentration group, the left bar represents NRF2 and the right bar represents AKR1C3.

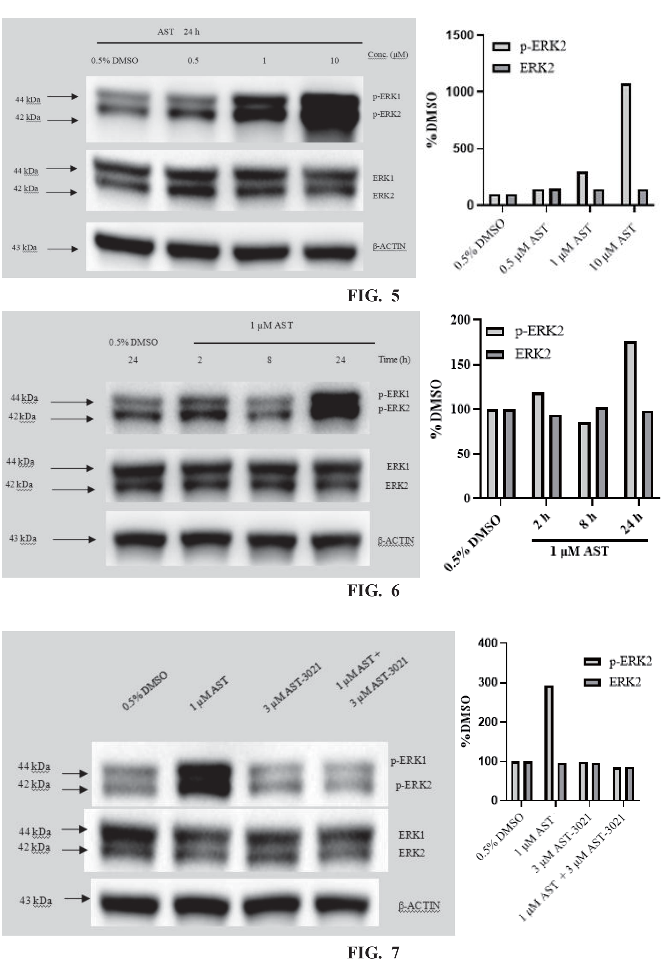

FIG. 5 shows the results of Western Blot assay of target proteins after cell lysis under different concentrations of AST treatment. Among them, the left panel is the Western Blot image under different concentrations of AST treatment, and the right panel is a bar graph of the protein band density analysis of p-ERK2 and ERK2 under different concentrations of AST treatment. In each AST concentration group, the left bar represents p-ERK2 and the right bar represents ERK2.
FIG. 6 shows the results of Western Blot assay of target proteins after cell lysis under treatment with 1 µM AST for different times. Among them, the left panel is the Western Blot image under treatment with 1 µM AST for different times, and the right panel is a bar graph of the protein band density analysis of p-ERK2 and ERK2 under treatment with 1 µM AST for different times. In each treatment time group, the left bar represents p-ERK2 and the right bar represents ERK2.
FIG. 7 shows the results of Western Blot assay of target proteins after AKR1C3-dependent cell lysis. Among them, the left panel is the AKR1C3-dependent Western Blot image, and the right panel is a bar graph of the AKR1C3-dependent protein band density analysis of p-ERK2 and ERK2. In each treatment group, the left bar represents p-ERK2 and the right bar represents ERK2.

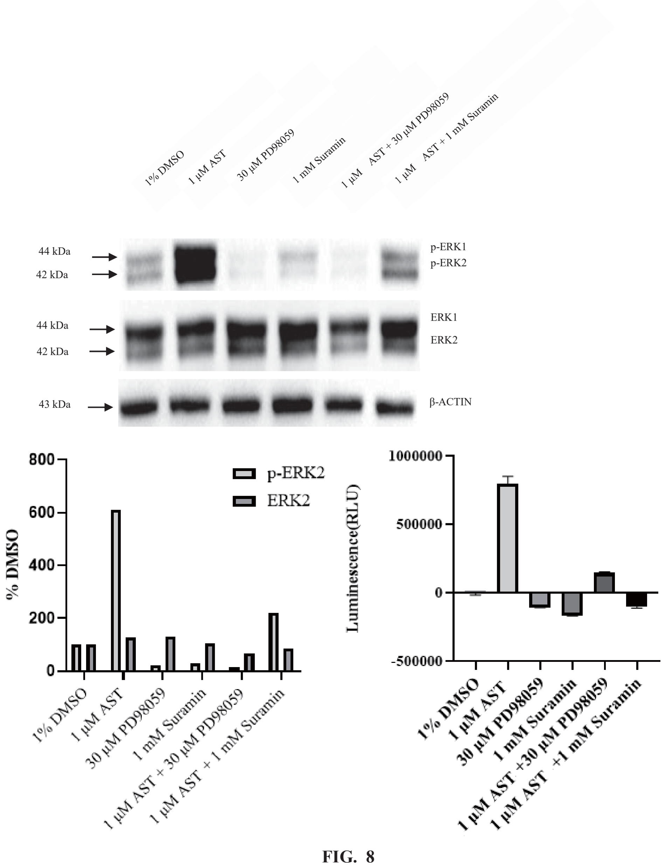

FIG. 8 shows the results of Western Blot assay of target proteins and cell apoptosis assay after cell lysis in the ERK2 inhibitor, AST single-agent, and combination therapy groups. Among them, the upper panel is the Western Blot image of the ERK2 inhibitor groups (PD98059, Suramin) as single agents or in combination; and the lower left panel is a bar graph of the protein band density analysis of the ERK2 inhibitor groups (PD98059, Suramin) as single agents or in combination. In each treatment group, the left bar represents p-ERK2 and the right bar represents ERK2; the lower right panel is a bar graph of cell apoptosis of the ERK2 inhibitor groups (PD98059, Suramin) as single agents or in combination, wherein Luminescence (RLU) indicates the relative luminescence unit of apoptotic signals.

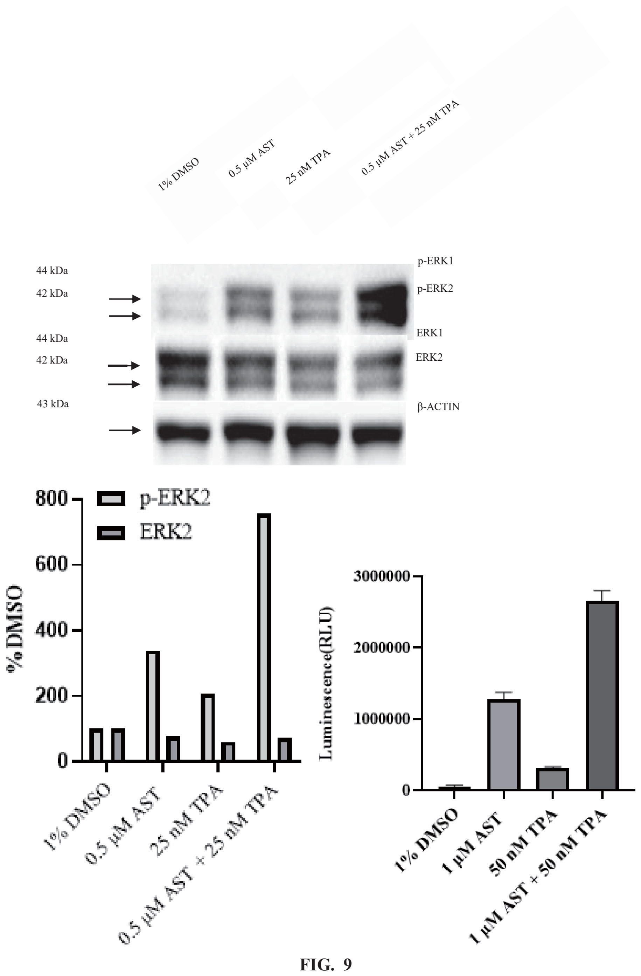

FIG. 9 shows the results of Western Blot assay of target proteins and cell apoptosis assay after cell lysis in the ERK2 activator, AST single-agent, and combination therapy groups. Among them, the upper panel is the Western Blot image of the ERK2 activator group (TPA) as a single agent or in combination; the lower left panel is a bar graph of the density analysis of the ERK2 activator group (TPA) as a single agent or in combination. In each treatment group, the left bar represents p-ERK2 and the right bar represents ERK2; the lower right panel is a bar graph of cell apoptosis of the ERK2 activator group (TPA) as a single agent or in combination, wherein Luminescence (RLU) indicates the relative luminescence unit of apoptotic signals.

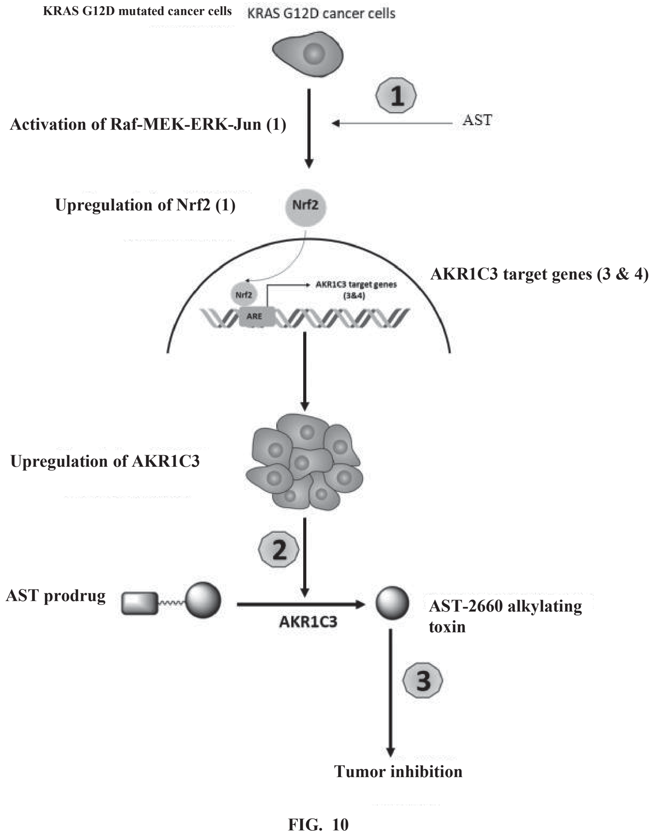

FIG. 10 is a schematic diagram of the process by which AST induces apoptosis of cancer cells with KRAS G12D pathogenic mutation through the ERK2 signaling pathway. Among them, (1), and (3&4) indicate relevant supporting literatures:
   (1) Prior IA, Hood FE and Hartley JL. The Frequency of Ras Mutations in Cancer. Cancer Res 2020; 80: 2969-2974;
   (3) Park HE, Yoo SY, Cho NY, Bae JM, Han SW, Lee HS, Park KJ, Kim TY and Kang GH. Tumor microenvironment-adjusted prognostic implications of the KRAS mutation subtype in patients with stage III colorectal cancer treated with adjuvant FOLFOX. Sci Rep 2021; 11: 14609;
   (4) Patricelli MP, Janes MR, Li LS, Hansen R, Peters U, Kessler LV, Chen Y, Kucharski JM, Feng J, Ely T, Chen JH, Firdaus SJ, Babbar A, Ren P and Liu Y. Selective Inhibition of Oncogenic KRAS Output with Small Molecules Targeting the Inactive State. Cancer Discov 2016; 6: 316-329;
   ① indicates that the AST compound acts on the MEK-ERK signaling pathway and activates it;
   ② the AKR1C3 enzyme activates the AST prodrug compound and ultimately releases the DNA alkylating agent AST-2660;
   ③ the proliferation of tumor cells is inhibited by AST-2660.
FIG. 11 shows the results of AKR1C3 expression levels measured by Western Blot assay in Capan-1, PLC/PRF/5, HT29, and H460 cell lines.
FIG. 12 shows the results of AKR1C3 expression levels measured by Western Blot assay in Capan-1, HepG2, and H460 cell lines.
FIG. 13 is a growth curve of tumor volume in mice of each group in the *in vivo* efficacy experiment of AST-3424 in the Capan-1 CDX model with BRCA pathogenic mutation.
FIG. 14 is a change curve of body weight of mice of each group in the *in vivo* efficacy experiment of AST-3424 in the Capan-1 CDX model with BRCA pathogenic mutation.
FIG. 15 is a growth curve of tumor volume in mice of each group in the *in vivo* efficacy experiment of AST-3424 in the non-BRCA pathogenic mutation model HepG2-GFP.
FIG. 16 is a change curve of body weight of mice of each group in the *in vivo* efficacy experiment of AST-3424 in the non-BRCA pathogenic mutation model HepG2-GFP.
FIG. 17 is a growth curve of tumor volume in mice of each group in the *in vivo* efficacy experiment of compound S in the BRCA pathogenic mutation model HuPrime^{®} gastric cancer GA6201.
FIG. 18 is a curve of the percentage change in body weight of mice of each group during treatment in the *in vivo* efficacy experiment of compound S in the BRCA pathogenic mutation model HuPrime^{®} gastric cancer GA6201.
FIG. 19 is a growth curve of tumor volume in mice of each group in the *in vivo* efficacy experiment of compound S in the non-BRCA pathogenic mutation model HuPrime^{®} lung cancer LU5173.
FIG. 20 is a curve of the percentage change in body weight of mice of each group during treatment in the *in vivo* efficacy experiment of compound S in the non-BRCA pathogenic mutation model HuPrime^{®} lung cancer LU5173.
FIG. 21 is a schematic diagram of the detection content and positive judgment criteria of two FDA-approved HRD detection kits: FoundationFocus CDx BRCA LOH Assay and Myriad Genetics myChoice^{®} HRD.

### Detailed description of the invention

The present invention will be described below with reference to specific examples. Those skilled in the art can understand that these examples are only used to illustrate the present invention and do not limit the scope of the present invention in any way.

The experimental methods in the following examples are conventional methods unless otherwise specified. The medicinal raw materials, reagent materials, etc. used are commercially available products unless otherwise specified.

The above description of the specific embodiments of the present invention does not limit the present invention. Those skilled in the art can make various changes or modifications according to the present invention, as long as they do not depart from the spirit of the present invention, they shall all belong to the scope of the appended claims of the present invention.

Specific experiments/examples related to the present invention are provided below to illustrate the present invention.

AST-3424:

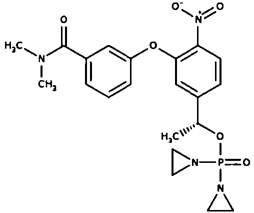

AST-3424 analog compound AST (referred to as compound S in some examples):

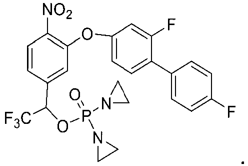

### 1. Distribution of AKR1C3 in KRAS G12D PDXs and correlation statistics with NRF2

A total of 179 model data with KRAS-G12D mutation were derived from the public tumor PDX model gene database of CrownBio (https://www.crownbio.cn/model-systems/in-vivo/pdx-models/), and the RNA expression level of AKR1C3 in these models was counted. The results were shown in Table 1:

**Table 1: Statistical results of the corresponding relationship between the expression level grading of AKR1C3 RNA (expressed as LOG2(FPKM)) and KRAS-G12D**

| AKR1C3 RNA Expression | AKR1C3 RNA Expression Level | % Distribution in KRAS G12D mutant PDXs |
|---|---|---|
| ≥8 | H | 8.9% |
| 6-7.9 | M-H | 57.5% |
| 4-5.9 | M | 24.0% |
| 2-3.9 | M-L | 7.8% |
| <2 | L | 1.7% |
| Total #PDX | | ADC #PDX 179 |

Furthermore, the NRF2 expression level of these KRAS-G12D mutation models was statistically analyzed. The RNA expression level of AKR1C3 and the RNA expression level of NRF2 in these 179 PDX models were plotted on an X-Y coordinate graph, obtaining FIG. 1.

The distribution trend of AKR1C3 expression level in KRAS G12D PDX models was mainly concentrated in medium and high expressions (LOG2(FPKM) ≥ 4), accounting for 90.4%.

In KRAS G12D PDX models, the distribution trend of NRF2 expression level was also concentrated in medium and high expressions, consistent with the protein expression level trend of AKR1C3, showing a certain correlation.

### 2. Distribution of AKR1C3 in KRAS G12C PDXs and correlation statistics with NRF2

A total of 51 model data with KRAS-G12C mutation were derived from the public tumor PDX model gene database of CrownBio (https://www.crownbio.cn/model-systems/in-vivo/pdx-models/), and the RNA expression level of AKR1C3 in these models was counted. The results were shown in Table 2:

**Table 2: Statistical results of the corresponding relationship between the expression level grading of AKR1C3 RNA (expressed as LOG2(FPKM)) and KRAS-G12C**

| AKR1C3 RNA Expression | AKR1C3 RNA Expression Level | % Distribution in KRAS G12C mutant PDXs |
|---|---|---|
| ≥ 8 | H | 23.5% |
| 6-7.9 | M-H | 21.6% |
| 4-5.9 | M | 21.6% |
| 2-3.9 | M-L | 19.6% |
| <2 | L | 13.7% |
| Total #PDX | | ADC #PDX 51 |

Furthermore, the NRF2 expression level of these KRAS-G12C mutation models was statistically analyzed. The RNA expression level of AKR1C3 and the RNA expression level of NRF2 in these 51 PDX models were plotted on an X-Y coordinate graph, obtaining FIG. 2.

The AKR1C3 expression level in the KRAS G12C PDX models was equally distributed in low, medium and high expressions, wherein the expression levels above medium expression levels (LOG2(FPKM) ≥ 4) account for 66.7%.

In KRAS G12C PDX models, the distribution trend of NRF2 expression level had a weak correlation with the protein expression level distribution of AKR1C3.

### 3. Distribution of AKR1C3 in KRAS G13D PDXs and correlation statistics with NRF2

A total of 41 model data with KRAS-G13D mutation were derived from the public tumor PDX model gene database of CrownBio (https://www.crownbio.cn/model-systems/in-vivo/pdx-models/), and the RNA expression level of AKR1C3 in these models was counted. The results were shown in Table 3:

**Table 3: Statistical results of the corresponding relationship between the expression level grading of AKR1C3 RNA (expressed as LOG2(FPKM)) and KRAS-G13D**

| **AKR1C3 RNA Expression** | **AKR1C3 RNA Expression Level** | **% Distribution in KRAS G12C mutant PDXs** |
|---|---|---|
| ≥8 | H | 8.3% |
| 6-7.9 | M-H | 39.6% |
| 4-5.9 | M | 35.4% |
| 2-3.9 | M-L | 10.4% |
| <2 | L | 6.3% |
| Total #PDX | | ADC #PDX 48 |

Furthermore, the NRF2 expression level of these KRAS-G13D mutant models was statistically analyzed. The RNA expression level of AKR1C3 and the RNA expression levels of NRF2 in 48 PDX models were plotted on an X-Y coordinate graph, obtaining FIG. 3.

The AKR1C3 expression level in the KRAS G13D PDX models was equally distributed in low, medium and high expressions, wherein the expression levels above medium expression levels account for 83.3%.

In KRAS G13D PDX models, the distribution trend of NRF2 expression level had a certain correlation with the protein expression level distribution of AKR1C3.

The above statistical results revealed that NRF2 expression level was positively correlated with AKR1C3 expression level: high NRF2 expression often indicated high AKR1C3 expression; cells and tissues with KARS mutations often showed medium and high AKR1C3 expression. In summary, KRAS mutations (including KRAS-G13D, **KRAS G12C, and KRAS G12D**) were associated with medium and high expression of NRF2 and AKR1C3: KRAS mutations (including KRAS-G13D, **KRAS G12C, and KRAS G12D**) were often accompanied by medium and high expression of NRF2 and AKR1C3.

To further illustrate the relationship between KRAS mutations and NRF2, AKR1C3, the inventors conducted relevant mechanism research experiments.

### 4. Expression level of AKRIC3 in HPAF II tumor cells with KRAS G12D mutation was regulated by NRF2

SFN compound (Sulforaphane, an NRF2 activator) solution was added to HPAF II cell suspension, cultured overnight in a 37°C, 5% CO2 incubator, and cell protein lysate was collected for Western Blot (WB) detection.

The experiment was divided into 5 groups: 0.5% DMSO, 0.875 µM SFN, 1.75 µM SFN, 3.5 µM SFN, and 7 µM SFN.

HPAF II cells were treated with different concentrations of the NRF2 activator SFN, and then the expression level of NRF2 and AKR1C3 was detected by Western Blot. The results of Western Blot and protein band density analysis of NRF2 and AKR1C3 under different SFN concentrations were shown in FIG. 4.

Experimental results showed that with the increase of SFN treatment concentration, the expression level of NRF2 increased significantly, and the expression of AKR1C3 also increased simultaneously.

The experimental results showed that the NRF2 activator SFN upregulated NRF2 expression in a concentration-dependent manner. Under the same experimental conditions, the expression of AKR1C3 was simultaneously upregulated with the increase of SFN concentration. This result clearly showed that the expression of AKR1C3 was regulated by NRF2. In addition, combined with the significant correlation between the mRNA levels of NRF2 and AKR1C3 in the medium and high expression ranges in Examples 1-3, it indicated that AKR1C3 expression level was positively correlated with NRF2 expression level in PDX models with KRAS G12D mutation.

The AKR1C3 enzyme-activated prodrugs AST-3424 and AST had significant anti-tumor effects on PDX and CDX models with high AKR1C3 enzyme expression (Examples 1-9 of PCT/CN2022/120817 with Publication No. WO2023/046060) and were well-tolerated.

Combined with the experimental conclusion that AKR1C3 expression level was positively correlated with NRF2 expression level in Examples 1-3, those skilled in the art can reasonably deduce that KRAS gene mutations can upregulate or activate NRF2, thereby promoting high expression of AKR1C3, and further enabling the AKR1C3 enzyme-activated prodrugs AST-3424 and AST to exert significant anti-tumor effects.

The compounds of general formulae (1)-(12) in the present application are all AKR1C3 enzyme-activated anticancer prodrugs, and they are cleaved to produce anti-cancer active drugs such as AST-2660, paclitaxel, SN-38, gemcitabine, and KARS inhibitors, under the action of AKR1C3 enzyme. Therefore, those skilled in the art can reasonably deduce that KRAS gene mutations will lead to high expression of AKR1C3 enzyme by upregulating or activating NRF2, thereby enabling the compounds of general formulae (1)-(12) to be activated and metabolized into anti-cancer active drugs. That is to say, a patient who is detected to have a KRAS gene mutation can benefit from treatment with the compounds of general formulae (1)-(12), especially from treatment with AST-3424 or AST.

Taking the AKR1C3 enzyme-activated prodrug AST as an example, the following explores its mechanism of action in tumor cells with KRAS mutation, especially tumor cells with KRAS G12D mutation.

### 5. AST activates the MEK/ERK signaling pathway

AST-induced ERK phosphorylation experiments. Three groups of experiments were conducted with three sets of variables: AST concentration, AST action time, and AKR1C3 enzyme dependence. Among them, the AKR1C3 enzyme-dependent variable was based on the AKR1C3 enzyme-specific inhibitor AST-3021, and the experiment was performed in the presence or absence of the AKR1C3 enzyme-specific inhibitor AST-3021. The inhibitor used was AST-3021 (also known as TH-3021, which is compound 36 reported by Flanagan et al., in Bioorganic and Medicinal Chemistry (2014), pp. 962-977, with the structural formula:

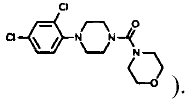

The experiment was divided into three groups, treated with different concentrations of AST (Group 1), 1 µM AST for different times (Group 2), or with/without the AKR1C3 inhibitor AST-3021 (Group 3), respectively.

The specific compounds to be treated in the three groups were as follows:
- Group 1 (concentration group): 0.5% DMSO treatment group (24h), 0.5 µM AST treatment group (24h), 1 µM AST treatment group (24h), 10 µM AST treatment group (24h);
- Group 2 (time group): 0.5% DMSO treatment group (24h), 1 µM AST treatment group (2h), 1 µM AST treatment group (8h), 1 µM AST treatment group (24h);
- Group 3 (AKR1C3-dependent group): 0.5% DMSO treatment group (24h), 1 µM AST treatment group (24h), 3 µM AST-3021 treatment group (24h), 1 µM AST + 3 µM AST-3021 treatment group (24h).

Compound solutions of each group were added to HPAF II cell suspensions respectively, cultured overnight in a 37°C, 5% CO₂ incubator. According to the experimental groups, cell protein lysates were collected for Western Blot (WB) detection.

Western Blot was used to detect the expression levels of NRF2 and AKR1C3. The results of Western Blot images and protein band density analysis of p-NRF2 and NRF2 in Group 1 treated with different concentrations of AST were shown in FIG. 5; the results of Western Blot images and protein band density analysis of p-NRF2 and NRF2 in Group 2 treated with 1 µM AST for different times were shown in FIG. 6; and the results of Western Blot images and protein band density analysis of p-NRF2 and NRF2 in Group 3 treated with/without the AKR1C3 inhibitor AST-3021 were shown in FIG. 7.

Experimental results showed that in FIG. 5, with the increase of AST treatment concentration, the expression level of p-NRF2 increased correspondingly, and when treated with 10 µM AST for 24h, the expression of p-NRF2 increased significantly, while AST treatment at all concentrations did not affect the expression level of NRF2; in FIG. 6, the expression level of p-NRF2 remained basically unchanged when treated with 1 µM AST for 2h and 8h, but significantly increased compared with the DMSO control group when treated for 24h; in Fig. 7, only the 1 µM AST single-agent treatment group (24h) showed a significant increase in p-NRF2 expression, while the p-NRF2 expression levels in the other groups (0.5% DMSO treatment group (24h), 3 µM AST-3021 treatment group (24h), and 1 µM AST + 3 µM AST-3021 treatment group (24h)) remained basically unchanged. In the above three groups of experiments, the expression of total ERK2 in all treatment groups did not change.

The above experiments showed that AST induced ERK2 phosphorylation in a concentration-dependent manner without affecting the expression level of ERK2. After 24 hours of treatment with 1 µmol/L AST, the phosphorylation level of ERK2 was significantly increased compared with the DMSO control group. As shown in FIG. 7, HPAF II cells were pretreated with the AKR1C3-specific inhibitor AST-3021, 2 hours before added with 1 µmol/L AST, and 3 µmol/L AST-3021 strongly inhibited the increase of AST-mediated phosphorylated ERK2. Treatment with AST-3021 alone did not affect the level of p-ERK2. Under all tested conditions, the expression of total ERK2 did not change. It was confirmed by actin Western Blot that loading of all samples was consistent. AST activated the MEK/ERK signaling pathway in a concentration, time, and AKR1C3-dependent manner.

### 6. AST induces apoptosis of KRAS G12D cells through the ERK2 signaling pathway

Three MEK/ERK modulators were used in this experiment, including two inhibitors and one activator. The inhibitors were PD98059, a specific inhibitor of MEK1/2, and Suramin, a broad-spectrum growth factor receptor inhibitor; the activator of the ERK signaling pathway was Phorbol Ester (TPA). The chemical structures of the inhibitor PD98059, inhibitor Suramin, and activator TPA are shown below:

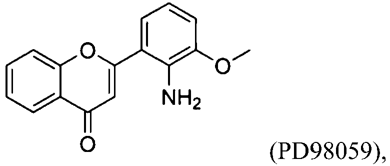

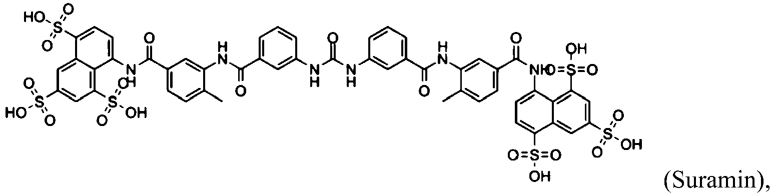

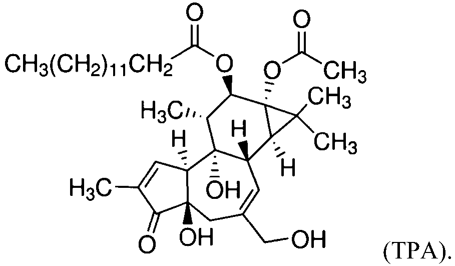

The experiment was divided into an inhibitor group and an activator group, both of which were subjected to Western Blot assays and cell apoptosis assays.

In the Western Blot assay, HPAF II cell suspensions were treated with compounds of each group respectively, cultured overnight in a 37°C, 5% CO₂ incubator, and cell protein lysates were collected for Western Blot (WB) detection.

The specific compounds to be treated in the inhibitor and activator groups for Western Blot were as follows:
- Inhibitor group: 1% DMSO treatment group, 1 µM AST treatment group, 30 µM PD98059 treatment group, 1 mM Suramin treatment group, 1 µM AST + 30 µM PD98059 treatment group, 1 µM AST + 1 mM Suramin treatment group;
- Activator group: 1% DMSO treatment group, 0.5 µM AST treatment group, 25 nM TPA treatment group, 0.5 µM AST + 25 nM TPA treatment group.

**In addition, cell apoptosis detection was performed:**
1) Caspase Glo^{®}3/7 reagent was prepared and equilibrated to room temperature. The buffer and substrate of the reagent were mixed at a ratio of 1:1 to prepare Caspase Glo^{®}3/7 reagent, which can be stored at 4°C for 3 days.
2) 100 µL of Caspase Glo^{®}3/7 reagent was added to each well of medium containing 100 µL of blank (cell-free wells), negative control cells, or treated cells. Due to the sensitivity of the assay, the pipette tip should be careful not to touch the wells containing samples to avoid cross-contamination. The plate was covered with a plate sealer or lid.
3) The reagent solution was gently mixed on a shaker at 300-500 rpm for 30 seconds, and placed at room temperature in the dark for 1 hour.
4) The luminescence of each sample was measured in a plate-reading luminometer. The luminescence value of each group was calculated and bar graph analysis was performed.

The specific compounds to be treated in the inhibitor and activator groups for cell apoptosis were as follows:
- Inhibitor group: 1% DMSO treatment group, 1 µM AST treatment group, 30 µM PD98059 treatment group, 1 mM Suramin treatment group, 1 µM AST + 30 µM PD98059 treatment group, 1 µM AST + 1 mM Suramin treatment group;
- Activator group: 1% DMSO treatment group, 1 µM AST treatment group, 50 nM TPA treatment group, 1 µM AST + 50 nM TPA treatment group.

The experimental results of Western Blot, protein band density analysis, and cell apoptosis of the ERK2 inhibitor, AST single-agent, and combination therapy groups were shown in FIG. 8; and the experimental results of Western Blot, protein band density analysis, and cell apoptosis of the ERK2 activator, AST single-agent, and combination therapy groups were shown in FIG. 9.

The experimental results showed in the inhibitor group of FIG. 8, the upper panel and lower left panel showed that treatment with PD98059 and Suramin alone slightly reduced the expression level of p-ERK2, while the combination therapy group of PD98059 and AST significantly reduced the expression level of p-ERK2, and the combination therapy group of Suramin and AST slightly increased the expression level of p-ERK2, but the AST single-agent group significantly increased the expression level of p-ERK2; the lower right panel showed that the AST single-agent group significantly affected cell apoptosis, while PD98059, Suramin single-agent groups, and their respective combination therapy groups with AST had almost no effect on cell apoptosis. In the activator group of Fig. 9, the upper panel and lower left panel showed that the AST and TPA single-agent treatment groups both increased the expression level of p-ERK2 compared with the DMSO control group, while the combination therapy group of AST and TPA significantly increased the expression level of p-ERK2; and the lower right panel showed that the AST single-agent group induced cell apoptosis, and its combination therapy group with TPA had a more significant effect on cell apoptosis.

The experimental results showed that treatment with two ERK inhibitors, PD98059 and Suramin alone, slightly reduced the level of endogenous ERK phosphorylation, while treatment with AST alone significantly increased the level of intracellular p-ERK2. Meanwhile, the combination of AST with the two ERK inhibitors almost completely offset the AST-induced increase of p-ERK2. The Caspase 3/7 kit was used to detect the effects of AST, ERK2 inhibitors alone and in combination on cell apoptosis, and the results showed that ERK2 inhibitors significantly inhibited AST-induced cell apoptosis. In contrast, treatment with the ERK2 activator TPA alone could also promote the level of intracellular p-ERK2, and combined treatment with TPA and AST further enhanced the level of AST-induced ERK2 phosphorylation and cell apoptosis. Treatment of cells with any of the above single agents or combination therapy did not affect the expression of total ERK2. These results clearly showed that AST induced apoptosis of KRAS G12D mutation cells through the ERK2 signaling pathway.

Combining Examples 4, 5, and 6, the mechanism schematic diagram of FIG. 10 can be deduced, that is, AST induced apoptosis of cancer cells with KRAS G12D pathogenic mutation through the ERK2 signaling pathway. Further combined with the research of relevant literature: the MEK/ERK signaling pathway can be activated by the action of DNA alkylating agents (Wang X, Martindale JL and Holbrook NJ. Requirement for ERK activation in cisplatin-induced apoptosis. J Biol Chem 2000; 275: 39435-39443). Examples 11 and 12 confirmed that AST, a DNA alkylating agent prodrug, had the effect of activating the MEK/ERK signaling pathway, which can induce apoptosis of KRAS G12D mutation cancer cells through the ERK2 signaling pathway, while AST compound was an AKR1C3 enzyme-activated DNA alkylating agent prodrug, which will be activated by the AKR1C3 enzyme to produce AST-2660 to exert anti-cancer effects.

Considering the relevant regulatory pathways of AKR1C3 and ERK2 revealed in FIG. 10, the inventors speculate that AST compound can activate ERK2 to phosphorylate it, thereby activating the MEK-ERK pathway, upregulating Nrf2, and ultimately leading to high expression of AKR1C3 enzyme, which in turn induces cancer cell apoptosis. That is, KRAS-G12D mutation can more effectively activate the AST drug through upregulating the RAF/MEK/ERK signaling pathway, upregulating the protein expression of Nrf2, thereby upregulating the protein expression of AKR1C3.

In fact, studies have shown that KRAS gene mutations can upregulate the protein expression of Nrf2 and then upregulate the protein expression of AKR1C3. These genes include various subtypes under KRAS mutations: KRAS-G12D mutation, KRAS-G12V mutation, KRAS-G12C mutation, KRAS-G12A, and KRAS-G13D.

Further extending the mechanism of AST compound revealed in FIG. 10 to other similar AKR1C3 enzyme-activated prodrugs: general formulae (1)-(12), KRAS mutations can more effectively activate AKR1C3 enzyme-activated prodrugs to induce cell apoptosis or inhibit cell proliferation through upregulating the RAF/MEK/ERK signaling pathway, upregulating the protein expression of Nrf2, thereby upregulating the protein expression of AKR1C3.

Furthermore, we found in experiments that the AKR1C3 enzyme-activated DNA alkylating agents AST-3424 and AST have more excellent therapeutic effects on tumor models with BRCA mutation.

Based on the two approved HRD detection kits: FoundationFocus CDx BRCA LOH Assay and Myriad Genetics myChoice^{®} HRD, cell lines with BRCA gene mutation were selected for relevant experiments. In some examples, cell lines or models with BRCA pathogenic mutation were used for experiments. According to the judgment rules of the above kits, BRCA mutation positivity can be determined as HRD positivity.

### 7. In vitro cytotoxicity comparison of compound AST-3424 in tumor cells with BRCA non-pathogenic mutation and pathogenic mutation

The *in vitro* cytotoxicity (IC₅₀ value) of human-derived tumor cell lines was used to compare whether compound AST-3424 has significant differences in tumor cells with BRCA non-pathogenic mutation and pathogenic mutation, thereby detecting whether BRCA pathogenic mutation enhances the sensitivity of tumor cells to AST-3424 at the *in vitro* cell level. The following cell lines were used as research objects in the experiment: BRCA pathogenic mutation cell line Capan-1 (human pancreatic cancer cell line, BRCA2 pathogenic mutation site: p.S1982RfsTer22), BRCA non-pathogenic mutation cell line PLC/PRF/5 (human hepatocellular carcinoma cell line) and HT29 (human colorectal cancer cell line).

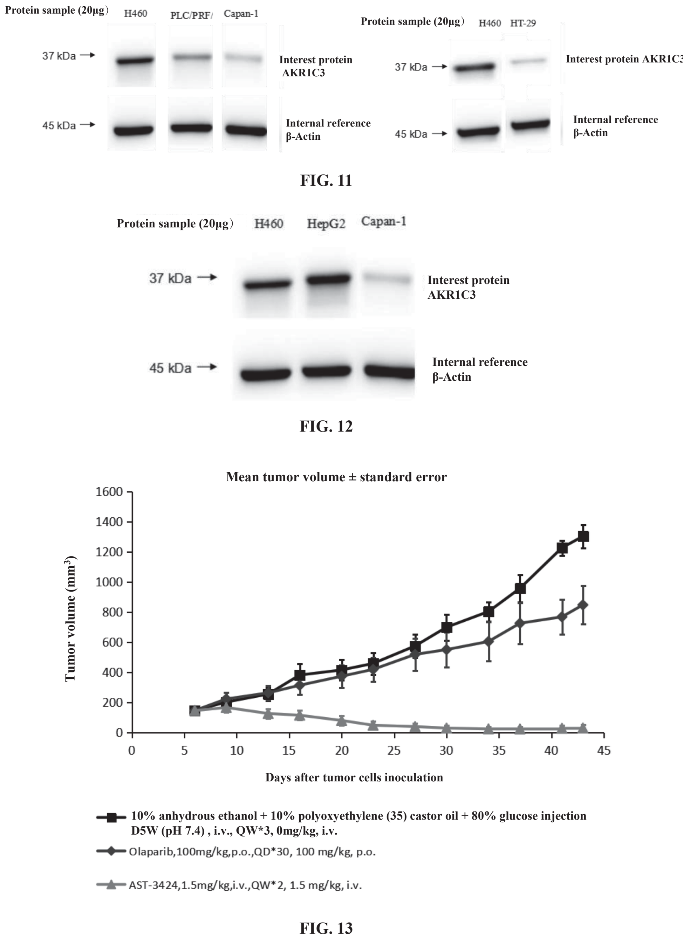

The activation mechanism of prodrug AST-3424 and its analog compound S (i.e., the aforementioned compound AST) is to release DNA alkylating agents through the action of AKR1C3 enzyme, thereby inhibiting and killing tumor cells. Therefore, in the presence or absence of pathogenic gene mutations, the higher the expression level of AKR1C3 enzyme, the stronger the expected cytotoxicity of the compound to tumor cells and the lower the IC₅₀ value. We chose to detect the effect of BRCA pathogenic mutation on the sensitivity of cells to AST-3424 and its analog compound S when the AKR1C3 protein expression levels of each cell line are close or the AKR1C3 enzyme protein expression level of BRCA pathogenic mutation cell line is relatively lower than that of non-BRCA pathogenic mutation cell line. We identified and quantitatively analyzed the AKR1C3 protein expression levels of the above three cell lines by Western blot assay. Meanwhile, the human lung cancer cell line H460 with high AKR1C3 protein expression was used as a control to compare the AKR1C3 protein expression levels of the three cell lines. Western Blot results showed that the AKR1C3 protein expression levels of Capan-1 and HT29 cell lines were close, and that of PLC/PRF/5 cell line was slightly higher (Table 4, Fig. 11).

**Table 4: AKR1C3 protein expression levels of each cell line compared with H460 cell line**

| Cell line | AKR1C3 protein expression in H460 cells% |
|---|---|
| Capan-1 | 19.45% |
| PLC/PRF/5 | 44.39% |
| HT29 | 13.76% |
| H460 | 100% |

Cell proliferation experiments were used to detect the sensitivity of various cells to AST-3424, i.e., IC₅₀ value. Unless otherwise specified, IC₅₀ values were tested by CTG assay.

The IC₅₀ values of the test compound AST-3424 in different cells measured by the above experimental method were listed in Table 5 below.

**Table 5: IC₅₀ data of compound AST-3424 for inhibiting three tumor cell lines**

| Compound | Cell line | IC₅₀ (nmol/L) | Fold difference |
|---|---|---|---|
| | Capan-1 | 8.33 | / |
| AST-3424 | PLC/PRF/5 | 186.60 | 22 |
| | HT29 | 559.00 | 67 |

Test results showed that compared with the BRCA pathogenic mutation cell line Capan-1, the IC₅₀ of AST-3424 in the BRCA non-pathogenic mutation cell line HT29 was 67 times that of Capan-1 cell line. Therefore, there was a significant difference in the IC₅₀ values of AST-3424 between the BRCA pathogenic mutation cell line Capan-1 and the BRCA non-pathogenic mutation cell line HT29 with similar AKR1C3 protein expression. This indicated that under the condition of similar AKR1C3 protein expression, tumor cells with BRCA pathogenic mutation were more sensitive to AST-3424. Even though the AKR1C3 protein expression level of BRCA pathogenic mutation cell line Capan-1 was relatively lower than that of BRCA non-pathogenic mutation cell line PLC/PRF/5, the IC₅₀ of AST-3424 in BRCA non-pathogenic mutation cell line PLC/PRF/5 was still 22 times that of Capan-1 cell line. Therefore, tumor cells with BRCA pathogenic mutation were more sensitive to AST-3424, i.e., HRD-positive tumor cells were more sensitive to AST-3424.

### 8. In vitro cytotoxicity comparison of AST-3424 analog compound S in tumor cells with BRCA non-pathogenic mutation and pathogenic mutation

Using the same experimental methods and cell lines as above, it was further tested whether compound S has significant differences in tumor cells with BRCA non-pathogenic mutation and pathogenic mutation, thereby detecting whether BRCA pathogenic mutation enhances the sensitivity of tumor cells to compound S. The *in vitro* cytotoxicity results of compound S on the above three cell lines were shown in Table 6 below.

**Table 6: IC₅₀ data of compound S for inhibiting three tumor cell lines**

| Compound | Cell line | IC₅₀ (nmol/L) | Fold difference |
|---|---|---|---|
| **AST-3424 analog compound S** | Capan-1 | 403.40 | / |
| | PLC/PRF/5 | 2015.00 | 5 |
| | HT29 | 4883.00 | 12 |

Test results showed that compared with the BRCA pathogenic mutation cell line Capan-1, the IC₅₀ of AST-3424 analog compound S in the BRCA non-pathogenic mutation cell line HT29 was 12 times that of Capan-1 cell line. Therefore, there was also a significant difference in the IC₅₀ values of AST-3424 analog compound S between the BRCA pathogenic mutation cell line Capan-1 and the BRCA non-pathogenic mutation cell line HT29 with similar AKR1C3 protein expression. Even though the AKR1C3 protein expression level of BRCA pathogenic mutation cell line Capan-1 was relatively lower than that of BRCA non-pathogenic mutation cell line PLC/PRF/5, the IC₅₀ of AST-3424 analog compound S in BRCA non-pathogenic mutation cell line PLC/PRF/5 was still 5 times that of Capan-1 cell line. Therefore, tumor cells with BRCA pathogenic mutation models were more sensitive to AST-3424 analog compound S.

The results of the above two experiments both indicated that under the condition of similar AKR1C3 protein expression levels, AST-3424 and its analog compound S had more obvious *in vitro* killing effects on tumors with BRCA pathogenic mutation models. Even if the AKR1C3 protein expression level of BRCA pathogenic mutation cell lines was relatively lower than that of BRCA non-pathogenic mutation cell lines, AST-3424 and AST-3424 analog compound S still had more obvious *in vitro* killing effects on tumors with BRCA pathogenic mutation models.

### 9. In vivo efficacy comparison of compound AST-3424 in BRCA non-pathogenic mutation and pathogenic mutation tumor models

To verify whether there is a difference in the *in vivo* efficacy of AST-3424 between BRCA pathogenic mutation and non-pathogenic mutation tumor cells, we selected two CDX models: human pancreatic cancer Capan-1 (BRCA2 pathogenic mutation model, mutation site: p.S1982RfsTer22) and BRCA non-pathogenic mutation model HepG2 (human hepatocellular carcinoma model). The detected mRNA expression levels of AKR1C3 in Capan-1 and HepG2 cell lines were LOG2(FPKM)=6.6363 and LOG2(FPKM)=6.8572, respectively, indicating that the mRNA expression levels of AKR1C3 in the two models were close. The comparison of AKR1C3 protein expression levels with that of H460 cell line was shown in Table 7 and FIG. 12. Protein expression results showed that the AKR1C3 protein expression level of Capan-1 cells with BRCA pathogenic mutation was lower than that of HepG2 cells with BRCA non-pathogenic mutation. On this basis, we compared the effect of BRCA pathogenic mutation on the *in vivo* efficacy of AST-3424.

**Table 7: AKR1C3 protein expression levels of each cell line compared with that of H460 cell line**

| Cell line | AKR1C3 protein expression in H460 cells% |
|---|---|
| Capan-1 | 19.45% |
| HepG2 | 117.36% |
| H460 | 100% |

### 10. In vivo efficacy experiment of AST-3424 in Capan-1 CDX model with BRCA pathogenic mutation

BALB/c nude mice were subcutaneously inoculated with human pancreatic cancer Capan-1 cells to establish a human pancreatic cancer subcutaneous xenograft model. The experiment was divided into three groups: test drug Olaparib 100 mg/kg single-agent group, AST-3424 1.5 mg/kg single-agent group, and 10% anhydrous ethanol + 10% polyoxyethylene (35) castor oil + 80% glucose injection D5W (pH 7.4) vehicle control group, with 6 mice in each group. The test drug Olaparib single-agent group was administered by oral gavage once a day for 30 consecutive days. Both the vehicle control group and the AST-3424 single-agent group were administered by tail vein injection, wherein the vehicle control group was administered once a week for three weeks; the AST-3424 single-agent group was administered once a week for two weeks. The specific administration regimen and TGI were shown in Table 8. The test drug Olaparib 100 mg/kg treatment group showed a slight tumor inhibitory effect on Day 43 after tumor cell inoculation, with a relative tumor growth inhibition rate TGI(%) of 37.43%, which was not statistically significant compared with the control group (p>0.05). The test drug AST-3424 1.5 mg/kg treatment group showed an extremely significant tumor inhibitory effect on Day 43 after tumor cell inoculation, with a relative tumor growth inhibition rate TGI(%) of 97.97%. Tumors in four mice were completely cleared, with a complete tumor inhibition rate of 66.7%, which was statistically significant compared with the control group (p<0.001).

**Table 8: Efficacy analysis of each group in the human pancreatic cancer Capan-1 subcutaneous model**

| **Experimental group** | **Day 43 after cell inoculation** | | | | | |
|---|---|---|---|---|---|---|
| | **Tumor volume mm³ (*x̅*±S)** | **Relative tumor volume mm³ (*x̅* ±S)** | **TGI (%)** | **T/C (%)** | **P Value (compared with the control group)** | **CR rate (complete clearance)** |
| Vehicle control group | 1301.38±76.61 | 9.15±0.70 | - | - | - | 0/6 |
| Olaparib, 100 mg/kg | 846.86±128.85 | 5.72±0.54 | 37.43 | 62.57 | >0.05 | 0/6 |
| AST-3424, 1.5 mg/kg | 30.74±21.47 | 0.19±0.12 | 97.97 | 2.03 | <0.001 | 4/6 |

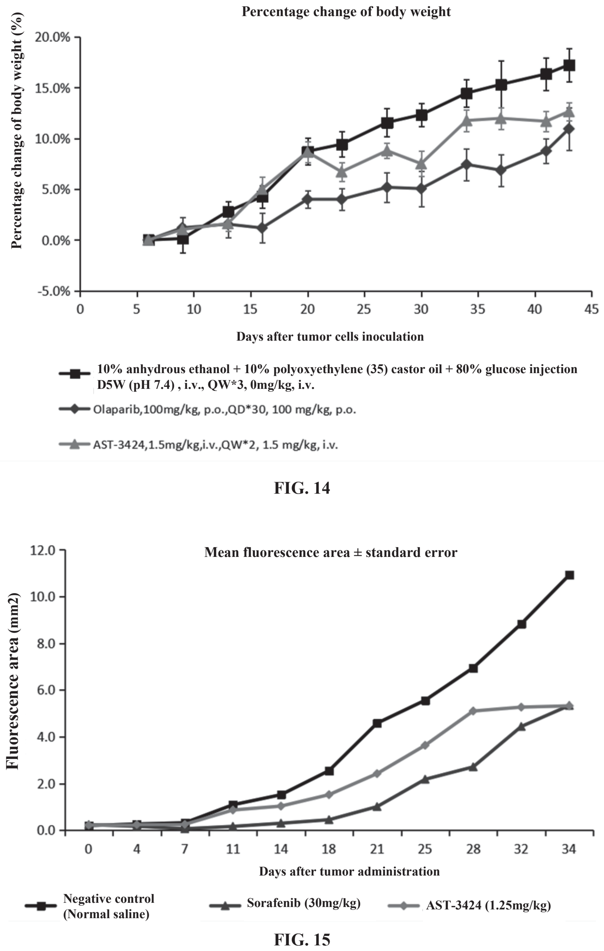

The corresponding curves of tumor volume and mouse body weight percentage over time were shown in FIG. 13 and FIG. 14.

Experimental results showed that in the Capan-1 CDX model with BRCA pathogenic mutation, the AST-3424 1.5 mg/kg single-agent group showed a significant tumor inhibitory effect, with a TGI of 97.97% at the end of the experiment, and tumors in 4 mice in this experimental group were completely cleared. The body weight percentage data of the above animal experiment showed that the body weight of experimental animals in the AST-3424 administration group did not decrease, proving that the drug was well-tolerated.

### 11. In vivo efficacy experiment of AST-3424 in non-BRCA pathogenic mutation model HepG2-GFP

BALB/c nude mice were orthotopically inoculated with human hepatocellular carcinoma HepG2-GFP cells in the liver tissue to establish a human hepatocellular carcinoma orthotopic xenograft model, and the fluorescence area of green fluorescent protein (GFP) was used to define the tumor size. The experiment was divided into three groups: test drug Sorafenib single-agent group, AST-3424 1.25 mg/kg single-agent group, and normal saline injection vehicle control group, with 10 mice in each group. The test drug Sorafenib single-agent group was administered by oral gavage once a day for 35 consecutive days. Both the vehicle control group and the AST-3424 single-agent group were administered by tail vein injection, wherein both were administered once a week for two weeks, discontinued for one week, and then administered once a week for another two consecutive weeks. The specific administration regimen and TGI were shown in Table 9.

**Table 9: Efficacy analysis of each group in human hepatocellular carcinoma HepG2-GFP orthotopic tumor model**

| **Experimental group** | **Day 35 after administration** | | | | |
|---|---|---|---|---|---|
| | **Average tumor fluorescence area mm²** (*x̅*±S) | **Average tumor weight (g)** (*x̅*±S) | **TGI (%)** | **P Value (compared with the control group)** | **CR rate (complete clearance)** |
| Normal saline | 10.9±6.0 | 0.0879±0.0590 | - | - | |
| Sorafenib 30 mg/kg | 5.3±5.3 | 0.0432±0.0373 | 51 | <0.05 | 0/10 |
| AST-3424, 1.25 mg/kg | 5.3±3.1 | 0.0350±0.0318 | 60 | <0.05 | 0/10 |

| | | | | | |
|---|---|---|---|---|---|
| Note: TGI was calculated by the following formula (based on tumor weight): TGI% = (1 - treatment(T)/control(C)) × 100% (T and C are the tumor weights of the treatment group and control group at the end of the experiment, respectively). | | | | | |

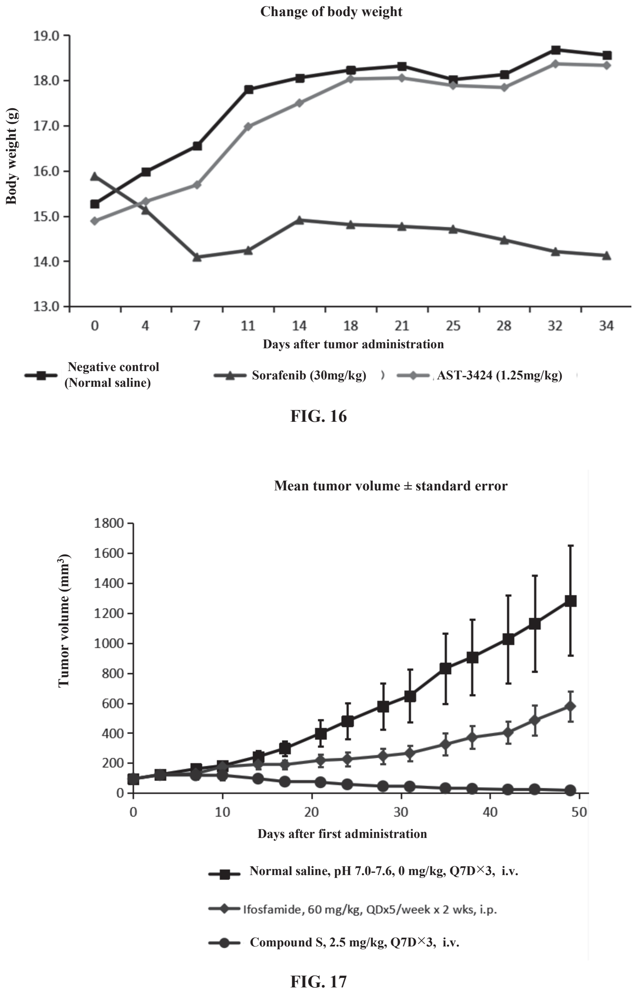

The corresponding curves of tumor volume and mouse body weight over time were shown in FIG. 15 and FIG. 16.

Experimental results showed that in the BRCA non-pathogenic mutation model, the tumor fluorescence area reading of the AST-3424 1.25 mg/kg group was significantly smaller than that of the vehicle control group, but there was no statistically significant difference compared with the vehicle control group (p>0.05), with a TGI of 60%. This suggested that AST-3424 1.25 mg/kg had a trend of growth inhibition on the human hepatocellular carcinoma HepG2-GFP orthotopic model, but no statistically significant inhibitory effect, and no tumors in mice were completely cleared. The body weight data of the above animal experiment showed that the body weight of experimental animals in the AST-3424 administration group did not decrease, proving that the drug was well-tolerated.

### Experimental summary

From the above experimental Examples 9-11, we found that there was a significant difference in the *in vivo* efficacy of AST-3424. AST-3424 1.5 mg/kg (administered once a week for two weeks) had a very significant *in vivo* efficacy on the Capan-1 CDX model with BRCA pathogenic mutation, with a TGI of 97.97%, and tumors in 4 out of 6 mice in the AST-3424 administration group were completely cleared, with a clearance rate of 67%. However, AST-3424 1.25 mg/kg (administered once a week for two weeks, discontinued for one week, then administered once a week for another two consecutive weeks) had no significant *in vivo* efficacy on the BRCA non-pathogenic mutation model HepG2-GFP when the total administration dose exceeded that of the Capan-1 model, with a TGI of 60%, no significant difference compared with the control group, and no tumors in mice in the administration group were completely cleared. The above results showed that although the AKR1C3 protein expression level of the Capan-1 model was lower than that of the HepG2-GFP model, the *in vivo* efficacy of AST-3424 was significantly different because the Capan-1 model had BRCA pathogenic mutation while the HepG2-GFP model had no BRCA pathogenic mutation. BRCA pathogenic mutation enhanced the sensitivity of the Capan-1 model to AST-3424 and promoted the killing of tumor tissue by AST-3424.

### 12. In vivo efficacy comparison of compound S in BRCA non-pathogenic mutation and pathogenic mutation tumor models

To verify the difference in *in vivo* efficacy of **AST-3424 analog compound S** between BRCA pathogenic mutation and non-pathogenic mutation models, we selected two PDX models: human gastric cancer PDX model GA6201 (BRCA2 pathogenic mutation model, mutation site: L1732PfsTer9) and BRCA non-pathogenic mutation PDX model LU5173 (human lung cancer PDX model). According to the results detected by existing methods, the mRNA expression levels of AKR1C3 in GA6201 and LU5173 tumor tissues were LOG2(FPKM)=6.78 and LOG2(FPKM)=8.88, respectively, indicating that the RNA expression levels of AKR1C3 in the two models were close. The AKR1C3 protein expression levels of these two models were detected by immunohistochemistry (IHC), and the results were shown in Table 10. Immunohistochemistry and database suggested that the AKR1C3 protein expression levels of these two PDX models were close. This part of the study used these two models with similar AKR1C3 protein expression levels to compare the effect of BRCA pathogenic mutation on the *in vivo* efficacy of **AST-3424 analog compound S.**

**Table 10: Comparison of AKR1C3 protein and RNA expression in two PDX subcutaneous models**

| **Model** | **IHC of AKR1C3 protein (percentage of different staining intensities)** | | | | **AKR1C3 RNA LOG2(FPKM)** |
|---|---|---|---|---|---|
| | **None** | **Weak** | **Moderate** | **Strong** | |
| GA6201 | 1.07 | 8.75 | 30.54 | 59.63 | 6.78 |
| LU5173 | 1.97 | 7.83 | 20.77 | 69.43 | 8.88 |

| | | | | | |
|---|---|---|---|---|---|
| Note: The scoring standard for IHC staining of AKR1C3 protein expression refers to the literature (Guise et al., Cancer Res, 2010 (70): 1573), wherein "weak" corresponds to "Focal" in the literature, "moderate" corresponds to "Moderate", and "strong" corresponds to "Diffuse". | | | | | |

### 13. In vivo efficacy experiment of compound S in BRCA pathogenic mutation model HuPrime^{®} gastric cancer GA6201

BALB/c nude mice were subcutaneously inoculated with HuPrime^{®} model GA6201 tumor blocks to establish a human gastric cancer subcutaneous xenograft tumor model. The experiment was divided into three groups: test drug Ifosfamide 60 mg/kg group, test drug compound S 2.5 mg/kg group, and normal saline (pH 7.0-7.6) vehicle control group, with 5 mice in each group. Among them, the normal saline (pH 7.0-7.6) vehicle control group and the test drug **compound S** 2.5 mg/kg group were administered by tail vein injection once a week for three weeks, and observed for four weeks. The Ifosfamide 60 mg/kg group was administered by intraperitoneal injection once a day for five consecutive days, discontinued for two days, administration for a total of two weeks, and observed for five weeks. The specific administration regimen and TGI are shown in Table 11.

**Table 11: Efficacy analysis of each group in HuPrime^{®} gastric cancer GA6201 model**

| **Experimental group** | **Day 3 after the end of all administration (i.e., Day 38)** | | | | |
|---|---|---|---|---|---|
| | **Tumor volume mm³** (*x̅*±S) | **Relative tumor volume mm³** (*x* ±S) | **TGI (%)** | **T/C (%)** | **P Value (compared with the mtrol group)** |
| Normal saline, pH 7.0-7.6 | 905.75±252.4 | 9.34±2.42 | - | - | - |
| Ifosfamide 60 mg/kg | 371.02±75.96 | 3.85±0.63 | 58.83 | 41.17 | 0.857 |
| **Compound S** 2.5 mg/kg | 28.69±8.2 | 0.29±0.08 | 96.85 | 3.15 | 0.000135 |

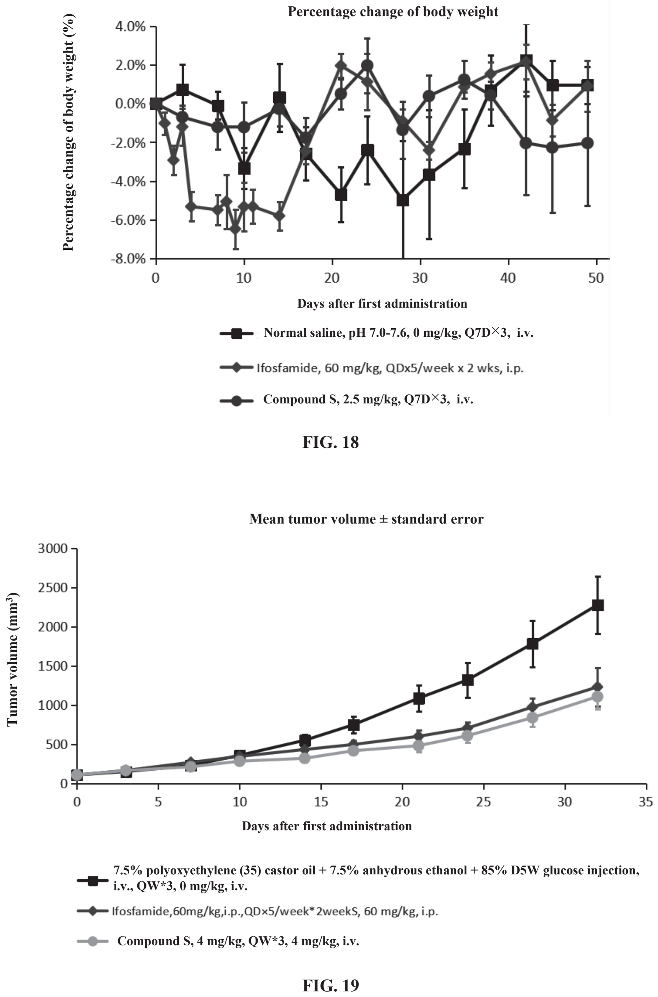

The corresponding curves of tumor volume and mouse body weight percentage over time were shown in FIG. 17 and FIG. 18.

Experimental results showed that in the HuPrime^{®} gastric cancer GA6201 model with BRCA pathogenic mutation, the test drug Ifosfamide (60 mg/kg) treatment group showed a certain tumor inhibitory effect on Day 3 after the end of all administration cycles (Day38), with no statistically significant difference compared with the control group (p=0.857) and a relative tumor growth inhibition rate TGI(%) of 58.83%. The test drug compound S (2.5 mg/kg) treatment group showed a significant tumor inhibitory effect on Day 3 after the end of all administration cycles (Day38), with statistically significant differences compared with the control group (p=0.000135) and a relative tumor growth inhibition rate TGI(%) of 96.85%. The body weight of mice in the test drug compound S (2.5 mg/kg) treatment group did not decrease, showing no obvious drug toxicity and good tolerance during treatment.

### 14. In vivo efficacy experiment of compound S in non-BRCA pathogenic mutation model HuPrime^{®} lung cancer LU5173

NOD/SCID female mice were subcutaneously inoculated with HuPrime^{®} model LU5173 tumor blocks to establish a human lung cancer subcutaneous xenograft model. The experiment was divided into three groups: positive control drug Ifosfamide 60 mg/kg group, test drug **compound S** 4 mg/kg group, and 7.5% anhydrous ethanol + 7.5% polyoxyethylene (35) castor oil + 85% glucose injection D5W vehicle control group, with 6 mice in each group. Among them, the 7.5% anhydrous ethanol + 7.5% polyoxyethylene (35) castor oil + 85% glucose injection D5W vehicle control group and the test drug **compound S** group were administered by tail vein injection once a week for three consecutive weeks. The positive control drug Ifosfamide 60 mg/kg group was administered by intraperitoneal injection once a day for five consecutive days, discontinued for two days, administration for two consecutive weeks. The specific administration regimen and TGI are shown in Table 12.

**Table 12: Efficacy analysis of each group in HuPrime^{®} lung cancer LU5173 model**

| **Experimental group** | **Day 32 after the first administration (i.e., 32 days post the first administration)** | | | | |
|---|---|---|---|---|---|
| | **Tumor volume** mm³ (*x̅*±S) | **Relative tumor volume** mm³ (*x̅*±S) | **TGI (%)** | **T/C (%)** | **P Value (compared with the control group)** |
| 7.5% anhydrous ethanol + 7.5% polyoxyethylene (35) castor oil + 85% glucose injection D5W, i.v. | 2276.68±367.07 | 21.55±3.26 | - | - | - |
| Ifosfamide, 60 mg/kg, i.p. | 1229.15±246.65 | 11.97±2.43 | 44.45% | 55.55% | >0.05 |
| **Compound S,** 4 mg/kg, i.v. | 1107.01±162.08 | 10.22±1.11 | 52.58% | 47.42% | <0.05 |

The corresponding curves of tumor volume and mouse body weight percentage over time were shown in FIG. 19 and FIG. 20.

Experimental results showed that in the non-BRCA pathogenic mutation HuPrime^{®} lung cancer LU5173 model, the test drug Ifosfamide (60 mg/kg) treatment group showed a certain tumor inhibitory effect on Day 32 after the first administration, with a relative tumor growth inhibition rate TGI(%) of 44.45%, which was not statistically significant compared with the control group (p>0.05). The test drug compound S 4 mg/kg treatment group showed an significant tumor inhibitory effect on Day 32 after the first administration, with a relative tumor growth inhibition rate TGI(%) of 52.58%, which was statistically significant compared with the control group (p<0.05). Mice in the test drug **compound S** (4 mg/kg) treatment group showed no obvious body weight loss and were well-tolerated during treatment.

### Experimental summary

From the above experimental Examples 12-14, we found that there was a significant difference in the *in vivo* efficacy of **compound S** between the two models with similar AKR1C3 expression levels. In the BRCA pathogenic mutation HuPrime^{®} gastric cancer GA6201 model, the test drug **compound S** 2.5 mg/kg (administered once a week for three weeks) treatment group showed a significant tumor inhibitory effect, with statistically significant differences compared with the control group (p=0.000135) and a relative tumor growth inhibition rate TGI(%) of 96.85%. In the non-BRCA pathogenic mutation HuPrime^{®} lung cancer LU5173 model, the higher dose of test drug compound S 4 mg/kg treatment group showed an significant tumor inhibitory effect on Day 32 after the first administration, with a relative tumor growth inhibition rate TGI(%) of 52.58%, which was significantly lower than that in the HuPrime^{®} gastric cancer GA6201 model. The above results indicated that, **compound S** in the non-BRCA pathogenic mutation model, even at a higher dose of 4 mg/kg is used, the tumor inhibitory effect of **compound S** was lower than that of the low-dose 2.5 mg/kg administration group in the BRCA pathogenic mutation model. This also proved that BRCA pathogenic mutation enhanced the sensitivity of the HuPrime^{®} gastric cancer GA6201 model to **compound S** and improved the killing effect of compound S on tumor tissue.

### 15. Clinical detection of KRAS mutation and AKR1C3 enzyme protein expression level conducted in China

In a clinical trial approved by Chinese regulatory authority (CTR20220934), **clinical detection of KRAS mutation and AKR1C3 enzyme protein expression level** was performed on enrolled Chinese clinical trial patients.

The trial was approved by the Ethics Committee of the medical institutions participating in the clinical trial, conducted in accordance with the principles of the Declaration of Helsinki, and with the informed consent of all subjects.

The detection and results of AKR1C3 enzyme protein expression level were carried out in accordance with the method described in WO2022048492, and the H-score system was used for scoring to characterize the expression level, with H-score ≥135 defined as high expression.

Among 10 KRAS-positive patients, 7 had high AKR1C3 expression. The specific detection results were shown in Table 13 below.

**Table 13: Clinical detection data of KRAS mutation and AKR1C3 enzyme protein expression level**

| Case screening number/enrollment number | Percentage of 2+&3+ and H-score in AKR1C3 IHC detection | KRAS gene mutation or deficiency detection: positive/negative | Primary tumor |
|---|---|---|---|
| 01003/10202 | 25%/75 | Positive+, G12D | Rectal cancer |
| 01005/10301 | 2%/9 | Positive+, G12D | Rectal cancer |
| 03002/10303 | 65%/195 | Positive+, G12D | Pancreatic cancer |
| 01007/10402 | 80%/225 | Positive+, G12D | Appendiceal adenocarcinoma |
| 03004/10503 | 80%/230 | Positive+, G12D | Intrahepatic cholangiocarcinoma |
| 01009/10501 | 80%/245 | Positive+, G12D | Rectal cancer |
| 03005/10602 | 0/90 | Positive+, G12R | Ampullary carcinoma |
| 03001/10302 | 65%/210 | Positive+, G12D | Pancreatic cancer |
| 06002/10401 | 75%/225 | Positive+, G12D | Cervical cancer |
| 02002/10601 | 100%/300 | Positive+, G12V | Pancreatic cancer |

Similar to AST-3424 and compound S in toxin structure, the compounds of general formulas (1), (3)-(8), and (12) are all AKR1C3-activated anticancer prodrugs, which are lysed to release DNA alkylating agents with similar toxin structures after being activated by AKR1C3 enzyme. Therefore, combined with the experimental results of AST-3424 and compound S above, it can be deduced that the AKR1C3-activated DNA alkylating agent prodrugs with similar toxin structures (general formulas (1), (3)-(8), (12)) have more significant therapeutic effects on the treatment of cancer and tumor patients with homology-dependent recombination repair deficiency (HRD). Thus, it is feasible to further select a patient with high AKR1C3 expression (accompanied by KRAS gene mutation) for treatment using homology-dependent recombination repair deficiency (HRD) positivity.

Furthermore, the toxins of the above AKR1C3-activated DNA alkylating agents are all alkylating agents that act on DNA double strands to cause cross-linking. The above compounds should have more excellent therapeutic effects on tumor models with mutations in homologous DNA double-strand break repair genes (HRRm) compared with non-mutated tumor models. That is, the AKR1C3-activated DNA alkylating agent prodrugs with similar toxin structures have more significant therapeutic effects on the treatment of cancer and tumor patients with mutations in homologous DNA double-strand break repair genes when used alone or in combination with other therapeutic drugs.

## Claims

1. An administration method for cancer, tumor, or disorders or cell proliferative diseases caused by cancer or tumor, including administering an AKR1C3 enzyme-activated prodrug to a patient when the tumor or cancer tissue of the patient is detected to have a KRAS gene mutation or the patient is detected to have a KRAS gene mutation, without detecting the AKR1C3 protein expression level or the AKR1C3 RNA content in the tumor or cancer tissue of the patient.

2. A method for treating cancer, tumor, or disorders or cell proliferative diseases caused by cancer or tumor, including a step of administering a drug or formulation containing an AKR1C3 enzyme-activated prodrug and a step of detecting KRAS gene mutation, and not including a step of detecting the AKR1C3 protein expression level or the AKR1C3 RNA content in the tumor or cancer tissue of the patient, and administering the drug or formulation containing AKR1C3 enzyme-activated prodrug to the patient when the tumor or cancer tissue of the patient is detected to have a KRAS gene mutation or the patient is detected to have a KRAS gene mutation.

3. Pharmaceutical use of AKR1C3 enzyme-activated prodrug in the manufacture of a drug for treating cancer, tumor, or disorders or cell proliferative diseases caused by cancer or tumor, **characterized in that**: the drug is formulated for administration to the patient whose tumor or cancer tissue is detected to have a KRAS gene mutation or the patient who is detected to have a KRAS gene mutation; and the AKR1C3 protein expression level detection or the AKR1C3 RNA content detection in the tumor or cancer tissue of the patient is not required.

4. The method or use according to any one of claims 1, 2, 3, wherein the KRAS gene mutation is selected from KRAS-G12D mutation, KRAS-G12V mutation, KRAS-G12C mutation, KRAS-G12A and KRAS-G13D.

5. The method or use according to any one of claims 1, 2, 3, wherein the AKR1C3 enzyme-activated prodrug is selected from the compound of formulae (1) - (12) or pharmaceutically acceptable salt, solvate, isotope isomer thereof: 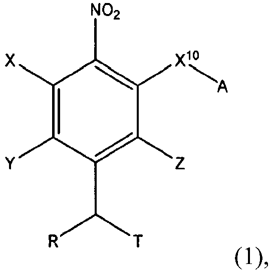
wherein the definitions of X, Y, Z, R, T, A and X¹⁰ are described in the claims of Patent Application PCT/US2016/021581 with Publication No. WO2016145092A1 (corresponding to the Chinese Application No. 2016800150788 with Publication No. CN107530556A); 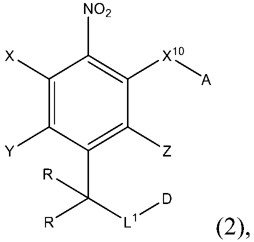
wherein the definitions of X, Y, Z, R, D, L¹, A and X¹⁰ are described in the claims of Patent Application PCT/US2016/025665 with Publication No. WO2016/161342A1 (corresponding to the Chinese Application No. 2016800200132 with Publication No. CN108136214A); 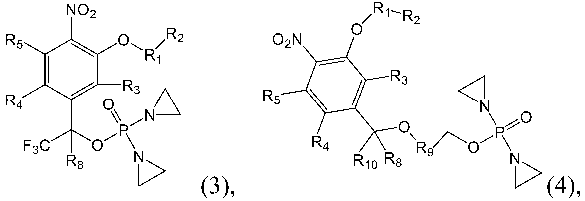
wherein the definitions of R₁, R₂, R₃, R₄, R₅, R₈, R₉, and R₁₀ are described in the claims of Patent Application PCT/CN2020/089692 with Publication No. WO2020228685A1 (corresponding to the Chinese Application No. 2020800358890 with Publication No. CN113853379A); 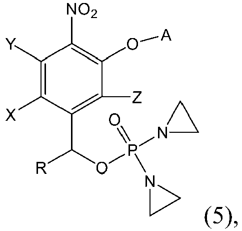
wherein:
A is a substituted or unsubstituted C₆-C₁₀ aryl, a biaryl or substituted biaryl, a 5-15-membered heteroaryl, or -N=CR¹R²; wherein the substituent is selected from the group consisting of halogeno, -CN, -NO₂, -O-(CH₂)-O-, -CO₂H and salt thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7-membered heterocycle;
wherein the alkyl group and the aryl group are each substituted by 1-3 halogeno groups or 1-3 C₁-C₆ alkyl groups;
R¹ and R² are each independently phenyl or methyl;
X, Y and Z are each independently hydrogen or halogeno; and
R is hydrogen or C₁-C₆ alkyl or halogen-substituted alkyl; 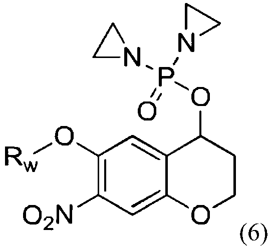
wherein the definition of Rw is described in the claims of Patent Application PCT/CN2020/120281 with Publication No. WO2021068952A1 (corresponding to the Chinese Application No. 202080071652.8 with Publication No. CN114555574A); 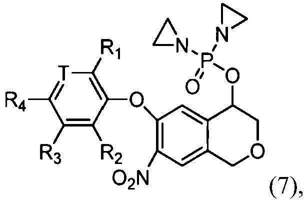
wherein the definitions of R₁, R₂, R₃, R₄ and T are described in the claims of Patent Application PCT/CN2021/118597 with Publication No. WO2022057838A1; 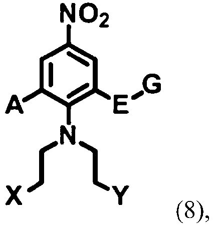
wherein the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A); 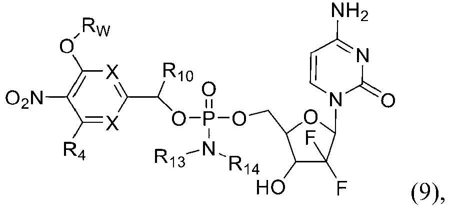 or pharmaceutically acceptable salt thereof,
wherein the definitions of R_{w}, X, R₄, R₁₀, R₁₃ and R₁₄ are described in the claims of Patent Application PCT/CN2022/098082 with Publication No. WO2022258043A1; 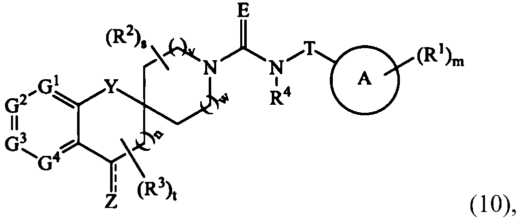
wherein the definitions of R₁, R₂, R₃, R₄, G₁, G₂, G₃, G₄, E, T, Y, Z, m, n, s, t, v, w and ring A are described in the claims of Patent Application CN202210585771.6 with Publication No. CN115403579A; 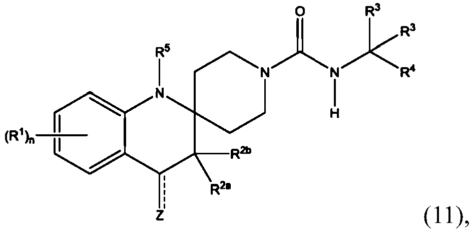 or pharmaceutically acceptable salt thereof,
wherein the definitions of R¹, R^{2a}, R^{2b}, R³, R⁴, R⁵, n and Z are described in the claims of Patent Application PCT/IB2020/057285 with Publication No. WO2021005586A1 (corresponding to Chinese Patent Application No. CN202080053804.1 with Publication No. CN114206870A); 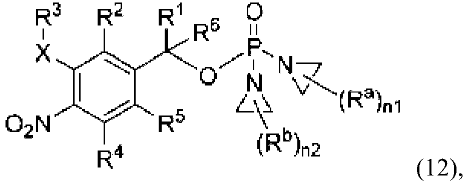 or pharmaceutically acceptable salt thereof,
wherein the definitions of R¹, R², R³, R⁴, R⁵, R⁶, R^{a}, R^{b}, n1 and n2 are described in the claims of Patent Application PCT/CN2023/123253 with Publication No. WO2024078392A1.

6. The method or use according to claim 5, when the AKR1C3 enzyme-activated prodrug is selected from the compound of formulae (1), (3) - (8), (12) or pharmaceutically acceptable salt, solvate, isotope isomer thereof,
the tumor or cancer tissue of the patient is detected to have homology-dependent recombination repair deficiency (HDR), or the patient is detected to have homology-dependent recombination repair deficiency (HDR).

7. The method or use according to claim 6, wherein having homology-dependent recombination repair deficiency must be met any one or both of the following conditions:
mutation occurs in one or more genes related to homology-dependent recombination repair (HRR) process;
genomic scar (GS) score reaches a predetermined value.

8. The method or use according to claim 7, wherein the gene related to homology-dependent recombination repair process is selected from ATM, ATR, BARD1, BLM, BRCA1, BRCA2, BRIP1, CDK12, CHEK1, CHEK2, FAAP20, FAN1, FANCE, FANCL, FANCM, MRE11A, NBN, PALB2, POLQ, PPP2R2A, RAD51B, RAD51C, RAD51D, RAD54L, RBBP8, SLX4, and XRCC2,
the genomic scar is selected from loss of heterozygosity (LOH), telomeric allelic imbalance (TAI) and large-scale state transition (LST).

9. A formulation unit package, comprising an independent package container for holding the drug formulation, an outer package component for accommodating the independent package container, and drug instructions, **characterized in that**: the drug formulation comprises an AKR1C3 enzyme-activated prodrug, and the drug instructions record:
the drug is used for treating a patient with cancer, tumor, or disorders or cell proliferative diseases caused by cancer or tumor;
the patient was conducted for KRAS gene mutation detection, without conducting the detection of the AKR1C3 protein expression level or the AKR1C3 RNA content in the tumor or cancer tissue;
when the tumor or cancer tissue of the patient is detected to have a KRAS gene mutation or the patient is detected to have a KRAS gene mutation, the drug or formulation containing AKR1C3 enzyme-activated prodrug is administered to the patient.

10. The formulation unit package according to claim 9, wherein
the KRAS gene mutation is selected from KRAS-G12D mutation, KRAS-G12V mutation, KRAS-G12C mutation, KRAS-G12A and KRAS-G13D;
the AKR1C3 enzyme-activated prodrug is selected from the compound of formulae (1) - (12) or pharmaceutically acceptable salt, solvate, isotope isomer thereof: 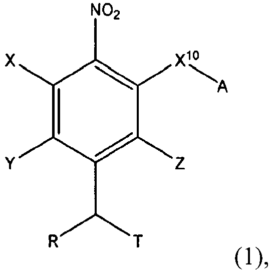
wherein the definitions of X, Y, Z, R, T, A and X¹⁰ are described in the claims of Patent Application PCT/US2016/021581 with Publication No. WO2016145092A1 (corresponding to the Chinese Application No. 2016800150788 with Publication No. CN107530556A); 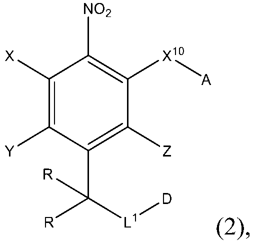
wherein the definitions of X, Y, Z, R, D, L¹, A and X¹⁰ are described in the claims of Patent Application PCT/US2016/025665 with Publication No. WO2016/161342A1 (corresponding to the Chinese Application No. 2016800200132 with Publication No. CN108136214A); 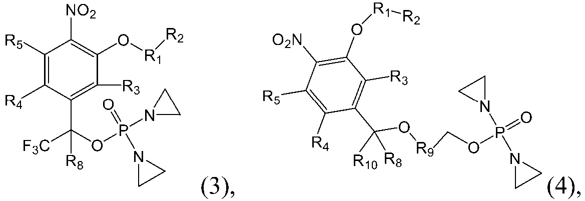
wherein the definitions of R₁, R₂, R₃, R₄, R₅, R₈, R₉, and R₁₀ are described in the claims of Patent Application PCT/CN2020/089692 with Publication No. WO2020228685A1 (corresponding to the Chinese Application No. 2020800358890 with Publication No. CN113853379A); 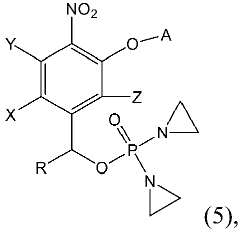
wherein:
A is a substituted or unsubstituted C₆-C₁₀ aryl, a biaryl or substituted biaryl, a 5-15-membered heteroaryl, or -N=CR¹R²; wherein the substituent is selected from the group consisting of halogeno, -CN, -NO₂, -O-(CH₂)-O-, -CO₂H and salt thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7-membered heterocycle;
wherein the alkyl group and the aryl group are each substituted by 1-3 halogeno groups or 1-3 C₁-C₆ alkyl groups;
R¹ and R² are each independently phenyl or methyl;
X, Y and Z are each independently hydrogen or halogeno; and
R is hydrogen or C₁-C₆ alkyl or halogen-substituted alkyl; 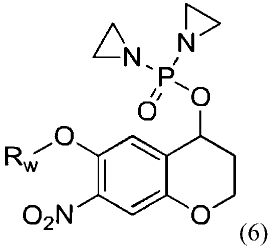
wherein the definition of Rw is described in the claims of Patent Application PCT/CN2020/120281 with Publication No. WO2021068952A1 (corresponding to the Chinese Application No. 202080071652.8 with Publication No. CN114555574A); 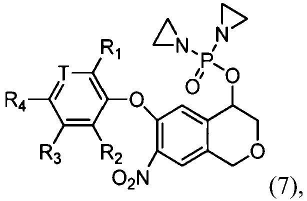
wherein the definitions of R₁, R₂, R₃, R₄ and T are described in the claims of Patent Application PCT/CN2021/118597 with Publication No. WO2022057838A1; 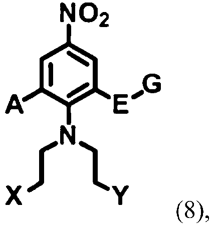
wherein the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A); 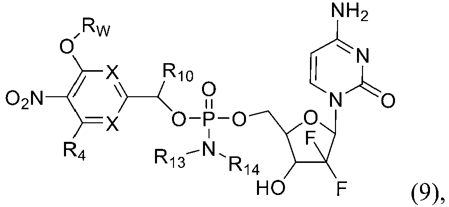 or pharmaceutically acceptable salt thereof,
wherein the definitions of R_{w}, X, R₄, R₁₀, R₁₃ and R₁₄ are described in the claims of Patent Application PCT/CN2022/098082 with Publication No. WO2022258043A1;
the AKR1C3 enzyme-activated prodrug is selected from the compound of formulae (10) - (11) or pharmaceutically acceptable salt, solvate, isotope isomer thereof: 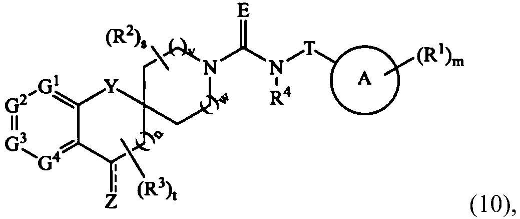
wherein the definitions of R₁, R₂, R₃, R₄, G₁, G₂, G₃, G₄, E, T, Y, Z, m, n, s, t, v, w and ring A are described in the claims of Patent Application CN202210585771.6 with Publication No. CN115403579A; 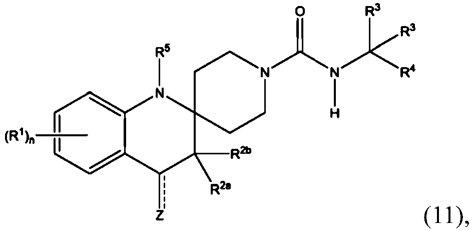 or pharmaceutically acceptable salt thereof,
wherein the definitions of R¹, R^{2a}, R^{2b}, R³, R⁴, R⁵, n and Z are described in the claims of Patent Application PCT/IB2020/057285 with Publication No. WO2021005586A1 (corresponding to Chinese Patent Application No. CN202080053804.1 with Publication No. CN114206870A); 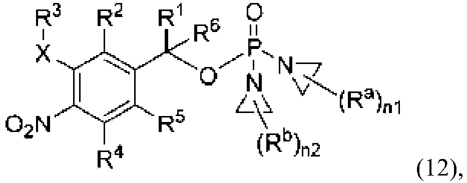 or pharmaceutically acceptable salt thereof,
wherein the definitions of R¹, R², R³, R⁴, R⁵, R⁶, R^{a}, R^{b}, n1 and n2 are described in the claims of Patent Application PCT/CN2023/123253 with Publication No. WO2024078392A1.

11. The formulation unit package according to claim 10, wherein
when the AKR1C3 enzyme-activated prodrug is selected from the compound of formulae (1) - (9), (12) or pharmaceutically acceptable salt, solvate, isotope isomer thereof, the tumor or cancer tissue of the patient is detected to have homology-dependent recombination repair deficiency (HDR), or the patient is detected to have homology-dependent recombination repair deficiency (HDR); preferably,
having homology-dependent recombination repair deficiency must be met any one or both of the following conditions: mutation occurs in one or more genes related to homology-dependent recombination repair (HRR) process or genomic scar (GS) score reaches a predetermined value;
preferably,
the gene related to homology-dependent recombination repair process is selected from ATM, ATR, BARD1, BLM, BRCA1, BRCA2, BRIP1, CDK12, CHEK1, CHEK2, FAAP20, FAN1, FANCE, FANCL, FANCM, MRE11A, NBN, PALB2, POLQ, PPP2R2A, RAD51B, RAD51C, RAD51D, RAD54L, RBBP8, SLX4, XRCC2,
the genomic scar is selected from loss of heterozygosity (LOH), telomeric allelic imbalance (TAI) and large-scale state transition (LST).
